# EUROPEAN PATENT SPECIFICATION

(11) **EP 1 743 021 B1**
(45) Date of publication and mention of the grant of the patent: **26.03.2014**
(21) Application number: 04756624.5
(22) Date of filing: 01.07.2004
(51) Int. Cl.: A61K 35/00

(54) **METHOD OF PREPARING REGENERATIVE CELLS ON A CELL CARRIER AND CELL CARRIER CONTAINMENT DEVICE**
VERFAHREN ZUM HERRICHTEN VON REGENERATIVEN ZELLEN AUF EINEM ZELLTRÄGER UND ZELLTRÄGERHALTEVORRICHTUNG
PROCEDE DE TRAITEMENT DES CELLULES REGENERATIVES POUR PLACEMENT SUR UN SUPPORT DE CELLULES ET DISPOSITIFS DE CONFINEMENT DE SUPPORT DE CELLULES

(30) Priority: 19.03.2004 US 554455 P
(43) Date of publication of application: 17.01.2007
(60) Divisional application: 12193602.5
(73) Proprietor: Cytori Therapeutics, Inc., San Diego, California 92121 (US)
(72) Inventor: HEDRICK, Marc, H., Encinitas, CA 92024 (US); FRASER, John, K., San Diego, CA 92131 (US); RILEY, Susan, Lynn, San Diego, California 92124 (US); TAI, Joseph, San Diego, California 92121 (US)
(74) Representative: Zacco Denmark A/S
(86) International application number: PCT/US2004/021417
(87) International publication number: WO 2005/095581

(56) References cited:
- US-A- 5 916 743
- US-A- 5 916 743
- US-A1- 2003 161 816

## Description

### BACKGROUND OF THE INVENTION

### 1. Field of the Invention

The invention is only limited by the appended claims. The present invention relates to methods of making and using a device, comprised of a cell carrier portion and a cell carrier containment portion, in combination with regenerative cells, e.g., adipose derived regenerative cells, for the treatment of bone related disorders. In particular, the novel combination disclosed herein is useful for promoting bone and/or cartilage formation. The present invention is especially useful for promoting bone formation in normally non-osteoconductive areas, *e.g.*, for disorders requiring therapy in the form of interbody spinal fusion surgery.

### 2. Description of Related Art

Back pain caused by disc degeneration and pressure on nerves near the spine affects nearly 65 million Americans. Common early treatment options include stretching exercises, pain medications or steroid injections into back muscles. If these treatments fail, spinal fusion surgery becomes an option. According to the American Academy of Orthopedic Surgeons, about 250,000 spinal fusion surgeries are performed every year, mostly on adults between the ages of 45 to 64.

Spinal fusion is a process by which two or more of the vertebrae that make up the spinal column are fused together with bone grafts and internal devices (such as rods) that heal into a single solid bone. Spinal fusion can eliminate unnatural motion between the vertebrae and, in turn, reduce pressure on nerve endings. In addition, spinal fusion can be used to treat, for example, injuries to spinal vertebrae caused by trauma; protrusion and degeneration of the cushioning disc between vertebrae (sometimes called slipped disc or herniated disc); abnormal curvatures (such as scoliosis or kyphosis); and weak or unstable spine caused by infections or tumors.

Autogenous bone (bone from the patient) or allograft bone (bone from another individual) are the most commonly used materials to induce bone formation. Generally, small pieces of bone are placed into the space between the vertebrae to be fused. Sometimes larger solid pieces of bone are used to provide immediate structural support. Autogenous bone is generally considered superior at promoting fusion. However, this procedure requires extra surgery to remove bone from another area of the patient's body such as the pelvis or fibula. Thus, it has been reported that about 30 percent of patients have significant pain and tenderness at the graft harvest site, which may be prolonged, and in some cases outlast the back pain the procedure intended to correct. Similarly, allograft bone and other bone graft substitutes, although eliminating the need for a second surgery, have drawbacks in that they have yet to be proven as cost effective and efficacious substitutes for autogenous bone fusion.

An alternative to autogenous or allograft bone is the use of growth factors that promote bone formation. For example, studies have shown that the use of bone morphogenic proteins ("BMPs") results in better overall fusion, less time in the operating room and, more importantly, fewer complications for patients because it eliminates the need for the second surgery. However, use of BMPs, although efficacious in promoting bone growth, can be prohibitively expensive.

Another alternative is the use of a genetically engineered version of a naturally occurring bone growth factor. This approach also has limitations. Specifically, surgeons have expressed concerns that genetically engineered BMPs can dramatically speed the growth of cancerous cells or cause non-cancerous cells to become more sinister. Another concern is unwanted bone creation. There is a chance that bone generated by genetically engineered BMPs could form over the delicate nerve endings in the spine or, worse, somewhere else in the body.

Regenerative medicine, which harnesses the ability of regenerative cells, e.g., stem cells (*i.e.*, the unspecialized master cells of the body) to renew themselves indefinitely and develop into mature specialized cells, may be a means of circumventing the limitations of the prior-art techniques. Stem cells, *i.e.*, both embryonic and adult stem cells, have been shown to possess the nascent capacity to become many, if not all, of the 200+ cell and tissue types of the body, including bone. Recently, adipose tissue has been shown to be a source of adult stem cells (Zuk et *al.*, 2001; Zuk *et al.*, 2002). Adipose tissue (unlike marrow, skin, muscle, liver and brain) is comparably easy to harvest in relatively large amounts with low morbidity (Commons *et al.*, 2001; Katz *et al.*, 2001b). Accordingly, given the limitations of the prior art spinal fusion techniques, there exists a need for a device that incorporates regenerative cells, e.g., stem cells that posses the ability to induce bone formation.

US 2003/0161816 describes processing adipose tissue to deliver to a patient.

### SUMMARY OF THE INVENTION

The invention is only limited by the appended claims. The present invention relates to methods of making and using a device comprised of a cell carrier portion and a cell carrier containment portion, and a therapeutically effective dose of regenerative cells, the combination of which promotes bone and/or cartilage formation in an intended area for bone formation in the recipient, *i.e.*, a target area. The device and cells of the present invention are especially useful for treating disorders that benefit from bone formation in normally non-osteoconductive areas, *e.g.*, for disorders requiring therapy in the form of interbody spinal fusion surgery. The device and cells of the present invention are also useful for treating other disorders or defects that cannot heal by osteoconduction, *e.g.*, defects in long bones as a result of trauma. In a preferred embodiment, one or more portions of the device is resorbable.

The present invention further relates to systems and methods for preparing regenerative cells, *e.g.*, adult stem and progenitor cells. The present invention is yet further based on the discovery of methods and compositions of regenerative cells, *e.g.*, adult stem and progenitor cells, that promote bone and/or cartilage formation. Accordingly, in one embodiment, the present invention is directed to compositions, methods, and systems for using cells derived from adipose tissue that are placed into the cell carrier portion of the device along with such additives necessary to promote, engender, or support bone and/or cartilage formation in a recipient.

In one embodiment, the entire procedure from tissue extraction through processing and placement of the device into the intended bone and/or cartilage formation site of the recipient would all be performed in the same facility, indeed, even within the same room of the patient undergoing the procedure. In a particular embodiment, an effective dose of the regenerative cells, *e.g.*, stem and progenitor cells, are placed onto the cell carrier portion of the device prior to insertion in the target area. In another embodiment, an effective dose of the regenerative cells, *e.g.*, stem and progenitor cells, are placed onto the cell carrier subsequent to placement of the device into the intended bone and/or cartilage formation site of the recipient, *e.g.*, via a syringe. In yet other embodiments, the cells may be placed into the cell carrier portion of the device in combination with other cells, tissue, tissue fragments, or other stimulators of cell growth and/or differentiation. In a preferred embodiment, the cells, with any of the above mentioned additives, are placed into the person (via the device) from whom they were obtained. In a preferred embodiment, the cell carrier portion and/or the cell carrier containment portion is resorbable.

In a certain embodiment, promoting bone and/or cartilage formation in a patient includes steps of: a) providing a tissue removal system; b) removing adipose tissue from a patient using the tissue removal system, the adipose tissue having a concentration of stem cells; c) processing at least a part of the adipose tissue to obtain a concentration of stem cells sufficient to constitute an effective dose for promoting bone and/or cartilage formation; d) adding the regenerative cells, *e.g.*, stem and progenitor cells, obtained from the processing step to a cell carrier portion of the device of the invention; and e) inserting the device containing the cells into an intended bone and/or cartilage formation site of the recipient. In a preferred embodiment, the cell carrier portion and/or the cell carrier containment portion is resorbable. In another preferred embodiment, the tissue processing system is automated.

In another embodiment, promoting bone and/or cartilage formation in a patient includes steps of: a) providing a tissue removal system; b) removing adipose tissue from a patient using the tissue removal system, the adipose tissue having a concentration of stem cells; c) processing at least a part of the adipose tissue to obtain a concentration of stem cells sufficient to constitute an effective dose for promoting bone and/or cartilage formation; d) inserting the device of the invention into an intended bone and/or cartilage formation site of the recipient; and e) adding the regenerative cells obtained from the processing step to the cell carrier portion of the device inserted into the recipient. In a preferred embodiment, the cell carrier portion and/or the cell carrier containment portion is resorbable. In another preferred embodiment, the tissue processing system is automated.

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description.

### BRIEF DESCRIPTION OF THE DRAWINGS

Figure 1. is an illustration of a system for separating and concentrating regenerative cells from tissue which includes one filter assembly.
Figure 2 is an illustration of a system similar to Figure 1 having a plurality of filter assemblies in a serial configuration.
Figure 3 is an illustration of a system similar to Figure 1 having a plurality of filter assemblies in a parallel configuration.
Figure 4 is an illustration of a system for separating and concentrating regenerative cells from tissue which includes a centrifuge chamber.
Figure 5 is a sectional view of a collection chamber including a prefixed filter utilized in a system for separating and concentrating regenerative cells from tissue.
Figure 6 is a sectional view of a processing chamber of a system for separating and concentrating regenerative cells from tissue utilizing a percolative filtration system.
Figure 7 is a sectional view of a processing chamber of a system for separating and concentrating regenerative cells utilizing a centrifuge device for concentrating the regenerative cells.
Figure 8 is another sectional view of the processing chamber of Figure 7.
Figures 9.1, 9.2 and 9.3 illustrate an elutriation component in use with the system of the invention.
Figure 10 is an illustration of a system for separating and concentrating regenerative cells from tissue utilizing vacuum pressure to move fluids through the system. A vacuum system can be constructed by applying a vacuum pump or vacuum source to the outlet of the system, controlled at a predetermined rate to pull tissue and fluid through, using a system of stopcocks, vents, and clamps to control the direction and timing of the flow.
Figure 11 is an illustration of a system for separating and concentrating regenerative cells from tissue utilizing positive pressure to move fluids through the system. A positive pressure system uses a mechanical means such as a peristaltic pump to push or propel the fluid and tissue through the system at a determined rate, using valves, stopcocks, vents, and clamps to control the direction and timing of the flow.
Figure 12A illustrates a filtration process in which the feed stream of fluid flows tangentially to the pores of the filter. Figure 12B illustrates a filtration process in which the feed stream of fluid flows perpendicular to the pores of the filter.
Figure 13 is an illustration of an exemplary disposable set for a system of the invention.
Figure 14 is an illustration of an exemplary re-usable component for a system of the invention.
Figure 15A is an illustration of an exemplary device of the invention assembled using the disposable set of Figure 13 and a re-usable component of Figure 14.
Figure 15B is a flowchart depicting exemplary pre-programmed steps, implemented through a software program, that control automated embodiments of a system of the present invention. Two alternative processing parameters are shown indicating the versatility of the system.
Figure 16 depicts a three-dimensional view of an exemplary cell carrier portion of the device of the invention.
Figure 17 depicts the cell carrier of Figure 16 seeded with the regenerative cells of the present invention.
Figure 18 depicts a three-dimensional view of an exemplary containment portion of the device of the invention.
Figure 19 depicts a three-dimensional view of an exemplary device of the invention.
Figure 20 depicts a three-dimensional view of an exemplary combination of the device and adipose-derived cells of the invention.
Figure 21 depicts a three-dimensional view of another exemplary containment portion of the device of the invention.
Figure 22 depicts a three-dimensional view of another exemplary containment portion of the device of the invention.
Figure 23 depicts a three-dimensional view of another exemplary containment portion of the device of the invention.

### DETAILED DESCRIPTION OF THE INVENTION

The present invention provides a minimally invasive and cost-effective device for promoting bone and/or cartilage formation using a therapeutically effective dose of regenerative cells. The present invention is based, in part, on the discovery that the regenerative cells of the invention are a rich source of bone and cartilage progenitor cells, *e.g.*, osteoprogenitor cells. Furthermore, the regenerative cells of the invention have been shown herein to form bone and cartilage *in vivo*. Accordingly, the device of the present invention can be used to treat bone related disorders, e.g., by promoting bone and/or cartilage formation. Specifically, the present invention provides a novel combination of 1) a device, comprising a porous cell carrier portion, and a cell carrier containment portion (hereinafter referred to herein as "the device"), and 2) a therapeutically effective dose of adipose derived regenerative cells, e.g., adult stem and progenitor cells, that promote bone and/or cartilage formation. In a particular embodiment, the device and cells of the present invention are useful for treating disorders requiring bone formation in normally non-osteoconductive areas, *e.g.*, for the treatment of disorders requiring therapy in the form of interbody spinal fusion surgery and the like.

The present invention also relates to rapid and reliable systems and methods for separating and concentrating regenerative cells, e.g., stem cells and/or progenitor cells, from a wide variety of tissues, including but not limited to, adipose, bone marrow, blood, skin, muscle, liver, connective tissue, fascia, brain and other nervous system tissues, blood vessels, and other soft or liquid tissues or tissue components or tissue mixtures (e.g., a mixture of tissues including skin, blood vessels, adipose, and connective tissue). In a preferred embodiment, the system separates and concentrates regenerative cells from adipose tissue. In another preferred embodiment, the system is automated such that the entire method may be performed with minimal user intervention or expertise. In a particularly preferred embodiment, the regenerative cells obtained using the systems and methods of the present invention are suitable for direct placement into a recipient suffering from a renal disease or disorder from whom the tissue was extracted.

Preferably, the entire procedure from tissue extraction through separating, concentrating and placement of the regenerative cells into the recipient would all be performed in the same facility, indeed, even within the same room of the patient undergoing the procedure. The regenerative cells may be used in a relatively short time period after extraction and concentration. For example, the regenerative cells may be ready for use in about one hour from the harvesting of tissue from a patient, and in certain situations, may be ready for use in about 10 to 40 minutes from the harvesting of the tissue. In a preferred embodiment, the regenerative cells may be ready to use in about 20 minutes from the harvesting of tissue. The entire length of the procedure from extraction through separating and concentrating may vary depending on a number of factors, including patient profile, type of tissue being harvested and the amount of regenerative cells required for a given therapeutic application. The cells may also be placed into the recipient in combination with other cells, tissue, tissue fragments, scaffolds or other stimulators of cell growth and/or differentiation in the context of a single operative procedure with the intention of deriving a therapeutic, structural, or cosmetic benefit to the recipient. It is understood that any further manipulation of the regenerative cells beyond the separating and concentrating phase of the system will require additional time commensurate with the manner of such manipulation.

In order that the present invention may be more readily understood, certain terms are first defined. Additional defmitions are set forth throughout the detailed description.

As used herein, "regenerative cells" refers to any heterogeneous or homologous cells obtained using the systems and methods of the present invention which cause or contribute to complete or partial regeneration, restoration, or substitution of structure or function of an organ, tissue, or physiologic unit or system to thereby provide a therapeutic, structural or cosmetic benefit. Examples of regenerative cells include: ASCs, endothelial cells, endothelial precursor cells, endothelial progenitor cells, macrophages, fibroblasts, pericytes, smooth muscle cells, preadipocytes, differentiated or de-differentiated adipocytes, keratinocytes, unipotent and multipotent progenitor and precursor cells (and their progeny), and lymphocytes.

One mechanism by which the regenerative cells may provide a therapeutic, structural or cosmetic benefit is by incorporating themselves or their progeny into newly generated, existing or repaired tissues or tissue components. For example, ASCs and/or their progeny may incorporate into newly generated bone, muscle, or other structural or functional tissue and thereby cause or contribute to a therapeutic, structural or cosmetic improvement. Similarly, endothelial cells or endothelial precursor or progenitor cells and their progeny may incorporate into existing, newly generated, repaired, or expanded blood vessels to thereby cause or contribute to a therapeutic, structural or cosmetic benefit. Another mechanism by which the regenerative cells may provide a therapeutic, structural or cosmetic benefit is by expressing and/or secreting molecules, e.g., growth factors, that promote creation, retention, restoration, and/or regeneration of structure or function of a given tissue or tissue component.

The regenerative cells may be used in their 'native' form as present in or separated and concentrated from the tissue using the systems and methods of the present invention or they may be modified by stimulation or priming with growth factors or other biologic response modifiers, by gene transfer (transient or stable transfer), by further sub-fractionation of the resultant population on the basis or physical properties (for example size or density), differential adherence to a solid phase material, expression of cell surface or intracellular molecules, cell culture or other *ex vivo* or *in vivo* manipulation, modification, or fractionation as further described herein. The regenerative cells may also be used in combination with other cells or devices such as synthetic or biologic scaffolds, materials or devices that deliver factors, drugs, chemicals or other agents that modify or enhance the relevant characteristics of the cells as further described herein.

As used herein, "regenerative cell composition" refers to the composition of cells typically present in a volume of liquid after a tissue, e.g., adipose tissue, is washed and at least partially disaggregated. For example, a regenerative cell composition of the invention comprises multiple different types of regenerative cells, including ASCs, endothelial cells, endothelial precursor cells, endothelial progenitor cells, macrophages, fibroblasts, pericytes, smooth muscle cells, preadipocytes, differentiated or de-differentiated adipocytes, keratinocytes, unipotent and multipotent progenitor and precursor cells (and their progeny), and lymphocytes. The regenerative cell composition may also contain one or more contaminants, such as collagen, which may be present in the tissue fragments, or residual collagenase or other enzyme or agent employed in or resulting from the tissue disaggregation process described herein.

As used herein, "regenerative medicine" refers to any therapeutic, structural or cosmetic benefit that is derived from the placement, either directly or indirectly, of regenerative cells into a subject. As used herein, the phrase "bone related disorder" is intended to include all bone related disorders, including, for example, disorders requiring spinal fixation, spinal stabilization, repair of segmental defects in the body (such as in long bones and flat bones) and growth of bone in a normally non-osteoconductive environment are bone related disorders. In a particular embodiment, the phrase is intended to include disorders requiring spinal fusion surgery. In addition, bone related disorders is intended to encompass disorders of the vertebrae and discs including, but are not limited to, disruption of the disc annulus such as annular fissures, chronic inflammation of the disc, localized disc herniations with contained or escaped extrusions, relative instability of the vertebrae surrounding the disc and degenerative disc disease. Treatment of a bone related disease or disorder is within the ambit of regenerative medicine.

As used herein, "stem cell" refers to a multipotent regenerative cell with the potential to differentiate into a variety of other cell types, which perform one or more specific functions and have the ability to self-renew. Some of the stem cells disclosed herein may be multipotent.

As used herein, "progenitor cell" refers to a multipotent regenerative cell with the potential to differentiate into more than one cell type and has limited or no ability to self-renew. "Progenitor cell", as used herein, also refers to a unipotent cell with the potential to differentiate into only a single cell type, which performs one or more specific functions and has limited or no ability to self-renew. In particular, as used herein, "endothelial progenitor cell" refers to a multipotent or unipotent cell with the potential to differentiate into vascular endothelial cells.

As used herein, "precursor cell" refers to a unipotent regenerative cell with the potential to differentiate into one cell type. Precursor cells and their progeny may retain extensive proliferative capacity, e.g., lymphocytes and endothelial cells, which can proliferate under appropriate conditions.

As used herein "stem cell number" or "stem cell frequency" refers to the number of colonies observed in a clonogenic assay in which adipose derived cells (ADC) are plated at low cell density (<10,000 cells/well) and grown in growth medium supporting MSC growth (for example, DMEM/F12 medium supplemented with 10% fetal calf serum, 5% horse serum, and antibiotic/antimycotic agents). Cells are grown for two weeks after which cultures are stained with hematoxylin and colonies of more than 50 cells are counted as CFU-F. Stem cell frequency is calculated as the number of CFU-F observed per 100 nucleated cells plated (for example; 15 colonies counted in a plate initiated with 1,000 nucleated regenerative cells gives a stem cell frequency of 1.5%). Stem cell number is calculated as stem cell frequency multiplied by the total number of nucleated ADC cells obtained. A high percentage (∼100%) of CFU-F grown from regenerative cells express the cell surface molecule CD105 which is also expressed by marrow-derived stem cells (Barry et al., 1999). CD105 is also expressed by adipose tissue-derived stem cells (Zuk et al., 2002).

As used herein, the term "adipose tissue" refers to fat including the connective tissue that stores fat. Adipose tissue contains multiple regenerative cell types, including ASCs and endothelial progenitor and precursor cells.

As used herein, the term "unit of adipose tissue" refers to a discrete or measurable amount of adipose tissue. A unit of adipose tissue may be measured by determining the weight and/or volume of the unit. Based on the data identified above, a unit of processed lipoaspirate, as removed from a patient, has a cellular component in which at least 0.1% of the cellular component is stem cells; that is, it has a stem cell frequency, determined as described above, of at least 0.1%. In reference to the disclosure herein, a unit of adipose tissue may refer to the entire amount of adipose tissue removed from a patient, or an amount that is less than the entire amount of adipose tissue removed from a patient. Thus, a unit of adipose tissue may be combined with another unit of adipose tissue to form a unit of adipose tissue that has a weight or volume that is the sum of the individual units.

As used herein, the term "portion" refers to an amount of a material that is less than a whole. A minor portion refers to an amount that is less than 50%, and a major portion refers to an amount greater than 50%. Thus, a unit of adipose tissue that is less than the entire amount of adipose tissue removed from a patient is a portion of the removed adipose tissue.

As used herein, the term "processed lipoaspirate" refers to adipose tissue that has been processed to separate the active cellular component (e.g., the component containing regenerative) from the mature adipocytes and connective tissue. This fraction is referred to herein as "adipose-derived cells" or "ADC." Typically, ADC refers to the pellet of regenerative cells obtained by washing and separating and concentrating the cells from the adipose tissue. The pellet is typically obtained by centrifuging a suspension of cells so that the cells aggregate at the bottom of a centrifuge chamber or cell concentrator.

As used herein, the terms "administering," "introducing," "delivering," "placement" and "transplanting" are used interchangeably herein and refer to the placement of the regenerative cells of the invention into a subject by a method or route which results in at least partial localization of the regenerative cells at a desired site. The regenerative cells can be administered by any appropriate route which results in delivery to a desired location in the subject where at least a portion of the cells or components of the cells remain viable. The period of viability of the cells after administration to a subject can be as short as a few hours, *e.g.*, twenty-four hours, to a few days, to as long as several years.

As used herein, the term "treating" includes reducing or alleviating at least one adverse effect or symptom of a disease or disorder

As used herein, "therapeutically effective dose of regenerative cells" refers to an amount of regenerative cells that are sufficient to bring about a beneficial or desired clinical effect. Said dose could be administered in one or more administrations. However, the precise determination of what would be considered an effective dose may be based on factors individual to each patient, including, but not limited to, the patient's age, size, type or extent of disease, stage of the disease, route of administration of the regenerative cells, the type or extent of supplemental therapy used, ongoing disease process and type of treatment desired (e.g., aggressive vs. conventional treatment).

As used herein, the term "subject" includes warm-blooded animals, preferably mammals, including humans. In a preferred embodiment, the subject is a primate. In an even more preferred embodiment, the subject is a human.

Reference will now be made in detail to the presently preferred embodiments of the invention, examples of which are illustrated in the accompanying figures. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives, and equivalents of the embodiments as may fall within the spirit and scope of the invention as defined by the appended claims. The present invention may be practiced in conjunction with various cell or tissue separation techniques that are conventionally used in the art, and only so much of the commonly practiced process steps are included herein as are necessary to provide an understanding of the present invention.

### I. Methods of the Invention

### 1. Methods of Obtaining Regenerative Cells (ADC)

As previously set forth herein, regenerative cells, e.g., stem and progenitor cells, can be harvested from a wide variety of tissues. The system of the present invention may be used for all such tissues. Adipose tissue, however, is an especially rich source of regenerative cells. Accordingly, the system of the present invention is illustrated herein using adipose tissue as a source of regenerative cells by way of example only and not limitation.

Adipose tissue can be obtained by any method known to a person of ordinary skill in the art. For example, adipose tissue may be removed from a patient by liposuction (syringe or power assisted) or by lipectomy, e.g., suction-assisted lipoplasty, ultrasound-assisted lipoplasty, and excisional lipectomy or combinations thereof. The adipose tissue is removed and collected and may be processed in accordance with any of the embodiments of a system of the invention described herein. The amount of tissue collected depends on numerous factors, including the body mass index and age of the donor, the time available for collection, the availability of accessible adipose tissue harvest sites, concomitant and pre-existing medications and conditions (such as anticoagulant therapy), and the clinical purpose for which the tissue is being collected. For example, the regenerative cell percentage of 100 ml of adipose tissue extracted from a lean individual is greater than that extracted from an obese donor (Table 1). This likely reflects a dilutive effect of the increased fat content in the obese individual. Therefore, it may be desirable, in accordance with one aspect of the invention, to obtain larger amounts of tissue from overweight donors compared to the amounts that would be withdrawn from leaner patients. This observation also indicates that the utility of this invention is not limited to individuals with large amounts of adipose tissue.

**Table 1: Effect of Body Mass Index on Tissue and Cell Yield**

| Body Mass Index Status | Amount of Tissue Obtained (g) | Total Regenerative Cell Yield (x10⁷) |
|---|---|---|
| Normal | 641±142 | 2.1±0.4 |
| Obese | 1,225±173 | 2.4±0.5 |
| p value | 0.03 | 0.6 |

After the adipose tissue is processed, the resulting regenerative cells are substantially free from mature adipocytes and connective tissue. Accordingly, the system of the present invention generates a heterogeneous plurality of adipose derived regenerative cells which may be used for research and/or therapeutic purposes. In a preferred embodiment, the cells are suitable for placement or re-infusion within the body of a recipient. In other embodiments, the cells may be used for research, e.g., the cells can be used to establish stem or progenitor cell lines which can survive for extended periods of time and be used for further study.

Referring now to the Figures, a system 10 of the present invention is generally comprised of one or more of a tissue collection chamber 20, a processing chamber 30, a waste chamber 40, an output chamber 50 and a sample chamber 60. The collection chamber 20 and processing chamber 30 may be referred to collectively as a chamber assembly. The various chambers are coupled together via one or more conduits 12 such that fluids containing biological material may pass from one chamber to another while maintaining a closed, sterile fluid/tissue pathway. The conduits may comprise rigid or flexible bodies referred to interchangeably herein as lumens and tubing, respectively. In certain embodiments, the conduits are in the form of flexible tubing, such as polyethylene tubing conventionally used in clinical settings, silicone or any other material known in the art. The conduits 12 can vary in size depending on whether passage of fluid or tissue is desired. The conduits 12 may also vary in size depending on the amount of tissue or fluid that is cycled through the system. For example, for the passage of fluid, the conduits may have a diameter ranging from about 0.060 to about 0.750 inches and for the passage of tissue, the conduits may have a diameter ranging from 0.312 to 0.750 inches. Generally, the size of the conduits is selected to balance the volume the conduits can accommodate and the time required to transport the tissue or fluids through said conduits. In automated embodiments of the system, the foregoing parameters, i.e., volume and time for transport, must be identified such that the appropriate signals can be transmitted to the processing device of the system. This allows the device to move accurate volumes of liquid and tissue from one chamber to another. The flexile tubing used should be capable of withstanding negative pressure to reduce the likelihood of collapse. The flexible tubing used should also be capable of withstanding positive pressure which is generated by, for example, a positive displacement pump, which may be used in the system.

All the chambers of the system may be comprised of one or more ports, e.g., outlet 22 or inlet 21 ports, which accept standard IV, syringe and suction tubing connections. The ports may be a sealed port such as a rubber septum closed syringe needle access port 51. The inlet ports may be coupled to one or more cannulas (not shown) by way of conduits. For example, a tissue inlet port 21 may be coupled to an integrated single use liposuction cannula and the conduit may be a flexible tubing. The conduits are generally positioned to provide fluid passageways from one chamber of the system to another. Towards this end, the conduits and ports may be coupled to, for example, a suction device (not shown) which may be manually or automatically operated. The suction device may be, e.g., a syringe or an electric pump. The suction device should be capable of providing sufficient negative pressure to aspirate tissue from a patient. Generally, any suitable suction device known to one of ordinary skill in the art, e.g., a surgeon, may be used.

The conduits 12 may further comprise one or more clamps (not shown) to control the flow of material among various components of the system. The clamps are useful for maintaining the sterility of the system by effectively sealing different regions of the system. Alternatively, the conduits 12 may comprise one or more valves 14 that control the flow of material through the system. The valves 14 are identified as open circles in the Figures. In preferred embodiments, the valves may be electromechanical pinch valves. In another embodiment, the valves may be pneumatic valves. In yet other embodiments, the valves may be hydraulic valves or mechanical valves. Such valves are preferably activated by a control system which may be coupled to levers. The levers may be manually manipulated such that the levers are activated. In automated embodiments, the control system may be coupled to the levers as well as to a processing device which may activate the valves at pre-determined activation conditions. In certain automated embodiments, activation of the valves may be partially automated and partially subject to the user's preference such that the process may be optimized. In yet other embodiments, certain valves may be activated manually and others automatically through the processing device. The valves 14 may also be used in conjunction with one or more pumps, e.g., peristaltic pumps 34 or positive displacement pumps (not shown). The conduits 12 and/or the valves 14 may also be comprised of sensors 29, e.g., optical sensors, ultrasonic sensors, pressure sensors or other forms of monitors known in the art that are capable of distinguishing among the various fluid components and fluid levels that flow through the system. In a preferred embodiment, the sensors 29 may be optical sensors.

The system may also include a plurality of filters 36. In certain embodiments, the filters may be within a chamber of the system 28. Different chambers within the system may be comprised of different filters. The filters are effective to separate the regenerative cells, e.g., stem cells and/or progenitor cells, from undesirable cells and disaggregation agents that may be used in accordance with the system. In one embodiment, a filter assembly 36 includes a hollow fiber filtration device. In another embodiment, a filter assembly 36 includes a percolative filtration device, which may or may not be used with a sedimentation process. In a further embodiment, the filter assembly 36 comprises a centrifugation device, which may or may not be used with an elutriation device and process. In yet another embodiment, the system comprises a combination of these filtering devices. The filtration functions of the present invention can be two-fold, with some filters removing things from the final concentration such as collagen, free lipid, free adipocytes and residual collagenase, and with other filters being used to concentrate the final product. The filters of the system may be comprised of a plurality of pores ranging in diameters and/or length from 20 to 800µm. In a preferred embodiment, the collection chamber 20 has a prefixed filter 28 with a plurality of pores ranging from 80 to 400 µm. In another preferred embodiment, the collection chamber 20 has a prefixed filter 28 with a plurality of 265 µm pores. In other embodiments, the filters may be detachable and/or disposable.

The system may also be comprised of one or more temperature control devices (not shown) that are positioned to adjust the temperature of the material contained within one or more chambers of the system. The temperature control device may be a heater, a cooler or both, i.e., it may be able to switch between a heater and a cooler. The temperature device may adjust the temperature of any of the material passing through the system, including the tissue, the disaggregation agents, the resuspension agents, the rinsing agents, the washing agents or the additives. For example, heating of adipose tissue facilitates disaggregation whereas the cooling of the regenerative cell output is desirable to maintain viability. Also, if pre-warmed reagents are needed for optimal tissue processing, the role of the temperature device would be to maintain the pre-determined temperature rather than to increase or decrease the temperature.

To maintain a closed, sterile fluid/tissue pathway, all ports and valves may comprise a closure that maintains the sealed configuration of the system. The closure may be a membrane that is impermeable to fluid, air and other contaminants or it may be any other suitable closure known in the art. Furthermore, all ports of the system may be designed such that they can accommodate syringes, needles or other devices for withdrawing the materials in the chambers without compromising the sterility of the system.

As set forth herein, tissue may be extracted from a patient via any art recognized method. The aspirated tissue may be extracted prior to being placed in the system for processing. The aspirated tissue is typically transferred to the collection chamber 20 through conduits 12 via a sealed entry port, such as a rubber septum closed syringe needle access port (not shown on collection chamber). Alternatively, the tissue extraction step may be part of the system. For example, the collection chamber 20 may be comprised of a vacuum line 11 which facilitates tissue removal using a standard cannula inserted into the patient. Thus, in this embodiment, the entire system is attached to the patient. The tissue may be introduced into the collection chamber 20 through an inlet port 21 via a conduit such as 12a which are part of a closed sterile pathway. The collection chamber 20 may be comprised of a plurality of flexible or rigid canisters or cylinders or combinations thereof. For example, the collection chamber 20 may be comprised of one or more rigid canisters of varying sizes. The collection chamber 20 may also be comprised of one or more flexible bags. In such systems, the bag is preferably provided with a support, such as in internal or external frame, that helps reduce the likelihood that the bag will collapse upon the application of suction to the bag. The collection chamber 20 is sized to hold the requisite amount of saline to appropriately wash and disaggregate the tissue prior to the wash and concentrate stage of the process performed in the processing chamber 30. Preferably, the volume of tissue or fluid present in the collection chamber 20 is easily ascertainable to the naked eye. For example, to obtain regenerative cells from adipose tissue, a suitable collection chamber has the capacity to hold 800 ml of lipoaspirate and 1200 ml of saline. Accordingly, in one embodiment, the collection chamber 20 has a capacity of at least 2 liters. In another embodiment, to separate and concentrate red blood cells from blood, the collection chamber 20 has a capacity of at least 1.5 liters. Generally, the size of the collection chamber 20 will vary depending on the type and amount of tissue collected from the patient. The collection chamber 20 may be sized to hold as little as about 5 ml to up to about 2 liters of tissue. For smaller tissue volumes, e.g., 5 mls to 100 mls, the tissue may be gathered in a syringe prior to transfer to the collection chamber 20.

The collection chamber 20 may be constructed using any suitable biocompatible material that can be sterilized. In a preferred embodiment, the collection chamber 20 is constructed of disposable material that meets biocompatibility requirements for intravascular contact as described in the ISO 10993 standard. For example, polycarbonate acrylic or ABS may be used. The fluid path of the collection chamber 20 is preferably pyrogen free, i.e., suitable for blood use without danger of disease transmittal. In one embodiment, the collection chamber 20 is constructed of a material that allows the user to visually determine the approximate volume of tissue present in the chamber. In other embodiments, the volume of tissue and/or fluid in the collection chamber 20 is determined by automated sensors 29. The collection chamber 20 is preferably designed such that in an automated embodiment, the system can determine the volume of tissue and/or fluid within the chamber with a reasonable degree of accuracy. In a preferred embodiment, the system senses the volume within the collection chamber with an accuracy of plus or minus fifteen percent.

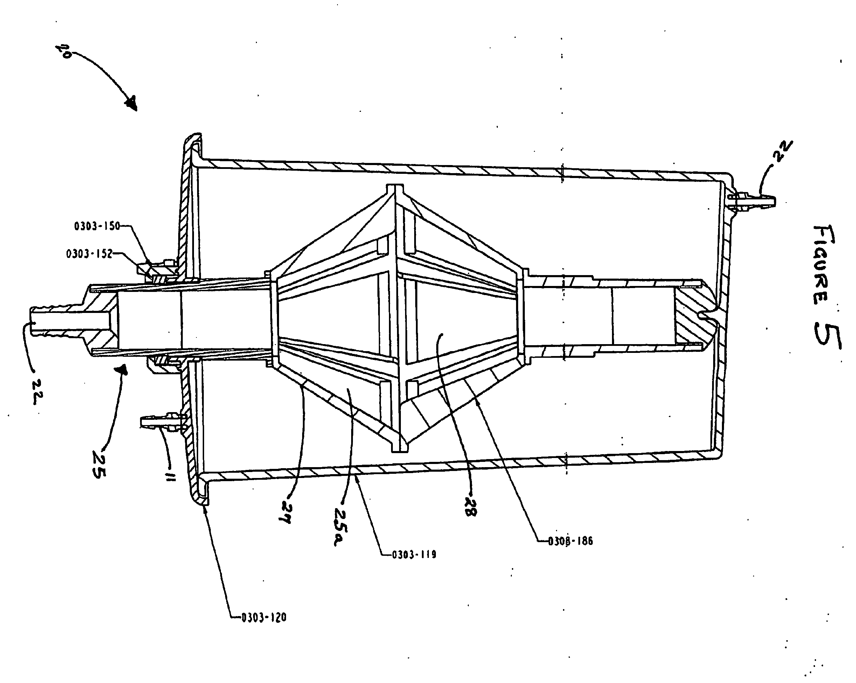

In a particular embodiment provided by way of example only, the collection chamber 20 is in the form of a rigid chamber, for example, a chamber constructed of a medical grade polycarbonate containing a roughly conical prefixes filter 28 of medical grade polyester with a mesh size of 265 µm (see Figure 5). The rigid tissue collection container may have a size of approximately eight inches high and approximately five inches in diameter; the wall thickness may be about 0.125 inches. The interior of the cylinder may be accessed through, for example, one or more ports for suction tubing, one or more ports with tubing for connection through sterile docking technology, and/or one or more ports for needle puncture access through a rubber septum. The prefixed filter 28 in the interior of the collection chamber 20 is preferably structured to retain adipose tissue and to pass non-adipose tissue as, for example, the tissues are removed from the patient More specifically, the filter 28 may allow passage of free lipid, blood, and saline, while retaining fragments of adipose tissue during, or in another embodiment after, the initial harvesting of the adipose tissue. In that regard, the filter 28 includes a plurality of pores, of either the same or different sizes, but ranging in size from about 20 µm to 5 mm. In a preferred embodiment, the filter 28 includes a plurality of 400µm pores. In a preferred embodiment, the filter 28 is a medical grade polyester mesh of around 200 µm thickness with a pore size of around 265 µm and around 47% open area. This material holds the tissue during rinsing but allows cells to pass out through the mesh following tissue disaggregation. Thus, when the tissues are aspirated from the patient, non-adipose tissue may be separated from adipose tissue. The same functionality could be achieved with different materials, mesh size, and the number and type of ports. For example, mesh pore sizes smaller than 100 µm or as large as several thousand microns would achieve the same purpose of allowing passage of saline and blood cells while retaining adipose tissue aggregates and fragments. Similarly, the same purpose could be achieved by use of an alternative rigid plastic material, or by many other modifications that would be known to those skilled in the art

The system 10 may also be comprised of one or more solution sources 22. The solution source may comprise a washing solution source 23, and a tissue disaggregation agent source 24, such as collagenase. The collection chamber 20 is comprised of closed fluid pathways that allows for the washing and disaggregating solutions or agents to be added to the tissue in an aseptic manner.

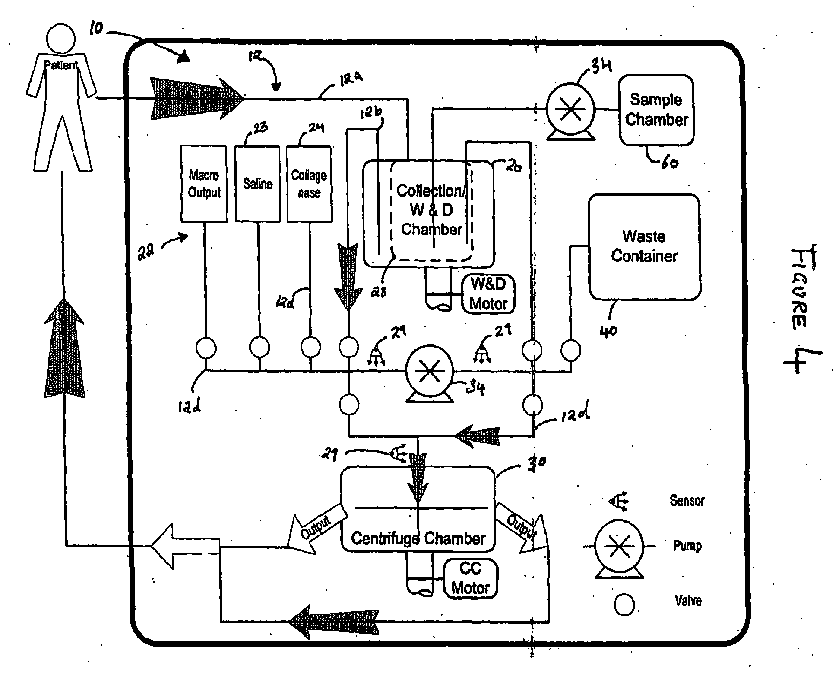

The containers for the washing solution 23 and the disaggregation agents 24 may be any suitable container that can hold their contents in a sterile manner, e.g., a collapsible bag, such as an IV bag used in clinical settings. These containers may have conduits 12, such as conduit 12e, coupled to the collection chamber 20 so that the washing solution and the disaggregation agent may be delivered to the interior of the collection chamber 20. The washing solution and the disaggregation agent may be delivered to the interior of the collection chamber 20 through any art-recognized manner, including simple gravity pressure applied to the outside of the containers for the saline 23 and/or the disaggregation agents 24 or by placement of a positive displacement pump on the conduits, e.g., conduit 12d in Figure 4. In automated embodiments, the processing device of the system calculates various parameters, e.g., the volume of saline and time or number of cycles required for washing as well as the concentration or amount of disaggregation agent and the time required for disaggregation based on information initially entered by the user (e.g., volume of tissue being processed). Alternatively, the amounts, times etc. can be manually manipulated by the user.

The tissue and/or fluid within the collection chamber should be maintained at a temperature ranging from 30 degrees Celsius to 40 degrees Celsius. In a preferred embodiment, the temperature of the suspension inside the collection chamber is maintained at 37 degrees Celsius. In certain embodiments, if the surgical procedure or therapeutic application needs to be delayed, the selected tissue may be stored in the collection chamber for later use. The tissue may be stored at or about room temperature or at about 4 degrees Celsius for up to 96 hours.

The washing solution may be any solution known to one of skill in the art, including saline or any other buffered or unbuffered electrolyte solution. The types of tissue being processed will dictate the types or combinations of washing solutions used. Typically, the washing solution, such as saline, enters the collection chamber 20 after the adipose tissue has been removed from the patient and placed in the collection chamber. However, the washing solution may be delivered to the collection chamber 20 before the adipose tissue is extracted, or may be delivered to the collection chamber 20 concurrently with the adipose tissue. In the collection chamber 20, the washing solution and the extracted adipose tissue may be mixed by any means including the methods described below.

For example, the tissue may be washed by agitation (which maximizes cell viability and minimizes the amount of free lipid released). In one embodiment, the tissue is agitated by rotating the entire collection chamber 20 through an arc of varying degrees (e.g., through an arc of about 45 degrees to about 90 degrees) at varying speeds, e.g., about 30 revolutions per minute. In other embodiments, the tissue is agitated by rotating the entire collection chamber 20, wherein the collection chamber 20 is comprised of one or more paddles or protrusions rigidly attached to an inside surface of the collection chamber, through an arc of varying degrees (e.g., through an arc of about 45 degrees to about 90 degrees) at varying speeds, e.g., about 30 revolutions per minute. The rotation of the collection chamber 20 described above may be accomplished by a drive mechanism attached to or in proximity with the collection chamber 20. The drive mechanism may be a simple belt or gear or other drive mechanism known in the art. The speed of the rotation may be, for example, 30 revolutions per minute. Generally, higher speeds have been found to generate larger volumes of free lipids and may not be optimal.

In other embodiments, the tissue is agitated by placing a rotatable shaft 25 inside the collection chamber 20, wherein the rotatable shaft is comprised of one or more paddles 25a or protrusions rigidly attached to the rotatable shaft 25 which pass through the mixture as the shaft is being rotated. In certain embodiments, the rotatable shaft 25 with rigidly attached 25a paddles may be rested on the bottom of the collection chamber 20. This may be accomplished, for example, by placing the paddle-like device into a spinning magnetic field (e.g., magnetic stirrer). Alternatively, agitating of the tissue may be accomplished using a simple agitator known in the art, i.e. a device implementing shaking up and down without rotation. The tissue may also be washed using any other art-recognized means including rocking, stirring, inversion, etc.

After a desired amount of wash cycles, a tissue disaggregation agent may be delivered to the collection chamber 20 to separate the regenerative cells from the remaining adipose tissue components. The disaggregation agent may be any disaggregation agent known to one of skill in the art. Disaggregation agents that may be used include neutral proteases, collagenase, trypsin, lipase, hyaluronidase, deoxyribonuclease, members of the Blendzyme enzyme mixture family, e.g., Liberase H1, pepsin, ultrasonic or other physical energy, lasers, microwaves, other mechanical devices and/or combinations thereof. A preferred disaggregation agent of the invention is collagenase. The disaggregation agents may be added with other solutions. For example, saline, such as saline delivered from a saline source 23 as described above, may be added to the adipose tissue along with or immediately followed by addition of collagenase. In one embodiment, the washed adipose tissue is mixed with a collagenase-containing enzyme solution at or around 37° C for about 20-60 minutes. In other embodiments, a higher concentration of collagenase or similar agent may be added to decrease the digestion time. The washed adipose tissue and the tissue disaggregation agent may then be agitated in manners similar to the agitation methods described above, until the washed adipose tissue is disaggregated. For example, the washed adipose tissue and the tissue disaggregation agent may be agitated by rotating the entire collection chamber through an arc of approximately 90 degrees, by having a shaft which contains one or more paddles which pass through the solution as the shaft is being rotated, and/or by rotating the entire collection chamber which contains paddles or protrusions on the inside surface of the collection chamber.

Depending on the purpose for which the adipose derived cells will be used, the adipose tissue may either be partially disaggregated, or completely disaggregated. For example, in embodiments in which the adipose derived cells are to be combined with a unit of adipose tissue, it may be desirable to partially disaggregate the harvested adipose tissue, to remove a portion of the partially disaggregated adipose tissue, and then continue disaggregating the remaining portion of adipose tissue remaining in the collection chamber. Alternatively, a portion of washed adipose tissue may be removed and set aside in a sample container prior to any digestion. In another embodiment, harvested adipose tissue is partially disaggregated to concentrate cells before being reintroduced back into the patient. In one embodiment, the adipose tissue is mixed with a tissue disaggregation agent for a period of time generally less than about 20 minutes. A portion of the partially disaggregated tissue may then be removed from the collection chamber, and the remaining partially disaggregated tissue may be further disaggregated by mixing the adipose tissue with a tissue disaggregation agent for another 40 minutes. When the adipose derived cells are to be used as an essentially pure population of regenerative cells, the adipose tissue may be fully disaggregated.

After digestion, the tissue and disaggregation agent solution is allowed to settle for a period of time sufficient to allow the buoyant and non-buoyant components of the solution to differentiate within the collection chamber. Typically, the time ranges from about 15 seconds to several minutes but other times may be implemented in modified embodiments. The buoyant layer is comprised of the regenerative cells that require further washing and concentrating. The non-buoyant layer comprises blood, collagen, lipids and other non-regenerative cell components of the tissue. The non-buoyant layer must be removed to the waste chamber.

Accordingly, the collection chamber 20 is preferably comprised of an outlet port 22 at the lowest point of the chamber such that blood and other non-buoyant components of the tissue may be drained to one or more waste containers 40 via one or more conduits 12. The collection chamber 20 is generally in (or may be placed in) an upright position such that the outlet ports 22 are located at the bottom of the collection chamber. The draining may be passive or active. For example, the non-buoyant components described above could be drained using gravity, by applying positive or negative pressure, by use of pumps 34 or by use of vents 32. In automated embodiments, the processing device can signal certain valves and/or pumps to drain the non-buoyant layer from the collection chamber 20. The automated embodiments may also be comprised of sensors 29 which can detect when the interface between the buoyant and non-buoyant liquids has been reached. The automated embodiments may also be comprised of a sensor 29, e.g., an optical sensor, which may be capable of detecting a change in the light refraction of the effluent which is flowing in the conduit leading out of the collection chamber. The appropriate change in the light refraction may signal the presence of the buoyant layer in the outgoing conduits which indicates that the non-buoyant layer has been drained. The sensor 29 can then signal the processing device to proceed with the next step.

In certain embodiments however, the tissue may be processed to retrieve the non-regenerative cell component of the tissue. For example, in certain therapeutic or research applications, collagen, proteins, matrix or stromal components, lipids, adipocytes or other components of the tissue may be desired. In such embodiments, it is the buoyant layer comprising the regenerative cells that must be removed as described above to the waste chamber. The non-buoyant layer is then retained in the system for further processing as needed.

Once the non-buoyant layer is removed, the buoyant layer comprising the regenerative cells may be washed one or more times to remove residual contaminants. Accordingly, the collection chamber 20 typically includes one or more ports 21 for permitting the washing solution to be delivered to the interior of the chamber, and one or more ports 22 for permitting waste and other materials to be directed out from the collection chamber 20. For example, the collection chamber may include one or more sealed entry ports as described herein. The collection chamber 20 may also include one or more caps (not shown), such as a top cap and a bottom cap to further ensure that the system remains sterile while washing solution is delivered into the collection chamber and/or waste is transported out. The ports 21 may be provided on the caps of the collection chamber or on a sidewall of the collection chamber.

The process of washing with fresh wash solution may be repeated until the residual content of non-buoyant contaminants in the solution reaches a pre-determined level. In other words, the remaining material in the collection chamber 20, which comprises the buoyant material of the mixture described above, including adipose tissue fragments, may be washed one or more additional times until the amount of undesired material is reduced to a desired pre-determined level. One method of determining the end point of the washing is to measure the amount of red blood cells in the tissue solution. This can be accomplished by measuring the light absorbed on the 540 nm wavelength. In a preferred embodiment, a range between about 0.546 and about 0.842 is deemed acceptable.

During the washing and/or disaggregation, one or more additives may be added to the various containers as needed to enhance the results. Some examples of additives include agents that optimize washing and disaggregation, additives that enhance the viability of the active cell population during processing, anti-microbial agents (e.g., antibiotics), additives that lyse adipocytes and/or red blood cells, or additives that enrich for cell populations of interest (by differential adherence to solid phase moieties or to otherwise promote the substantial reduction or enrichment of cell populations). Other possible additives include those that promote recovery and viability of regenerative cells (for example, caspase inhibitors) or which reduce the likelihood of adverse reaction on infusion or emplacement (for example, inhibitors of re-aggregation of cells or connective tissue).

After a sufficient settling time has elapsed, the non-buoyant fraction of the resulting mixture of washed adipose tissue fragments and tissue disaggregation agents will contain regenerative cells, e.g., stem cells and other adipose derived progenitor cells. As discussed herein, the non-buoyant fraction containing the regenerative cells will be transferred to the processing chamber 30 wherein the regenerative cells of interest, such as the adipose derived stem cells, will be separated from other cells and materials present in the non-buoyant fraction of the mixture. This non-buoyant fraction is referred to herein as the regenerative cell composition and comprises multiple different types of cells, including stem cells, progenitor cells, endothelial precursor cells, adipocytes and other regenerative cells described herein. The regenerative cell composition may also contain one or more contaminants, such as collagen and other connective tissue proteins and fragments thereof, which were present in the adipose tissue fragments, or residual collagenase from the tissue disaggregation process.

The processing chamber 30 of the invention is preferably positioned within the system such that the regenerative cell composition moves from the collection chamber 20 to the processing chamber 30 by way of tubing 12, valves 14 and pump 34 in a sterile manner. The processing chamber is sized to accommodate tissue/fluid mixtures ranging from 10mL to 1.2L. In a preferred embodiment, the processing chamber is sized to accommodate 800 mLs. In certain embodiments, the entire regenerative cell composition from the collection chamber 20 is directed to the processing chamber 30. However, in other embodiments, a portion of the regenerative cell composition is directed to the processing chamber 30, and another portion is directed to a different region of the system, e.g., the sample chamber 60, to be recombined with cells processed in the processing chamber 30 at a later time.

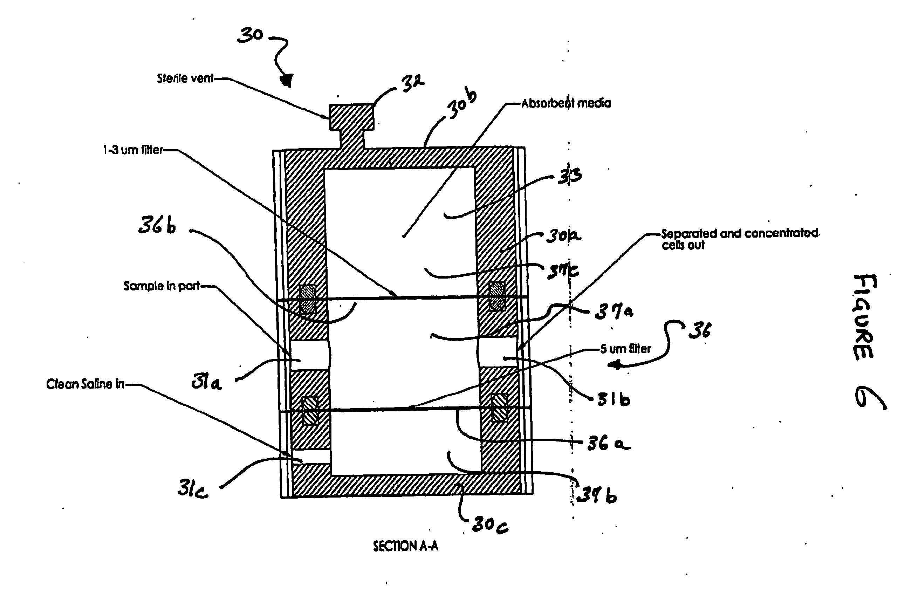

The processing chamber 30 may be constructed using any suitable biocompatible material that can be sterilized. In a preferred embodiment, the processing chamber 30 is constructed of disposable material that meets biocompatibility requirements for intravascular contact, as described in the ISO 10993 standard. For example, polycarbonate, acrylic, ABS, ethylene vinyl acetate or styrene-butadiene copolymers (SBC) may be used. In another embodiment, the fluid path of the disposable processing chamber is pyrogen free. The processing chamber may be in the form of a plastic bag, such as those conventionally used in processing blood in blood banks; or in other embodiments, it may be structurally rigid (Figure 6).

The processing chamber 30 may be constructed in any manner suitable for separating and concentrating cells, including filtration and centrifugation and/or combinations thereof. In certain embodiments, the regenerative cell composition from the collection chamber 20 is introduced into the processing chamber 30 where the composition can be filtered to separate and/or concentrate a particular regenerative cell population. Cell filtration is a method of separating particular components and cells from other different components or types of cells. For example, the regenerative cell composition of the invention comprises multiple different types of cells, including stem cells, progenitor cells and adipocytes, as well as one or more contaminants, such as collagen, which was present in the adipose tissue fragments, or residual collagenase from the tissue disaggregation process. The filters 36 present in the processing chamber 30 may allow for separation and concentration of a particular subpopulation of regenerative cells, e.g., stem cells or endothelial progenitors cells etc.

Some variables which are associated with filtration of cells from a liquid include, but are not limited to, pore size of the filter media, geometry (shape) of the pore, surface area of the filter, flow direction of the solution being filtered, trans-membrane pressure, dilution of the particular cell population, particulate size and shape as well as cell size and cell viability. In accordance with the disclosure herein, the particular cells that are desired to be separated or filtered are typically adipose derived stem cells. However, in certain embodiments, the particular cells may include adipose derived progenitor cells, such as endothelial precursor cells, alone or in combination with the stem cells.

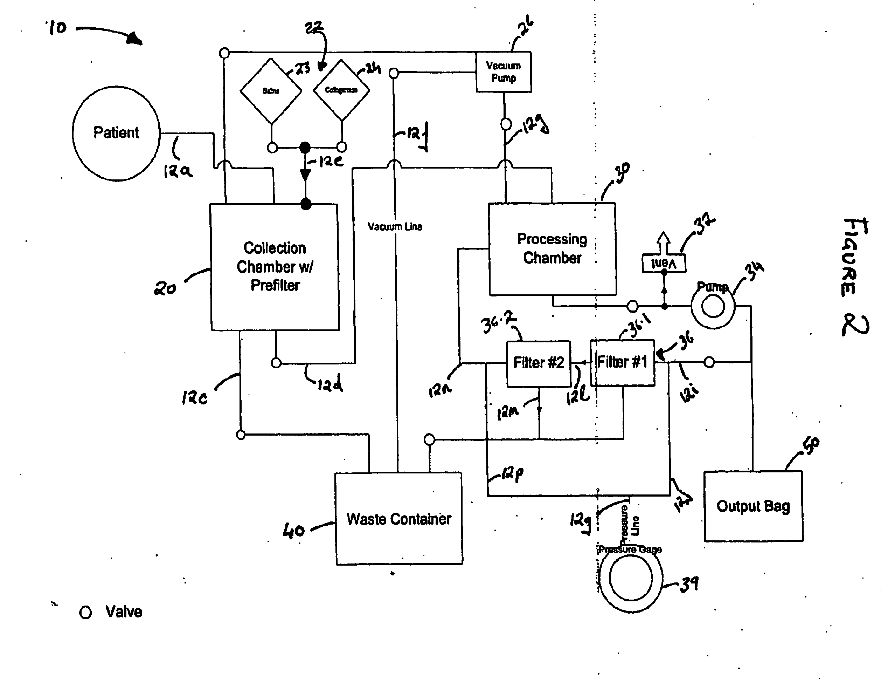

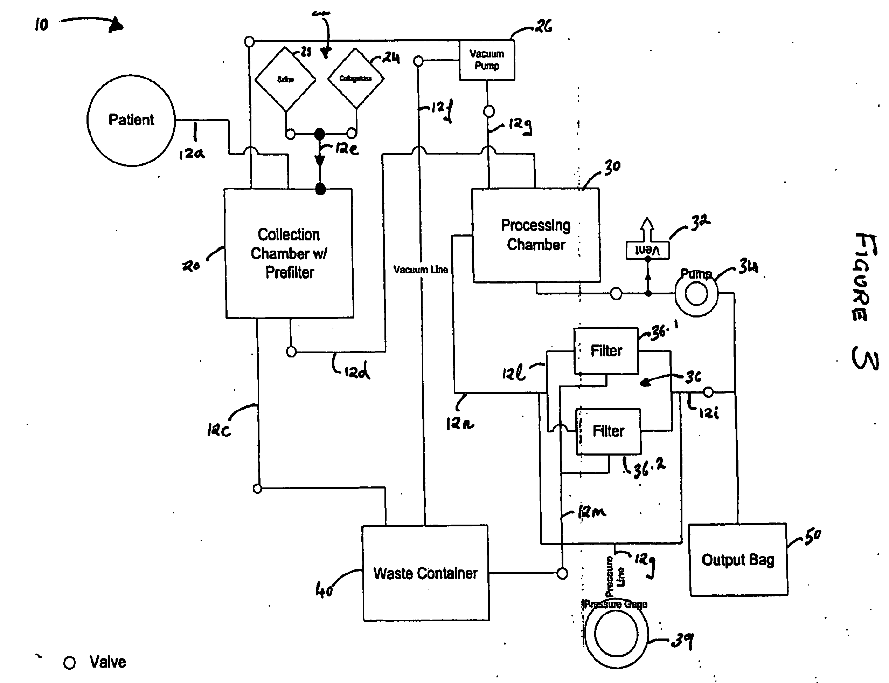

The regenerative cell composition may be directed through a filter assembly, such as filter assembly 36. In certain embodiments, the filter assembly 36 comprises a plurality of filters which are structured to perform different functions and separate the regenerative cell composition into distinct parts or components. For example, one of the filters may be configured to separate collagen from the regenerative cell composition, one of the filters may be configured to separate adipocytes and/or lipid components from the regenerative cell composition, and one of the filters may be configured to separate residual enzymes, such as the tissue disaggregation agent, from the regenerative cell composition. In certain embodiments, one of the filters is capable of performing two functions, such as separating collagen and the tissue disaggregation agent from the composition. The plurality of filters are typically serially arranged; however, at least a portion of the filters may be arranged in parallel, as well. A serial arrangement of the filters of the filter assembly 36 is shown in Figure 2. A parallel arrangement of the filters of the filter assembly 36 is shown in Figure 3.

In one embodiment, the filter assembly 36 comprises a first filter, a second filter, and a third filter. The first filter is configured to remove collagen particles present in the regenerative cell composition. These collagen particles are typically approximately 0.1 microns in diameter and can be up to 20 microns long. The collagen particles may be of varying sizes depending on the digestion. They also may be fibrils, meaning they have twists and turns. Any of the filters described herein may be made from polyethersulfone, polyester, PTFE, polypropylene, PVDF, or possibly cellulose. There are two possibilities for filtering the collagen. One is to try to remove the larger particles first, letting the cells go through, which would require for example a filter probably in the 10 micron range. The second method is to use a smaller size filter, such as 4.5 micron, with the intent that the collagen would be well digested, so as to trap the cells, and let the collagen pass through. This would require a means to float the cells back off the filter. There may also be a possibility of implementing a filter which would attract and hold the collagen fibers.

The second filter is configured to remove free immature adipocytes which are not buoyant in the regenerative cell composition. In one embodiment the second filter can be constructed of polyester and have a pore size between about 30 and about 50 microns with a preferred pore size being about 40 microns. Although referred to as a second filter, placement of such a device may be in a first, rather than second, position to facilitate an initial removal of larger cells and particles. The third filter is configured to remove the unused or residual collagenase or other tissue disaggregation agent present in the composition. In a preferred implementation, the collagenase may degenerate over time. In one embodiment, the third filter comprises a plurality of pores having a diameter, or length less than 1 µm. In certain embodiments, the pores may have diameters that are smaller than 1µm. In other embodiments, the pores have diameters between 10 kD and 5 microns. In certain embodiments, the third filter may be configured to concentrate the regenerative cell population into a small volume of saline or other washing solution, as discussed herein. As presently preferred, only the final filter is the hollow fiber unit. It is not necessary for any of the filters to be of the hollow fiber type. The hollow fiber unit is used for the final filter in a preferred implementation because it is the most efficient in removing the collagenase with the smallest detrimental effect to the regenerative cells. In an embodiment wherein the device is a collection of off the shelf items, the three filters are in separate housings. It is feasible to have the first and second filters combined into one housing if a hollow fiber unit is used for the third filter. If the final filter is not a hollow fiber set-up then all three filters can be contained in one housing.

The filters of the filter assembly 36 may be located in the processing chamber 30 or may be provided as components separate from the processing chamber 30. In addition, the filters of the filter assembly 36 may be provided in multiple processing chambers or in an inline fashion. In certain embodiments, the conduits or tubing may act as a processing chamber or chambers. The processing chamber can be reduced in size such that it becomes the inside volume of the conduits which connect the filters. This type of system will function correctly if the volume of tissue solution is sized appropriately. Thus, the conduits may act as the processing chamber by containing the fluid with cells as it is being run through the filters. Care may be taken to minimize the volume of the conduits so that cells/tissue are not unnecessarily lost in the process of priming and running the system.

Referring to the embodiment described above, the regenerative cell composition, containing the washed cells and residual collagen, adipocytes, and/or undigested tissue disaggregation agent, may be directed through the first filter to remove at least a portion of and preferably substantially all of the collagen particles from the composition so that fewer, and preferably no, collagen particles are present in the filtered solution. The filtered regenerative cell composition containing the adipocytes and/or undigested tissue disaggregation agent, may then be directed through the second filter to remove at least a portion of and preferably substantially all of the free adipocytes from the filtered regenerative cell composition. Subsequently, the twice filtered regenerative cell composition, containing the undigested tissue disaggregation agent, may be directed through the third filter, such as a hollow fiber filtration device, as discussed herein, to remove or reduce the undigested tissue disaggregation agent from the regenerative cell composition.

The thrice-filtered regenerative cell composition (i.e., the composition remaining after being passed through the first, second, and third filters) may then be directed to multiple outlets, which may include a portion of the processing chamber 30 comprising multiple outlets. These outlets can serve to maintain the necessary pressure, as well as to provide connections via conduits to other containers which may include the collection chamber 20, the output chamber 50, and/or the waste container 40.

In one embodiment, a filter of the filter assembly 36 comprises a hollow-fiber filtration member. Or, in other words, the filter comprises a collection of hollow tubes formed with the filter media. Examples of filter media which can be used with the disclosed system 10 include polysulfone, polyethersulfone or a mixed ester material, and the like. These hollow fibers or hollow tubes of filter media may be contained in a cylindrical cartridge of the filter assembly 36. The individual tubes or fibers of filter media typically have an inside diameter which ranges from about 0.1 mm to about 1 mm with a preferred value being about 0.5 mm. The diameter and length of a suitable cylindrical cartridge will determine the number of individual tubes of filter media which can be placed inside the cartridge. One example of a suitable hollow fiber filter cartridge is the FiberFlo^{®} Tangential Flow Filter, catalog #M-C-050-K(Minntech, Minneapolis, Minnesota). Pore sizes of the filter media can range between about 10 kiloDaltons and about 5 microns with a preferred pore size being about 0.5 microns.

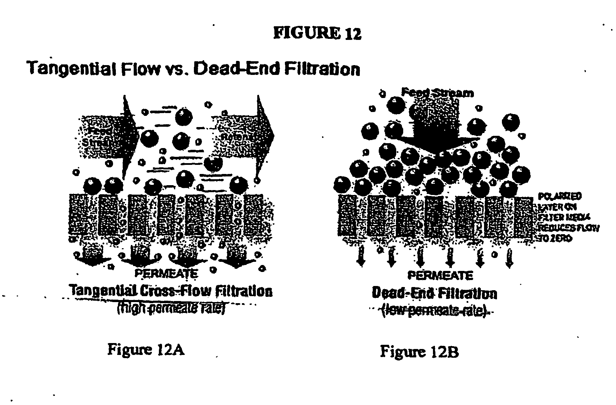

In the hollow-fiber filter, each hollow tube has a body with a first end, a second end, and a lumen located in the body and extending between the first end and second end. The body of each hollow tube includes a plurality of pores. The pores are generally oriented in the body so that a regenerative cell composition is filtered by flowing through the lumen of the body, and the products to be filtered tangentially pass through the pores, as shown in Figure 12A. In other words, the smaller particles in the liquid pass tangentially through the pores relative the flow of fluid through the lumen of the body. The composition with the regenerative cells passes through the lumen of each hollow tube when the composition is being filtered. Preferably, the flow of the composition is tangential to the pores of the body of each hollow tube.

By using a tangential flow of fluid, the efficiency of filtration of the stem cells may be enhanced relative to other filtration techniques. For example, in accordance with some filtration techniques, the pores of the filter media are placed in such a manner that the filter is orientated perpendicular to the flow of the fluid so that the Filter media blocks the path of the fluid being filtered, as illustrated in Figure 12B. In this type of filtration, the particles which are being filtered out of the regenerative cell composition, e.g., the stem cells, tend to build up on one side of the filter and block the flow of the fluid through the pores. This blockage can reduce the efficiency of the filter. In addition, the cells are constantly compressed by the pressure of the fluid flow as well as the weight of the cells accumulating on the upstream side of the filter. This can lead to increased lysis of stem cells. Thus, in such filtration techniques wherein the flow of fluid is parallel to the orientation of the pores in the filter, both large cells and small particles can be undesirably directed against the filter media as the fluid is passed through the pores. Consequently, larger products in the liquid such as cells may block the pores, thereby decreasing the filtering effect and increasing an occurrence of cell rupture or injury.

In contrast, in the hollow fiber configuration of the present system 10, the fluid which is being filtered flows inside the lumen of the hollow tube. The portion of the fluid which has the ability to pass through the pores of the body of the filter does so with the aid of the positive pressure of the fluid on the inside of the body as well as a negative pressure which is applied on the outside of the body. In this embodiment, the cells typically are not subjected to the pressure of the fluid flow or the weight of other cells, and therefore, the shear forces on the stem cells are reduced Thus, the efficiency and effectiveness of the filtration can be enhanced by the reduction in clogging rates and the reduction in regenerative cell lysis. Due to the size of the saline and unwanted protein molecules, during filtration, these molecules and other small components pass through the pores of the bodies of the hollow tubes to the outside of the hollow tubes and are directed to the waste container 40. In one embodiment, filtration is enhanced by generating a vacuum on the outside of the hollow tube filter media. Due to the size of the regenerative cells, e.g., stem cells or progenitor cells, these cells typically cannot pass through the pores of the body and therefore remain on the inside of the hollow tube filter (e.g., in the lumens of the tubes) and are directed back to the processing chamber 30 via a conduit between the filter and the processing chamber, or to the output chamber 50.

In one specific embodiment, the hollow fiber filter has about a 0.05 micron pore size, and contains approximately 550 cm² surface area of filter media. An individual media tube typically has a diameter of about 0.5 mm. In processing 130 ml of the regenerative cell composition, approximately 120 ml of additional saline may be added to the composition. The processing or filter time may be approximately 8 minutes. The differential of the pressures on either side of the body of the hollow fiber tube (e.g., the pressure inside the lumen of the body, and outside the body) is considered the trans-membrane pressure. The trans-membrane pressure can range from about 1 mmHg to about 500 mmHg with a preferred pressure being about 200 mmHg. The average nucleated cell recovery and viability using hollow fiber filtration can be approximately 80% of viable cells.

The amount of collagenase which is typically removed in such a system equates to a three log reduction. For example if the initial concentration of collagenase in the regenerative cell composition which is transferred from the collection chamber to the processing chamber is 0.078 U/ml the collagenase concentration of the final regenerative cell composition would be 0.00078 U/ml. The collagenase is removed in the hollow fiber filter, and the hollow fiber filter corresponds to the third filter discussed above.

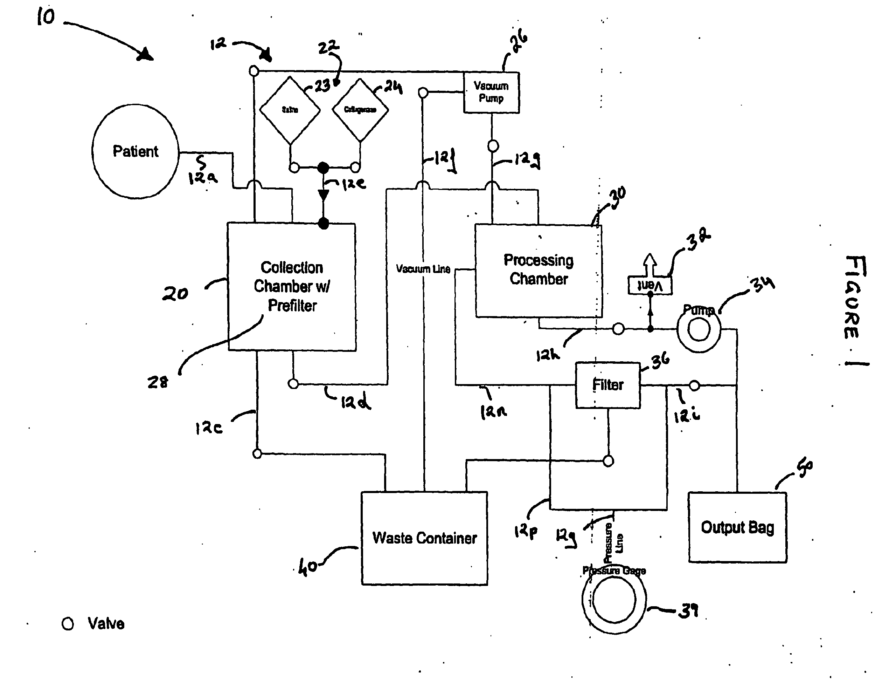

Processing chambers illustrating one or more cell filtration methods described above are shown in the Figures, particularly Figures 1-3. With reference to Figures 1-3, between the processing chamber 30 and the filtering chamber of the filter assembly 36, a pump may be provided, such as pump 34. In addition, vent and pressure sensors, such as vent 32, and pressure sensor 39, may be provided in line with the processing chamber 30 and the filter assembly 36. Fittings for the output chamber 50 may also be provided. These optional components (e.g., the pump 34, the vent 32, the pressure sensor 39, and the fittings for the output chamber 50) may be provided between the processing chamber 30 and the filter assembly 36 so that liquid contained in the processing chamber 30 may flow to one or more of these optional components before flowing through the filter assembly 36. For example, liquid may flow through the pump 34 before it is passed to the filter assembly 36. Or, liquid may pass through the pressure sensor 39 before passing through the filter assembly to obtain a pre-filter liquid pressure in the system. In certain situations, one or more of these components may also be provided as an element of the processing chamber 30, such as the vent 32 as illustrated in Figure. 6. In the illustrated embodiment, the pressure sensor 39 is in line to determine the pressure of the regenerative cell composition which is generated by the pump 34 as it enters the filtering chamber of the filter assembly 36. This construction can facilitate monitoring of the trans-membrane pressure across the filter membrane. Additional saline or other buffer and washing solution can be added to the regenerative cell composition to assist in the removal of unwanted proteins as the composition is being filtered through the filter assembly 36. This repeated washing can be performed multiple times to enhance the purity of the regenerative cells. In certain embodiments, the saline can be added at any step as deemed necessary to enhance filtration.

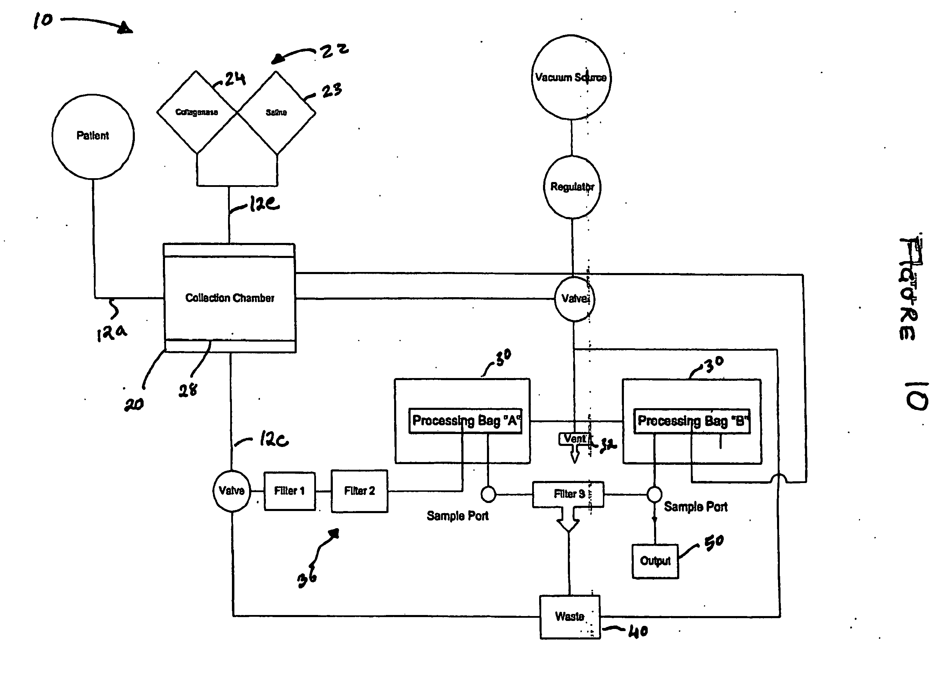

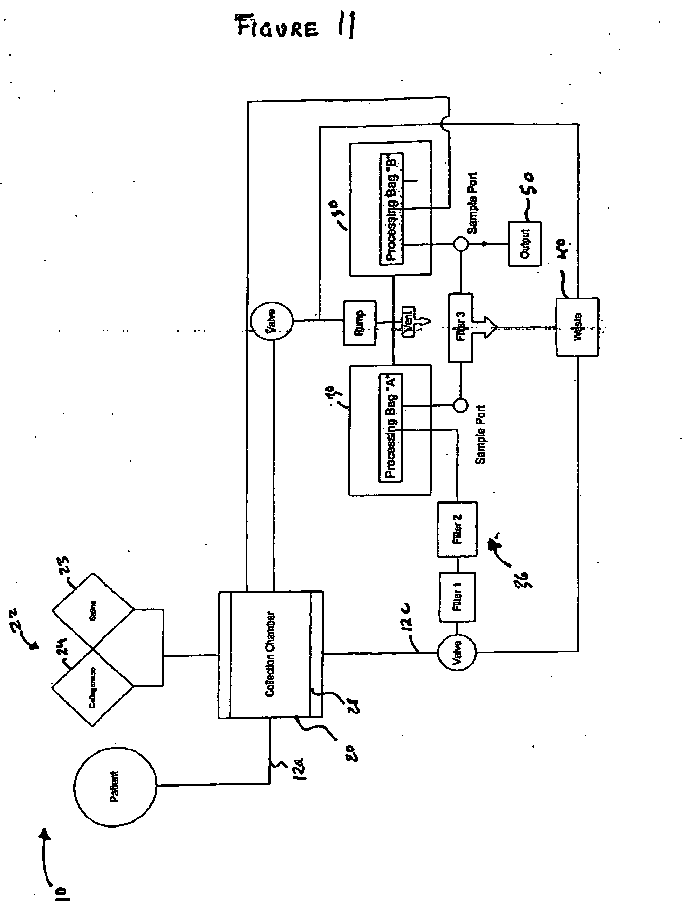

In one specific embodiment, which is provided by way of example and not limitation, the unwanted proteins and saline or other washing solution is removed in the following manner. The composition with the regenerative cells, as well as collagen and connective tissue particles or fragments, adipocytes, and collagenase, is cycled through a series of filters until a minimum volume is reached. The minimum volume is a function of the total hold up volume of the system and some predetermined constant. The hold up volume is the volume of liquid which is contained in the tubing and conduits if all of the processing chambers are empty. In one embodiment, the minimum volume is 15 ml. When the minimum volume is reached, a predetermined volume of washing solution is introduced into the system to be mixed with the regenerative cell composition. This mixture of washing solution and the regenerative cell composition is then cycled through the filters until the minimum volume is reached again. This cycle can be repeated multiple times to enhance the purity of the regenerative cells, or in other words, to increase the ratio of regenerative cells in the composition to the other materials in the composition. See Figures 10 and 11.

After it has been determined that the regenerative cell composition has been cleansed of unwanted proteins and concentrated sufficiently (in exemplary embodiments, minimum concentrations within a range of about 1 x 10⁵ to about 1x10⁷ cells/ml can be used and, in a preferred embodiment the minimum concentration can be about 1 x 10⁷ cells/ml), an output chamber 50, such as an output bag, may be connected to an outlet port of the processing chamber 30 and/or the filter assembly 36, depending on the specific embodiment. A vent, such as the vent 32, may then be opened to facilitate the output of the concentrated regenerative cells. In one implementation, this determination of when a minimum concentration has been reached is made empirically after experiments have been run and programmed into the electronic controls of the device. The determination can be an input into the process of what is desired to yield, i.e., how many stem/progenitor cells are desired, or range of cell concentration. Based on scientific data, a predefined amount of adipose tissue needs to be obtained and placed into the system to achieve the desired output. With the vent 32 open, a pump, such as the pump 34, can function to transfer the concentrated regenerative cells into the output bag. In one embodiment, the output bag 50 is similar to an empty blood bag which has a tube with a fitting on one end. In a sterile fashion, the fitting on the output bag may be attached to the outlet port, and the concentrated regenerative cells may be transferred to the output bag.

As illustrated in Figures 1-3, a vacuum pump 26 may be provided in the system 10 to change the pressure in the system, among other things. For example, the vacuum pump 26 may be coupled to the collection chamber 20 via a conduit, such as conduit 12b, to cause a decrease in pressure within the collection chamber 20. Vacuum pump 26 may also be coupled to the processing chamber 30 by way of a conduit, such as conduit 12g. Regarding the operation of vacuum pump 26 in connection with pump 34, two separate vacuum pumps or sources may be implemented, or a single one may be implemented by using valves which direct the vacuum pull to the different conduits that need it at specific points in the process. In addition, vacuum pump 26 may be coupled to the waste container 40 via a conduit, such as conduit 12f.

With reference to Figures 10 and 11, the pressure generated by the vacuum pump 26 can be used to direct the flow of fluids, including the regenerative cells, through the conduits 12. This pressure can be supplied in multiple directions, for example, by automatically or manually controlling the position of one or more valves 14 in the system 10. The system 10 can be made to function properly with the use of positive pressure or through the use of negative pressure, or combinations thereof. For instance, the regenerative cells can be pulled through the first and second filters described above into a soft sided container which is connected to the third filter. The soft-sided container can be in line (serial) connected ahead of the third filter. The final output chamber may be a soft sided container which is on the other side (e.g., the downstream side) of the third filter. In this embodiment, pressure is used to move the regenerative cells from one soft sided container to a second soft sided container through the filter.

In another embodiment of the system 10, the filtration of the stem cells and/or adipose derived progenitor cells may be accomplished using a combination of percolative filtration and sedimentation. For example, such a system uses saline that is passed through a tissue regenerative cell composition (e.g., the composition containing the stem cells and/or adipose derived progenitor cells) and then through a filter. Some of the variables which are associated with percolative filtration of cells from a regenerative cell composition include, but are not limited to, pore size of the filter media, pore geometry or shape, surface area of the filter, flow direction of the regenerative cell composition being filtered, flow rate of the infused saline, trans-membrane pressure, dilution of the cell population, cell size and viability.

In one embodiment of the system 10, the processing chamber 30 uses a filter assembly 36 which implements percolative filtration and sedimentation to separate and concentrate the regenerative cells. By way of example, and not by way of limitation, the processing chamber 30 is defined as a generally cylindrical body having a sidewall 30a, a top surface 30b, and a bottom surface 30c, as shown in Figure 6. A sterile vent 32 is provided in the top surface 30b.

In the embodiment of Figure 6, the processing chamber 30 is illustrated as including a filter assembly 36, which includes two filters, such as large pore filter 36a, and small pore filter 36b. The pore sizes of the filters 36a and 36b typically are in a range between about 0.05 microns and about 10 microns. The large pore filter 36a may comprise pores with a diameter of about 5 µm, and the small pore filter 36b may comprise pores with a diameter of about 1-3 µm. In one embodiment, the filters have a surface area of about 785 mm². Filters 36a and 36b divide an interior of the processing chamber 30 to include a first chamber 37a, a second chamber 37b, and a third chamber 37c. As shown in Figure 6, first chamber 37a is located between second chamber 37b and third chamber 37c. In addition, first chamber 37a is shown as being the region of the processing chamber 30 having an inlet port 31a and an outlet port 31b. The illustrated processing chamber 30 includes a plurality of ports providing communication paths from an exterior of the processing chamber 30 to the interior of the processing chamber 30, such as ports 31a, 31b, and 31c. The ports 31a, 31b, and 31c, are illustrated as being disposed in the sidewall 30a of a body of the processing chamber 30. However, the ports 31a, 31b, and 31c could be positioned in other regions, as well. Port 31a is illustrated as a sample inlet port, which is constructed to be coupled to a conduit so that a composition containing regenerative cells can be passed into the interior of the processing chamber 30. Port 31b is illustrated as an outlet port constructed to be coupled to a conduit so that the separated and concentrated cells may be removed from the interior of the processing chamber 30. Port 31c is illustrated as an inlet port constructed to be coupled to a conduit for delivery of a fresh washing solution, such as saline into the interior of the processing chamber 30.

In use, the regenerative cells may be introduced into the central chamber 37a via inlet port 31a. Saline or other buffer is introduced into the bottom chamber 37b through inlet port 31c. The saline may be directed through the regenerative cell composition in chamber 37a at a rate of about 10 ml/min. The flow rate of the saline is such that it counteracts the force of gravity. The flow of saline gives the cells in the chamber the ability to separate based on the density of the cells. Typically, as the saline is forced up through the composition the larger cells in the composition will settle to the bottom of the central chamber 37a, and the smaller cells and proteins will be carried away through the second filter 36b into the top chamber 37c. This filtering is accomplished by adjusting the flow rate of the saline such that the larger cells are rolled in place which allows the smaller particles to be liberated and carried off with the saline. The sterile vent 32 is included in the chamber 30 to ensure that the correct pressure gradient is maintained in the three chambers within the processing unit. The upper chamber 37c can comprise an absorbent media 33. The purpose of the absorbent media is to trap the unwanted proteins in the solution to ensure that they do not cross the filter media back into the processing solution, if, for example, the saline flow rate decreases. An absorbent media can be a type of filter material that is absorbent, or attracts materials or components to be filtered out. An outflow port can be added above the top filter to help draw off the waste. Another embodiment of this may be to apply a gentle vacuum from the top to help pull off waste. Absorbent media can be implemented when, as in the illustrated embodiment, the flow rates are relatively small. Excess saline and proteins are then carried away to a waste container.

When the larger cells, (e.g., the adipose derived stem cells and/or progenitor cells) have been sufficiently separated from smaller cells and proteins, the composition containing the separated cells may be concentrated, as discussed herein. The composition may be further concentrated after it has been removed from chamber 37a through outlet port 31b, or while it is in the chamber 37a. In one embodiment, the concentration of cells in the composition is increased in the following manner. After the cells have been sufficiently separated the filters, such as filters 36a and 36b, may be moved towards each other. This movement has the effect of reducing the volume between the two filters (e.g., the volume of chamber 37a). A vibrating member may also be provided in connection with the processing chamber 30 to facilitate concentrating of the cells in the composition. In one embodiment, the vibrating member may be coupled to the filter 36b (e.g., the small pore filter). Vibrating can reduce an incidence of cells becoming trapped in the filters. The reduction in volume of the composition allows the excess saline to be removed as waste and the cells to be concentrated in a smaller volume.

In another embodiment, the concentration of the regenerative cells is accomplished in the following manner. After the cells have been sufficiently separated, the regenerative cell composition can be transferred to another chamber (not shown) which uses gravity to filter out the excess saline. In a preferred embodiment, the sedimentation can occur at the same time as the percolation. This sedimentation may be accomplished by introducing the composition on top of a filter which has a pore size ranging from about 10 kD to about 2 microns. In one embodiment, a suitable filter has a pore size of about 1 micron. The force of gravity will allow the saline and smaller particles to be passed through the filter while preventing the cells in the composition to flow through the filter. After the desired concentration of cells has been obtained, and after the filtered smaller particles have been removed from below the filter, the regenerative cell composition may be agitated to remove the cells from the filter and, subsequently, the concentrated regenerative cells may be transferred to the output bag. The smaller particles can be drawn off as waste through an outlet.

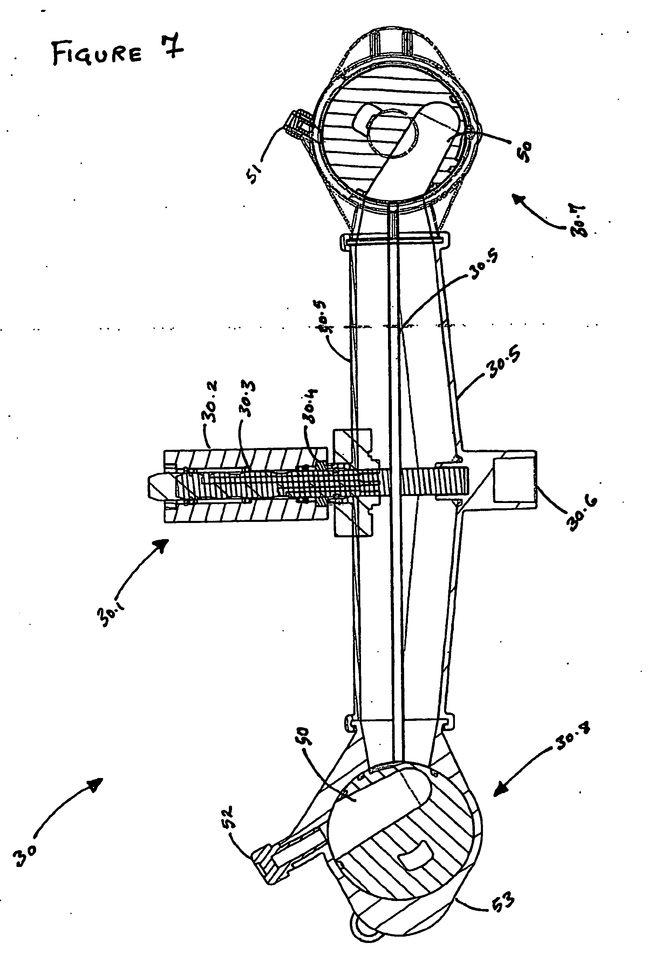

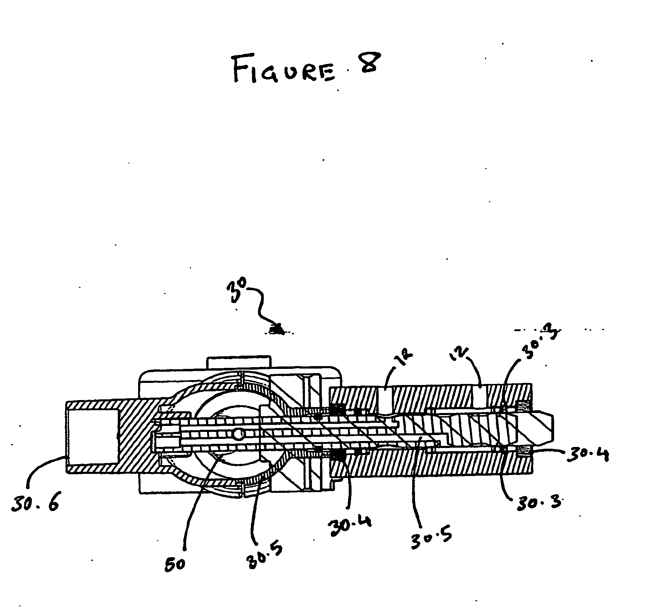

In a particular embodiment, the regenerative cell composition from the collection chamber 20 is transported to the processing chamber 30 wherein the composition can be centrifuged to separate and concentrate regenerative cells. Centrifugation principles are well know in the art and will be not be repeated herein in the interest of brevity. Standard, art-recognized centrifugation devices, components and parameters are utilized herein. An exemplary processing chamber for use as part of a centrifuge device is shown in Figures 7 and 8. Typically, a centrifuge device causes a centrifuge chamber (such as the one shown in Figure 7) to spin around an axis to thereby increasing the force on the cells in the solution to be greater than gravity. The denser or heavier materials in the solution typically settle to one end of the centrifuge chamber, i.e., an output chamber 50 of Figure 7, to form a regenerative cell pellet. The pellet may then be re-suspended to obtain a solution with a desired concentration of cells and/or a desired volume of cells and medium. The processing chamber shown in Figure 7 is constructed to separate and concentrate cells using both centrifugal and gravitational forces. Specifically, during centrifugation, centrifugal force directs the denser components of the regenerative cell composition, e.g., the regenerative cells, towards the outermost ends of the centrifuge chamber. As the centrifuge chamber slows down and eventually stops, gravitational force helps the regenerative cells to remain in the outermost ends of the centrifuge chamber and form a cell pellet. Accordingly, the unwanted components of the regenerative cell composition, i.e., the waste, can be removed without disturbing the cell pellet.

In yet another embodiment of the invention, the processing chamber may be comprised of a cell concentrator in the form of a spinning membrane filter. In a further embodiment of the centrifugation process, centrifugal elutriation may also be applied. In this embodiment, the cells may be separated based on the individual cell sedimentation rate such that the directional (e.g., outward) force applied by centrifugation causes cells and solutes to sediment at different rates. In elutriation, the sedimentation rate of the target cell population is opposed by an opposite (e.g., inward) flow rate applied by pumping solution in the opposite direction to the centrifugal force. The counterflow is adjusted so that the cells and particles within the solution are separated. Elutriation has been applied in many instances of cell separation (Inoue, Carsten et al. 1981; Hayner, Braun et al. 1984; Noga 1999) and the principles and practices used to optimize flow and centrifugal parameters can be applied herein in light of the present disclosure by one skilled in the art.

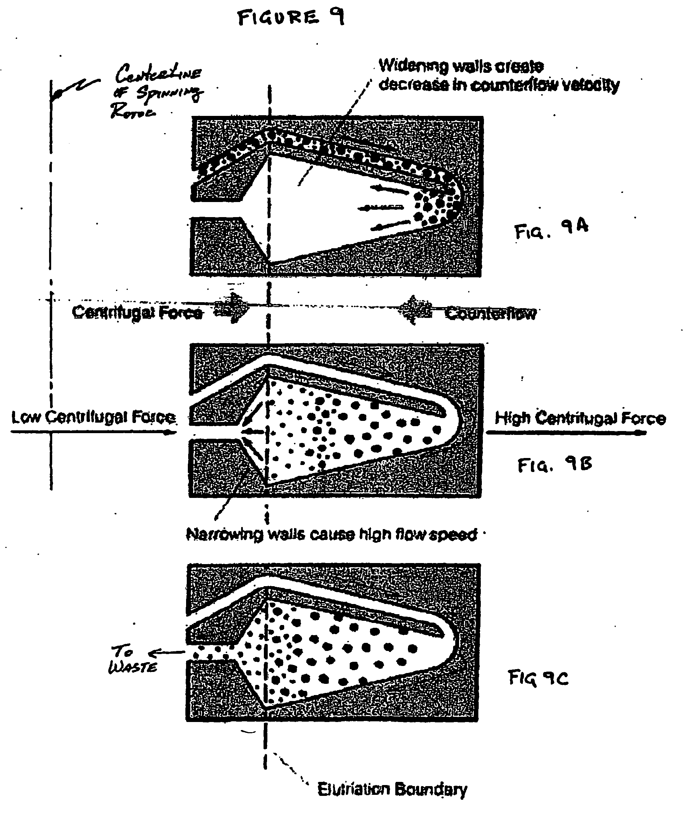

Figure 9 illustrates principles associated with an elutriation implementation in accordance with the present invention. The elutriation embodiment can be similar to a centrifugation implementation to the extent that a force is applied to the solution using a spinning rotor. Some of the variables which are associated with the presently embodied elutriation separation include, but are not limited to, the size and shape of the spinning chamber, the diameter of the rotor, the speed of the rotor, the diameter of the counter flow tubing, the flow rate of the counter flow, as well as the size and density of the particles and cells which are to be removed from solution. As in centrifugation, the regenerative cells can be separated based on individual cell densities.

In one embodiment the regenerative cell composition, e.g., the solution containing the regenerative cells and the collagenase, is introduced into a chamber of a spinning rotor, as shown in Figure 9.1. After the solution is added to the chamber additional saline is added to the chamber at a predetermined flow rate. The flow rate of the saline can be predetermined as a function of the speed of the rotor, the cell diameter, and the chamber constant which has been established empirically. The flow rate will be controlled for example with a device similar to an IV pump. A purpose of the additional saline is to provide a condition inside the rotor chamber where the larger particles will move to one side of the chamber and the smaller particles will move to the other, as illustrated in Figure 9.2. The flow is adjusted so that, in this application, the smaller particles will exit the chamber and move to a waste container, as shown in Figure 9.3. This movement results in the solution in the rotor chamber having a substantially homogenous population of cells, such as stem cells. After it has been determined that the stem cells have been separated from the rest of the items in the solution (with unwanted proteins and free lipids having been removed from the chamber), the counter flow is stopped. The cells inside the chamber will then form a concentrated pellet on the outside wall of the chamber. The counter flow is reversed and the cell pellet is transferred to the output bag.

As previously set forth herein, the processing chamber 30 or the output chamber 50 may include one or more ports, e.g., ports 51 or 52. One or more of these ports may be designed to transport the regenerative cells obtained using any combination of methods described above, or a portion thereof, via conduits to other surgical devices, cell culturing devices, cell marinading devices, gene therapy devices or purification devices. These ports may also be designed to transport the regenerative cells via conduits to additional chambers or containers within the system or as part of another system for the same purposes described above. The ports and conduits may be also be used to add one or more additives, e.g., growth factors, re-suspension fluids, cell culture reagents, cell expansion reagents, cell preservation reagents or cell modification reagents including agents that transfer genes to the cells. The ports and conduits may also be used to transport the regenerative cells to other targets such as implant materials (e.g., scaffolds or bone fragments) as well as other surgical implants and devices.

Further processing of the cells may also be initiated by reconfiguring the interconnections of the disposable sets of the existing system, re-programming the processing device of the existing system, by providing different or additional containers and/ or chambers for the existing system, by transporting the cells to a one or more additional systems or devices and/or any combinations thereof. For example, the system can be reconfigured by any of the means described above such that the regenerative cells obtained using the system may be subject to one or more of the following: cell expansion (of one or more regenerative cell types)and cell maintenance (including cell sheet rinsing and media changing); sub-culturing; cell seeding; transient transfection (including seeding of transfected cells from bulk supply); harvesting (including enzymatic, non-enzymatic harvesting and harvesting by mechanical scraping); measuring cell viability; cell plating (e.g., on microtiter plates, including picking cells from individual wells for expansion, expansion of cells into fresh wells); high throughput screening; cell therapy applications; gene therapy applications; tissue engineering applications; therapeutic protein applications; viral vaccine applications; harvest of regenerative cells or supernatant for banking or screening, measurement of cell growth, lysis, inoculation, infection or induction; generation of cells lines (including hybridoma cells); culture of cells for permeability studies; cells for RNAi and viral resistance studies; cells for knock-out and transgenic animal studies; affinity purification studies; structural biology applications; assay development and protein engineering applications.

For example, if expansion of a regenerative cell population is required for a particular application, an approach using culture conditions to preferentially expand the population while other populations are either maintained (and thereby reduced by dilution with the growing selected cells) or lost due to absence of required growth conditions could be used. Sekiya et al have described conditions which might be employed in this regard for bone marrow-derived stem cells (Sekiya et al., 2002). This approach (with or without differential adherence to the tissue culture plastic) could be applied to a further embodiment of this invention. In this embodiment the final regenerative cell pellet is removed from the output chamber and placed into a second system providing the cell culture component. This could be in the form of a conventional laboratory tissue culture incubator or a Bioreactor-style device such as that described by Tsao et al., US Patent No. 6,001,642, or by Armstrong et al., US Patent No. 6,238,908. In an alternative embodiment, the cell expansion or cell culture component could be added to the existing system, e.g., into the output chamber, allowing for short-term adherence and/or cell culture of the adipose derived cell populations. This alternate embodiment would permit integration of the cell culture and/or cell expansion component to the system and remove the need for removing the cells from this system and placement within another.

During the processing, one or more additives may be added to or provided with the various chambers or containers as needed to enhance the results. These additives may also be provided as part of another system associated with the existing system or separate from the existing system. For example, in certain embodiments, the additives are added or provided without the need for removing the regenerative cells from the system. In other embodiments, the additives are added or provided by connecting a new container or chamber comprising the additives into an unused port of the system in a sterile manner. In yet other embodiments, the additives are added or provided in a second system or device that is not connected to the system of the present invention. Some examples of additives include agents that optimize washing and disaggregation, additives that enhance the viability of the active cell population during processing, anti-microbial agents (e.g., antibiotics), additives that lyse adipocytes and/or red blood cells, or additives that enrich for cell populations of interest (by differential adherence to solid phase moieties or to otherwise promote the substantial reduction or enrichment of cell populations) as described herein.

For example, to obtain a homogenous regenerative cell population, any suitable method for separating and concentrating the particular regenerative cell type may be employed, such as the use of cell-specific antibodies that recognize and bind antigens present on, for example, stem cells or progenitor cells, e.g., endothelial precursor cells. These include both positive selection (selecting the target cells), negative selection (selective removal of unwanted cells), or combinations thereof. Intracellular markers such as enzymes may also be used in selection using molecules which fluoresce when acted upon by specific enzymes. In addition, a solid phase material with adhesive properties selected to allow for differential adherence and/or elution of a particular population of regenerative cells within the final cell pellet could be inserted into the output chamber of the system.

An alternate embodiment of this differential adherence approach would include use of antibodies and/or combinations of antibodies recognizing surface molecules differentially expressed on target regenerative cells and unwanted cells. Selection on the basis of expression of specific cell surface markers (or combinations thereof) is another commonly applied technique in which antibodies are attached (directly or indirectly) to a solid phase support structure (Geiselhart et al., 1996; Formanek et al., 1998; Graepler et al., 1998; Kobari et al., 2001; Mohr et al., 2001).

In another embodiment the cell pellet could be re-suspended, layered over (or under) a fluid material formed into a continuous or discontinuous density gradient and placed in a centrifuge for separation of cell populations on the basis of cell density. In a similar embodiment continuous flow approaches such as apheresis (Smith, 1997), and elutriation (with or without counter-current) (Lasch et al., 2000) (Ito and Shinomiya, 2001) may also be employed.

Other examples of additives may include additional biological or structural components, such as cell differentiation factors, growth promoters, immunosuppressive agents, medical devices, or any combinations thereof, as discussed herein. For example, other cells, tissue, tissue fragments, growth factors such as VEGF and other known angiogenic or arteriogenic growth factors, biologically active or inert compounds, resorbable scaffolds, or other additives intended to enhance the delivery, efficacy, tolerability, or function of the population of regenerative cells may be added. The regenerative cell population may also be modified by insertion of DNA or by placement in a cell culture system (as described herein or known in the art) in such a way as to change, enhance, or supplement the function of the regenerative cells for derivation of a structural or therapeutic purpose. For example, gene transfer techniques for stem cells are known by persons of ordinary skill in the art, as disclosed in (Morizono et al., 2003; Mosca et al., 2000), and may include viral transfection techniques, and more specifically, adeno-associated virus gene transfer techniques, as disclosed in (Walther and Stein, 2000) and (Athanasopoulos et al., 2000). Non-viral based techniques may also be performed as disclosed in (Muramatsu et al., 1998). A gene encoding one or more cellular differentiating factors, e.g., a growth factor(s) or a cytokine(s), could also be added. Examples of various cell differentiation agents are disclosed in (Gimble et al., 1995; Lennon et al., 1995; Majumdar et al., 1998; Caplan and Goldberg, 1999; Ohgushi and Caplan, 1999; Pittenger et al., 1999; Caplan and Bruder, 2001; Fukuda, 2001; Worster et al., 2001; Zuk et al., 2001). Genes encoding anti-apoptotic factors or agents could also be added. Addition of the gene (or combination of genes) could be by any technology known in the art including but not limited to adenoviral transduction, "gene guns," liposome-mediated transduction, and retrovirus or lentivirus-mediated transduction, plasmid, adeno-associated virus. These regenerative cells could then be implanted along with a carrier material bearing gene delivery vehicle capable of releasing and/or presenting genes to the cells over time such that transduction can continue or be initiated *in situ*.

When the cells and/or tissue containing the cells are administered to a patient other than the patient from whom the cells and/or tissue were obtained, one or more immunosuppressive agents may be administered to the patient receiving the cells and/or tissue to reduce, and preferably prevent, rejection of the transplant. As used herein, the term "immunosuppressive drug or agent" is intended to include pharmaceutical agents which inhibit or interfere with normal immune function. Examples of immunosuppressive agents suitable with the methods disclosed herein include agents that inhibit T-cell/B-cell costimulation pathways, such as agents that interfere with the coupling of T-cells and B-cells via the CTLA4 and B7 pathways, as disclosed in U.S. Patent Pub. No. 20020182211. A preferred immunosuppressive agent is cyclosporine A. Other examples include myophenylate mofetil, rapamicin, and anti-thymocyte globulin. In one embodiment, the immunosuppressive drug is administered with at least one other therapeutic agent. The immunosuppressive drug is administered in a formulation which is compatible with the route of administration and is administered to a subject at a dosage sufficient to achieve the desired therapeutic effect. In another embodiment, the immunosuppressive drug is administered transiently for a sufficient time to induce tolerance to the regenerative cells of the invention.

In these embodiments, the regenerative cells may be contacted, combined, mixed or added to the additives through any art recognized manner, including devices such as the agitation devices and associated methods described herein. For example, rocking, inversion, compression pulsed or moving rollers may be used.

In another aspect, the cell population could be placed into the recipient and surrounded by a resorbable plastic sheath or other materials and related components such as those manufactured by MacroPore Biosurgery, Inc. (see e.g., U.S. Patent Nos. 6,269,716; 5,919,234; 6,673,362; 6,635,064; 6,653,146; 6,391,059; 6,343,531; 6,280,473).

In all of the foregoing embodiments, at least a portion of the separated and concentrated regenerative cells may be cryopreserved, as described in U.S. Patent Application No. 10/242,094, entitled PRESERVATION OF NON EMBRYONIC CELLS FROM NON HEMATOPOIETIC TISSUES, filed September 12, 2002, which claims the benefit of U.S. Provisional Patent Application 60/322,070 filed September 14, 2001, which is commonly assigned, and the contents of which in their entireties are expressly incorporated herein by reference.

At the end of processing, the regenerative cells may be manually retrieved from the output chamber. The cells may be loaded into a delivery device, such as a syringe, for placement into the recipient by either, subcutaneous, intramuscular, or other technique allowing delivery of the cells to the target site within the patient. In other words, cells may be placed into the patient by any means known to persons of ordinary skill in the art. Preferred embodiments include placement by needle or catheter, or by direct surgical implantation. In other embodiments, the cells may be automatically transported to an output chamber which may be in the form of a container, syringe or catheter etc., which may be used to place the cells in the patient. The container may also be used to store the cells for later use or for cryopreservation. All retrieval methods are performed in a sterile manner. In the embodiment of surgical implantation, the cells could be applied in association with additives such as a preformed matrix or scaffold as described herein.

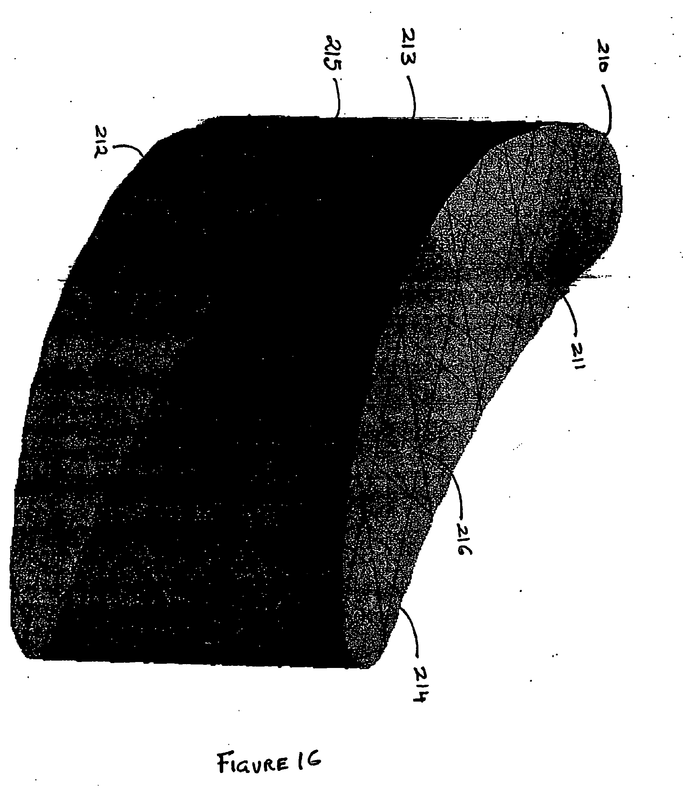

In preferred embodiments of the invention (e.g., the embodiment shown in Figure 4), the system is automated. In another embodiment, the system has both automated and manual components. The system may be comprised of one or more disposable components connected to or mounted on a re-usable hardware component or module. The automated systems of the invention provide screen displays (see Figure 16) that prompt proper operation of the system. The automated systems may also provide a screen that provides status of the procedure and/or the step by step instructions as to the proper setup of the disposable components of the system. The screen may also indicate problems or failures in the system if they occur and provide "troubleshooting" guidance if appropriate. In one embodiment, the screen is a user interface screen that allows the user to input parameters into the system through, e.g., a touch screen.

The partial and fully automated systems may include a processing device (e.g., microprocessor or personal computer) and associated software programs that provide the control logic for the system to operate and to automate one or more steps of the process based on user input. In certain embodiments, one or more aspects of the system may be user-programmable via software residing in the processing device. The processing device may have one or more pre-programmed software programs in Read Only Memory (ROM). For example, the processing device may have pre-programmed software tailored for processing blood, another program for processing adipose tissue to obtain small volumes of regenerative cells and another program for processing adipose tissue to obtain larger volumes of regenerative cells. The processing device may also have pre-programmed software which provides the user with appropriate parameters to optimize the process based on the user's input of relevant information such as the amount of regenerative cells required, the type of tissue being processed, the type of post-processing manipulation required, the type of therapeutic application, etc.

The software may also allow automation of steps such as controlling the ingress and egress of fluids and tissues along particular tubing paths by controlling pumps and valves of the system; controlling the proper sequence and/or direction of activation; detecting blockages with pressure sensors; mixing mechanisms, measuring the amount of tissue and/or fluid to be moved along a particular pathway using volumetric mechanisms; maintaining temperatures of the various components using heat control devices; and integrating the separation and concentration process with timing and software mechanisms. The processing device can also control centrifuge speeds based on the tissue type being processed and/or the cell population or sub-population being harvested, and the types of procedures to be performed (e.g., tissue enhancement using adipose tissue augmented with regenerative cells, or processing of cells for bone repair applications using regenerative cell coated bone grafts). The processing device may also include standard parallel or serial ports or other means of communicating with other computers or networks. Accordingly, the processing device can be a stand alone unit or be associated one or more additional devices for the further processing methods described herein.

The software may allow for automated collection of "run data" including, for example, the lot numbers of disposable components, temperature and volume measurements, tissue volume and cell number parameters, dose of enzyme applied, incubation time, operator identity, date and time, patient identity, etc. In a preferred embodiment of the device a character recognition system, such as a bar code reading system would be integrated to permit data entry of these variables (for example disposable set lot number and expiration date, lot number and expiration date of the Collagenase, patient/sample identifiers, etc.) into the processing device as part of documentation of processing. This would reduce the opportunity for data entry errors. Such a bar code reading system could be easily incorporated into the processing device using a USB or other interface port and system known to the art. In this way the device would provide integrated control of the data entry and documentation of the process. A print-out report of these parameters would be part of the user-defined parameters of a programmed operation of the system. Naturally this would require integration of a printer component (hardware and driver) or printer driver in software plus an interface output connector for a printer (e.g., a USB port) in the hardware of the device.

In certain embodiments, the system is a fully automated system. For example, the user may initially select the amount of tissue to be processed, attach the system to the patient and the system may automatically aspirate the required tissue and separate and concentrate regenerative cells in an uninterrupted sequence without further user input. The user may also input the amount of regenerative cells required and allow the system to aspirate the requisite amount of tissue and process the tissue. A fully automated system also includes a system which is capable of being reconfigured based on a number of (e.g., two or more) user input parameters, e.g., number of wash cycles, speed of centrifugation etc. The system can also be run in semiautomatic mode during which the system goes through certain steps without user intervention but requires user intervention before certain processes can occur. In other embodiments, the system is a single integrated system that displays instructions to guide the user to perform predetermined operations at predetermined times. For example, the processing device may prompt users through the steps necessary for proper insertion of tubing, chambers and other components of the system. Accordingly, the user can ensure that the proper sequence of operations is being performed. Such a system can additionally require confirmation of each operational step by the user to prevent inadvertent activation or termination of steps in the process. In a further embodiment, the system may initiate automated testing to conform correct insertion of tubing, chambers, absence of blockages etc. In yet another embodiment, the system of the present invention is capable of being programmed to perform multiple separation and concentration processes through automated control of tissue flow through the system. This feature may be important, for example, during surgery on a patient where tissue that would otherwise be lost is collected into the system, and regenerative cells from the tissue are separated and concentrated and returned to the patient.

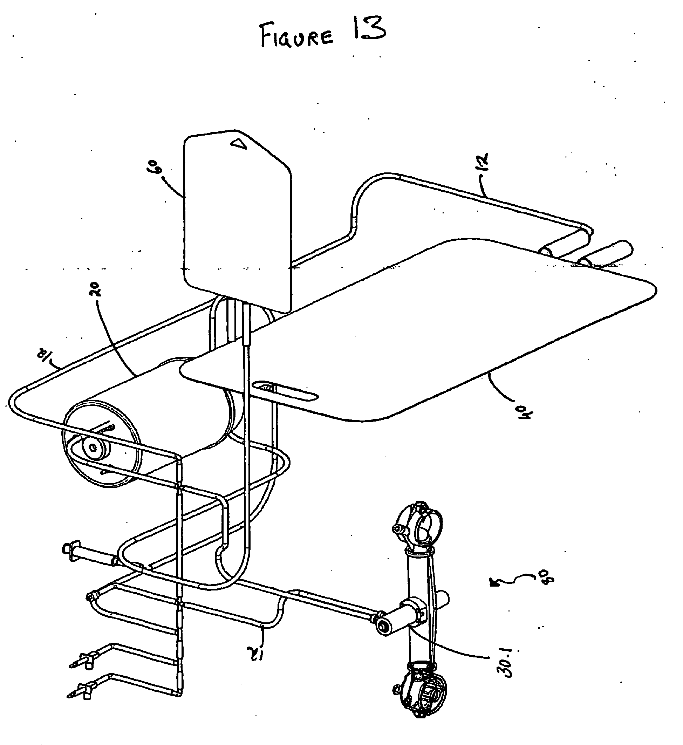

As set forth above, components of the system may be disposable (referred to herein as "disposable set(s)"), such that portions of the system can be disposed of after a single use. This implementation can help ensure that any surface which comes in contact with the patient's tissue will be disposed of properly after being used. An exemplary disposable set is illustrated in Figure 13. In a preferred embodiment, the disposable components of the system are pre-sterilized and packaged so as to be usable "off the shelf' that are easy to use and easy to load and that eliminate the need for many tubing connections and complex routing of tubing connections. Such disposable components are relatively inexpensive to manufacture, and therefore, do not create a substantial expense due to their disposal. In one embodiment, the disposable system (referred to interchangeably herein as "disposable set(s)") comprises, consists essentially of, or consists of, the collection chamber 20, the processing chamber 30, the waste chamber 40, the output chamber 50, the filter assemblies 36, the sample bag 60 and the associated conduits 12 or tubing. In preferred embodiments of the disposable sets of the system, the collection chamber 20 and the processing chamber 30 are connected by way of conduits 12 that are housed in a rigid frame. The rotating seal network (Figures 7 & 8) of a processing chamber 30 may also be housed in the same rigid frame. In another preferred embodiment, the various chambers and containers of the disposable set are comprised of the necessary interfaces that are capable of communicating with the processing device of the system such that the pumps, valves, sensors and other devices that automate the system are appropriately activated or de-activated as needed without user intervention. The interfaces also reduce the time and expertise required to set up the system and also reduce errors by indicating how to properly set up the system and alerting the user in the event of an erroneous setup.

Most of the disposable sets of the invention will have many common elements. However, the ordinarily skilled artisan will recognize that different applications of the system may require additional components which may be part of the disposable sets. Accordingly, the disposable sets may further comprise one or more needles or syringes suitable for obtaining adipose or other tissue from the patient and returning regenerative cells to the patient. The type number and variety of the needles and syringes included will depend on the type and amount of tissue being processed. The disposable sets may further comprise one or more rigid or flexible containers to hold washing fluids and other processing reagents used in the system. For example, the disposable sets may comprise containers to hold saline, enzymes and any other treatment or replacement fluids required for the procedure. In addition, suitable washing solutions, re-suspension fluids, additives, agents or transplant materials may be provided with the disposable sets for use in conjunction with the systems and methods of the invention.

Any combination of system components, equipment or supplies described herein or otherwise required to practice the invention may be provided in the form of a kit. For example, a kit of the invention may include, e.g., the optimal length and gage needle for the syringe based liposuction and sterile syringes which contain the preferred filter media which allows for the processing of small volumes of tissue. Other exemplary equipment and supplies which may be used with the invention and may also be included with the kits of the invention are listed in Tables II and III.

Table II below identifies examples of supplies that can be used in to obtain adipose derived regenerative cell in accordance with the systems and methods of the present invention:

**Table II**

| **Description** | **Vendor** | ***Quantity*** | **Note** |
|---|---|---|---|
| 10 ml syringe | Becton-Dickinson | as req'd | Optional, used for liposuction |
| 14GA blunt tip needle | | as req'd | Optional, used for liposuction |
| Single Blood Pack (600ml) | Baxter Fenwal | 1 | Main cell processing bag; bag has spike adaptor on line and two free spike ports |
| Transfer pack with coupler (150ml) | Baxter Fenwal | 1 | Quad bag set |
| Transfer pack with coupler (1L) | Baxter Fenwal | 1 | Waste bag |
| Sample Site Coupler | Baxter Fenwal | 2 | |
| 0.9% saline (for injection) | Baxter Fenwal | 1 | |
| 14GA sharp needle | Monoject | as req'd | For adding liposuction tissue to bag |
| 20GA sharp needle | Monoject | 3 | For adding collagenase and removing PLA cells |
| 0.2µm Sterflip filter | Millipore | 1 | For filtering collagenase |
| Teruflex Aluminium sealing clips | Terumo | 4 | ME*ACS121 for temporary tube sealing |
| Povidone Iodine prep pad | Triadine | as req'd | 10-3201 |
| Liberase H1 Collagenase | Roche | | See Procedure Note 1 |
| TSCD wafers | Terumo | 2 | 1SC*W017 for use with TSCD Sterile Tubing Welder |

Table III, below, identifies equipment that may be used with the systems and methods disclosed herein.

**Table III**

| Description | Vendor | Quantity | Note |
|---|---|---|---|
| Sorvall Legend T Easy Set Centrifuge | Fisher Scientific | 1 | 75-004-367 |
| Rotor | Kendro/Sorvall | 1 | TTH-750 rotor |
| Rotor buckets | Kenro/Sorvall | 4 | 75006441 round buckets |
| Adaptor for 150ml bags | Kendro/Sorvall | 4 | 00511 |
| Plasma Expressor | Baxter Fenwal | 1 | 4R4414 |
| Tube Sealer | Sebra | 1 | Model 1060 |
| TSCD Sterile Tubing Welder | Terumo | 1 | 3ME*SC201AD |
| LabLine Thermal Rocker | LabLine | 1 | 4637 |
| disposable' plastic hemostat-style clamp | Davron | 3 | |
| Balance Bags Sets | | 2 | Water-filled bags used to balance centrifuge |
| Biohazard Sharps Chamber | | 1 | |
| Biohazard Waste Chamber | | 1 | |

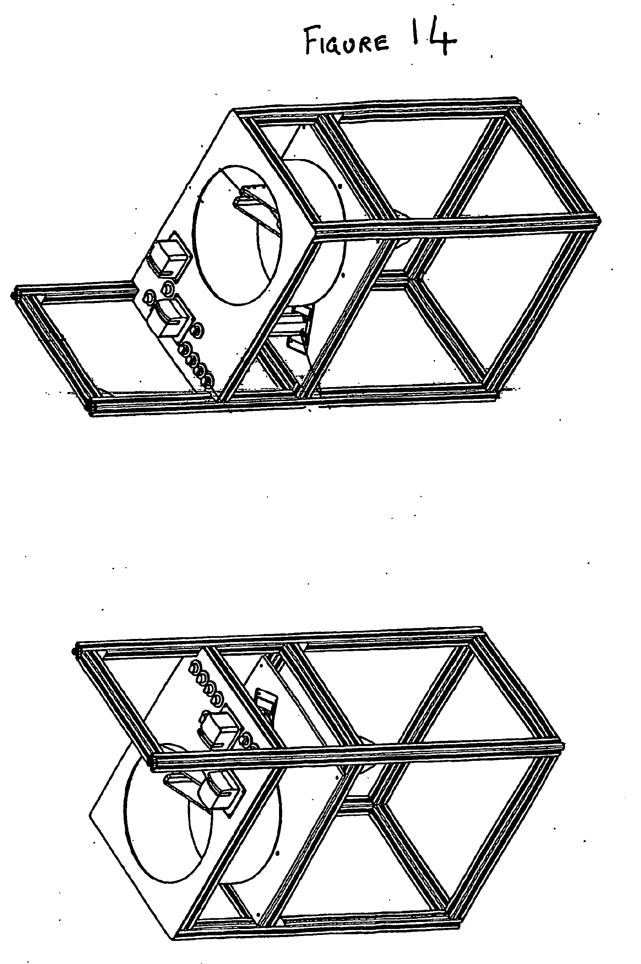

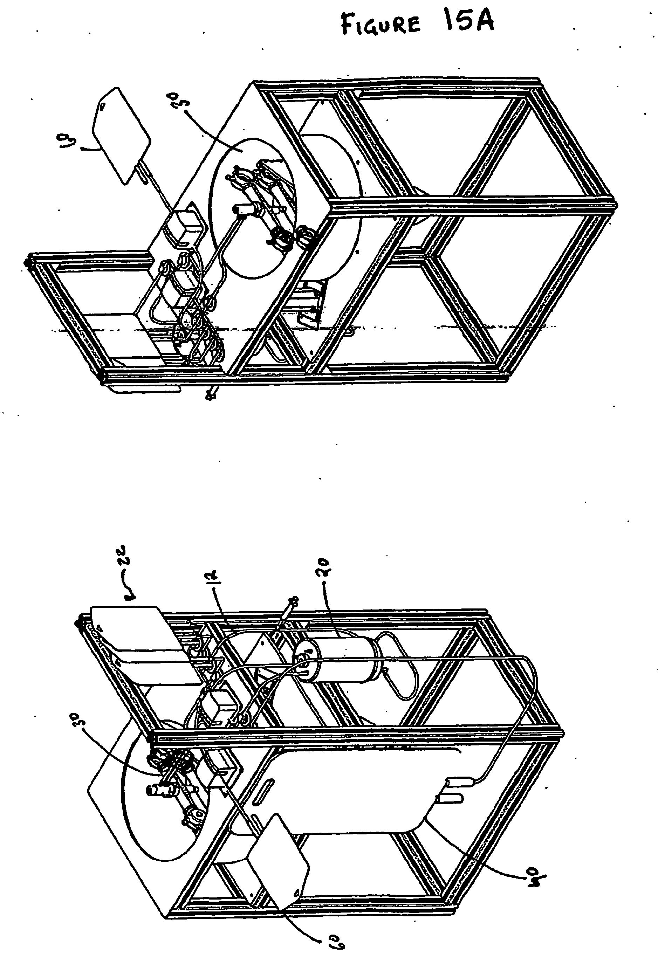

The re-usable component of the system comprises, consists essentially of, or consists of the agitation mechanism for the collection chamber, the pump, and assorted sensors which activate valves and pump controls, the centrifuge motor, the rotating frame of the centrifuge motor, the user interface screen and USB ports, an interlocking or docking device or configuration to connect the disposable set such that the disposable set is securely attached to and interface with the re-usable hardware component and other associated devices. An exemplary re-usable component is illustrated in Figure 14. In preferred embodiments, the re-usable component includes a means for separating and concentrating the regenerative cells from the regenerative cell composition, e.g., a rotating centrifuge. In this embodiment, the re-usable component is designed connect to and interface with a portion of the processing chamber (comprising a centrifuge chamber) of the disposable set as shown in Figure 15A. It is understood that the means for separating and concentrating regenerative cells in the re-usable component is not limited to a rotating centrifuge but may also include any other configuration described herein, including a spinning membrane filter. The re-usable component may also house the processing device described herein which contains pre-programmed software for carrying out several different tissue processing procedures and selectively activating the various pumps and valves of the system accordingly. The processor may also include data storage capability for storing donor/patient information, processing or collection information and other data for later downloading or compilation. The re-usable component may be used with a variety of disposable sets. The disposable set is connected to the re-usable component through, e.g., an interlocking device or configuration to connect the disposable set such that the disposable set is securely attached to and interfaces with the re-usable hardware component in a manner that the processing device present on the re-usable component can control, i.e., send and receive signals to and from the various components of the disposable set as well as various components of the re-usable component and other associated devices and systems.

In one embodiment, a disposable set for use in the system is comprised of a collection chamber 20 which can accommodate about 800 mL of tissue; a processing chamber 30 which can process the regenerative cell composition generated by about 800 mL of tissue washed and digested in the collection chamber 20; an output chamber 50 which can accommodate at least 0.5 mL of regenerative cells; and a waster container 40 which can accommodate about 10 L of waste. In this embodiment, the hardware device is no larger than 24"L X 18"W X 36"H. Alternative dimensions of the various components of the disposable sets as well as the hardware device may be constructed as needed and are intended to be encompassed by the present invention without limitation.

The disposable components of the system are easy to place on the device. An illustration of a disposable set utilized assembled together with a corresponding re-usable component is illustrated in Figure 15A. The system is preferably designed such that it can detect an improperly loaded disposable component. For example, the components of each disposable set may have color-guided marks to properly align and insert the tubing, chambers etc. into appropriate places in the system. In additional embodiments, the system disclosed herein is a portable unit. For example, the portable unit may be able to be moved from one location where adipose tissue harvesting has occurred, to another location for adipose tissue harvesting. In certain implementations, the portable unit is suitable for harvesting and processing of adipose tissue by a patient's bedside. Thus, a portable unit may be part of a system which can be moved from patient to patient. Accordingly, the portable unit may be on wheels which lock in place and, thus, can be easily placed and used in a convenient location in a stable and secure position throughout the procedure. In other embodiments, the portable unit is designed for set-up and operation on a flat surface such as a table top. The portable unit may also be enclosed in a housing unit. The portable unit may further be comprised of hangers, hooks, labels, scales and other devices to assist in the procedure. All of the herein described re-usable components of the system such as the centrifuge, processing device, display screen may be mounted on the portable unit of the system.

Alternate manual embodiments for obtaining regenerative cells are also within the scope of this invention. For example, in one embodiment, adipose tissue may be processed using any combination of the components of the system, equipment and/or supplies described herein.

A particular example of the system embodying the present invention is shown in Figure 4. Figure 4 illustrates an automated system and method for separating and concentrating regenerative cells from tissue, e.g., adipose tissue, suitable for re-infusion within a patient. In certain embodiments of the system shown in Figure 4, the system further includes an automated step for aspirating a given amount of tissue from the patient. The system shown in Figure 4 is comprised of the disposable set shown in Figure 13 which is connected to the re-usable component of the system shown in Figure 14 to arrive at an automated embodiment of the system shown in Figure 15A. The disposable set is connected to the re-usable component through, e.g., an interlocking or docking device or configuration, which connects the disposable set to the re-usable component such that the disposable set is securely attached to and associated with the re-usable hardware component in a manner that the processing device present on the re-usable component can control and interface with, i.e., send and receive signals to and from the various components of the disposable set as well as various components of the re-usable component and other associated devices and systems.

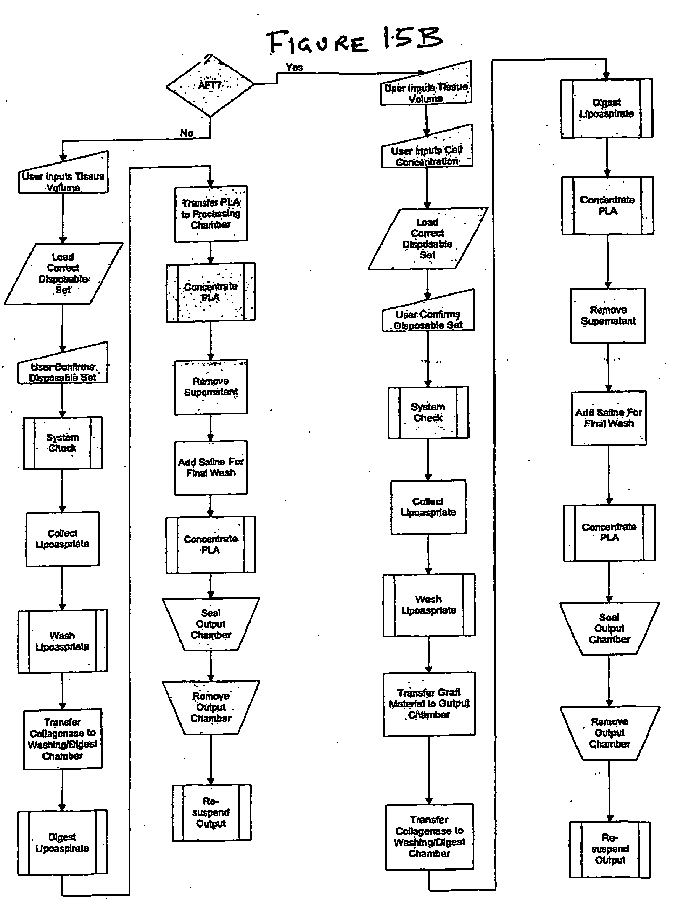

The user may connect the disposable set to the re-usable component, input certain parameters using the user interface, e.g., the volume of tissue being collected, attach the system to the patient, and the system automatically performs all of the steps shown in Figure 4 in an uninterrupted sequence using pre-programmed and/or user input parameters. One such sequence is illustrated in Figure 15B. Alternatively, the tissue may be manually aspirated from the patient by the user and transported to system for processing, i.e., separation and concentration of regenerative cells.

Specifically, as shown in Figure 4, tissue, e.g., adipose tissue, may be withdrawn from the patient using conduit 12 and introduced into collection chamber 20. A detailed illustration of the collection chamber of Figure 4 is shown in Figure 5. As illustrated in Figure 5, the collection chamber 20 may be comprised of a vacuum line 11 which facilitates tissue removal using a standard cannula. The user may enter the estimated volume of tissue directed to the collection chamber 20 at this point. The tissue is introduced into the collection chamber 20 through an inlet port 21 which is part of a closed fluid pathway that allows the tissue, saline and other agents to be added to the tissue in an aseptic manner. An optical sensor of the system, e.g., sensor 29, can detect when the user input volume of tissue is present in the collection chamber 20. In certain embodiments, if less tissue is present in the collection chamber than the user input, the user will have the option to begin processing the volume of tissue which is present in the collection chamber 20. In certain embodiments, a portion of the tissue removed from the patient may be directed to the sample chamber 60 through the use of a pump, e.g., a peristaltic pump, via a conduit, which may be activated via user input utilizing the user interface.

A sensor 29 can signal the processing device present in the re-usable component to activate the steps needed to wash and disaggregate the tissue. For example, the processing device may introduce a pre-set volume of washing agent based on the volume of tissue collected using automated valves and pumps. This cycle may be repeated in the collection chamber until the optical sensor determines that the effluent liquid is sufficiently clear and devoid of unwanted material. For example, an optical sensor 29 along the conduit leading out of the collection chamber 12b or 12d can detect that the unwanted materials have been removed and can signal the processing device to close the required valves and initiate the next step..

Next, the processing device may introduce a pre-programmed amount of disaggregation agent based on the volume of tissue collected. The processing device may also activate agitation of the tissue in the collection chamber for a preset period of time based on the initial volume of tissue collected or based on user input. In the embodiment shown in Figure 4, once the disaggregation agent, e.g., collagenase, is added to the collection chamber 20 through the collagenase source 24, the motor in the collection chamber 20 is activated via the processing device. The motor activates the rotatable shaft 25 which is comprised of a magnetic stirrer and a paddle-like device wherein one or more paddles 25a are rigidly attached to the filter cage 27 of a filter prefixed to the collection chamber 28. The paddles agitate the in the presence of the disaggregation agent such that the regenerative cells separate from the tissue.

The solution in the collection chamber 20 is allowed to settle for a preset period of time. The buoyant portion of the solution is allowed to rise to the top of the solution. Once the preset period of time elapses, the necessary valves and pumps are activated by the processing device to remove the non-buoyant portion to the waste chamber 40. The transfer into the waste chamber 40 continues until a sensor 29 along the conduit leading out of the collection chamber 12b or 12d can detect that the buoyant fraction of the solution is about to be transferred to the waste chamber 30. For example, a sensor 29 along the conduit leading out of the collection chamber 12b or 12d can detect that the unwanted materials have been removed and can signal the processing device to close the required valves.

At this time the non-buoyant fraction of the solution, i.e., the regenerative cell composition, is moved to the processing chamber 30. This is accomplished through the use of the necessary valves and peristaltic pumps. In certain embodiments, before transfer of the regenerative cell composition to the processing chamber 30, an additional volume of saline may be added to the buoyant fraction of solution remaining in the collection chamber 20. Another wash cycle may be repeated. After this cycle, the solution is allowed to settle and the non-buoyant fraction (which contains the regenerative cells) is transported to the processing chamber 30 and the buoyant fraction is drained to the waste chamber 40. The additional wash cycle is used to optimize transfer of all the separated regenerative cells to the processing chamber 30.

Once the regenerative cell composition is transported to the processing chamber 30 by way of conduits 12, the composition may be subject to one or more additional washing steps prior to the start of the concentration phase. This ensures removal of waste and residual contaminants from the collection chamber 20. Similarly, subsequent to the concentration step, the regenerative cell composition may be subjected to one or more additional washing steps to remove residual contaminants. The unwanted materials may be removed from the processing chamber 30 to the waste chamber 40 in the same manner, i.e., control of valves and pumps via signals from the processing device, as described above.

The various embodiments of the processing chamber 30 shown in Figure 4 are described in detail below. The processing chamber 30 shown in Figure 4 is in the form of a centrifuge chamber. A detailed illustration of the processing chamber of Figure 4 is shown in Figures 7 and 8. Such a processing chamber 30 is generally comprised of a rotating seal network30.1 comprising an outer housing 30.2, one or more seals 30.3, one or more bearings 30.4 and an attachment point 30.6 for connecting the processing chamber to the centrifuge device present in the re-usable component of the system; one or more fluid paths 30.5 in the form of conduits extending out from the rotating seal and ending in a centrifuge chamber on each end which is in the form of an output chamber 50 housed in a frame 53 wherein the frame is comprised of one or more ports 52 and one or more handles to manually re-position the output chamber 50.

The rotating seal network 30.1 is included to ensure that the fluid pathways of the processing chamber can be maintained in a sterile condition. In addition, the fluid pathways of the processing chamber can be accessed in a sterile manner (e.g., to add agents or washing solution) at any time, even while the centrifuge chamber of the processing chamber is spinning.

The rotating seal network 30.1 shown in Figures 7 and 8 includes a rotating shaft comprised of two or more bearings 30.4, three or more lip seals 30.3, and an outer housing 30.2. In this embodiment, the bearings 30.4 further comprise an outer and inner shaft (not shown) referred to herein as races. These races may be separated by precision ground spheres. The races and spheres comprising the bearings are preferably fabricated with material suitable for contact with bodily fluid, or are coated with material suitable for contact with bodily fluid. In a preferred embodiment, the races and spheres are fabricated using, for example, silicone nitride or zirconia. Furthermore, in this embodiment, the three lip seals are comprised of a circular "U" shaped channel (not shown) as well as a circular spring (not shown). The circular "U" shaped channel is preferably fabricated using flexible material such that a leakage proof junction with the rotating shaft of the rotating seal network 30.1 is formed. Additionally, the lip seals are preferably oriented in a manner such that pressure from the regenerative cell composition flowing through the processing chamber causes the seal assembly to tighten its junction with the rotating shaft by way of increased tension. The seals may be secured in position by way of one or more circular clips (not shown) which are capable of expanding and/or collapsing as needed in order to engage a groove in the outer housing 30.2 of the rotating seal network 30.1. The heat generated by or near the rotating seal network 30.1 must be controlled to prevent lysis of the cells in the solution which is being moved through the passage. This may be accomplished by, for example, selecting a hard material for constructing the rotating shaft, polishing the area of the rotating shaft which comes in contact with the seals and minimizing contact between the rotating shaft and the seal.

In another embodiment the rotating seal network 30.1 is comprised of a single rubber seal 30.3 and an air gasket (not shown). This seal and gasket provide a tortuous path for any biologic matter which could compromise the sterility of the system. In another embodiment the rotating seal network 30.1 is comprised of multiple spring loaded seals 30.3 which isolate the individual fluid paths. The seals 30.3 are fabricated of a material which can be sterilized as well as seal the rotating shaft without lubricant. In another embodiment the rotating seal network 30.1 is compromised of a pair of ceramic disks (not shown) which create the different fluid paths and can withstand the rotation of the system and not cause cell lysis. In another embodiment the fluid pathway is flexible and is allowed to wind and unwind with respect to the processing chamber. This is accomplished by having the flexible fluid pathway rotate one revolution for every two revolutions of the processing chamber 30. This eliminates the need for a rotating seal altogether.

The regenerative cell composition is pumped from the collection chamber 20 along a fluid path through the axis of rotation of the rotating seal network 30.1 and then divides into a minimum of two fluid pathways 30.5 each of which radiate outward from the central axis of the processing chamber 30 and terminate near the outer ends of the processing chamber 30, i.e., within the centrifuge chambers which house the output chambers 50 (Figure 7 and 8). Accordingly, in a preferred embodiment, the processing chamber 30 is comprised of two or more output chambers 50 as shown in Figures 7 and 8. These output chambers 50 are positioned such that they are in one orientation during processing 30.7 and another orientation for retrieval of concentrated regenerative cells 30.8. For example, the output changes are tilted in one angle during processing and another angle for cell retrieval. The cell retrieval angle is more vertical than the processing angle. The two positions of the output chamber 50 may be manually manipulated through a handle 53 which protrudes out of the processing chamber 30. The regenerative cells can be manually retrieved from the output chambers 50 when they are in the retrieval orientation 30.8 using a syringe. In another embodiment, fluid path 30.5 is constructed such that it splits outside the processing chamber and then connects to the outer ends of the processing chamber 30, i.e., within the centrifuge chambers which house the output chambers 50 (not shown). In this embodiment, large volumes of regenerative cell composition and/or additives, solutions etc. may be transported to the centrifuge chamber and/or the output chambers directly.

With reference to Figures 4 and 7-9, between the collection chamber 20 and the processing chamber 30, a pump 34 and one or more valves 14 may be provided. In a preferred embodiment, the valves 14 are electromechanical valves. In addition, sensors, such as pressure sensor 29, may be provided in line with the processing chamber 30 and the collection chamber 20. The valves, pumps and sensors act in concert with the processing device present on the re-usable component (Figure 14) to automate the concentration steps of the system

The sensors detect the presence of the regenerative cell composition in the centrifuge chambers and activate the centrifuge device through communication with the processing device of the system. The regenerative cell composition is then subjected to a pre-programmed load for a pre-programmed time based on the amount of tissue originally collected and/or user input. In certain embodiments, this step may be repeated either automatically or through user input For example, the composition is subjected to a load of approximately 400 times the force of gravity for a period of approximately 5 minutes. The output chamber 50 is constructed such that the outer extremes of the chamber form a small reservoir for the dense particles and cells. The output chamber 50 retains the dense particles in what is termed a 'cell pellet', while allowing the lighter supernatant to be removed through a fluid path, e.g., a fluid path which is along the axis of rotation of the rotating seal network 30.1 and travels from the low point in the center of the processing chamber 30 through the rotating seal network 30.1 to the waste container 40. The valves 14 and pumps 34 signal the processing device to activate steps to remove the supernatant to the waste container 40 without disturbing the cell pellet present in the output chamber 50.

The cell pellet that is obtained using the system shown in Figure 4 comprises the concentrated regenerative cells of the invention. In some embodiments, after the supernatant is removed and directed to the waste chamber 40, a fluid path 30.5 may be used to re-suspend the cell pellet that is formed after centrifugation with additional solutions and/or other additives. Re-suspension of the cell pellet in this manner allows for further washing of the regenerative cells to remove unwanted proteins and chemical compounds as well as increasing the flow of oxygen to the cells. The resulting suspension may be subjected to another load of approximately 400 times the force of gravity for another period of approximately 5 minutes. After a second cell pellet is formed, and the resulting supernatant is removed to the waste chamber 40, a final wash in the manner described above may be performed with saline or some other appropriate buffer solution. This repeated washing can be performed multiple times to enhance the purity of the regenerative cell solution. In certain embodiments, the saline can be added at any step as deemed necessary to enhance processing. The concentrations of regenerative cells obtained using the system shown in Figure 4 may vary depending on amount of tissue collected, patient age, patient profile etc. Exemplary yields are provided in Table 1.

The final pellet present in the output chamber 50 may then be retrieved in an aseptic manner using an appropriate syringe after the output chamber 50 is positioned in the orientation appropriate for cell removal. In other embodiments, the final pellet may be automatically moved to a container in the in the output chamber 50 which may be removed and stored or used as needed. This container may be in any appropriate form or size. For example, the container may be a syringe. In certain embodiments, the output container 50 itself may be heat sealed (either automatically or manually) and isolated from the other components of the processing chamber for subsequent retrieval and use of the regenerative cells in therapeutic applications as described herein including re-infusion into the patient. The cells may also be subject to further processing as described herein either prior to retrieval from the output chamber or after transfer to a second system or device. The re-usable component shown in Figure 14 is constructed such that it can be connected to one or more additional systems or devices for further processing as needed.

### 2. Methods of Manufacturing a Cell Carrier Portion of the Device

The cell carrier portion of the present invention is critical for maintaining the bone forming functions of the regenerative cells. Specifically, the cell carrier, on which the regenerative cells are placed, as further described herein, serves to organize the cells in three dimensions. Accordingly, in considering substrate materials, it is imperative to choose one that exhibits clinically acceptable biocompatibility. In addition, the mechanical properties of the cell carrier must be sufficient so that it does not collapse during the patient's normal activities.

A variety of cell carriers are known and used in the art and are intended to be encompassed by the present invention. Cell carriers in the form of gels, such as hydrogels are encompassed by the present invention. Fiber based cell carriers are also encompassed by the present invention and include woven meshes, knitted meshes, non-woven meshes (felts) and polymer coated meshes (to enhance strength). Methods for manufacture fiber based cell carriers are known in the art. For example, cell carriers based on knitted meshes can be made according to the methods described by H. J. Buchsbaum, W. Christopherson, S. Lifshitz, and S. Bernstein. Vicryl mesh in pelvic floor reconstruction. Arch.Surg 120 (12):1389-1391, 1985. Non-woven mesh based cell carriers can be made according to the methods described by L. E. Freed, G. Vunjak-Novakovic, R. J. Biron, D. B. Eagles, D. C. Lesnoy, S. K. Barlow, and R. Langer. Biodegradable polymer cell carriers for tissue engineering. Biotechnology (N Y) 12 (7):689-693, 1994. Polymer coated meshes (to enhance strength) can be made according to the methods described by W. S. Kim, J. P. Vacanti, L. Cima, D. Mooney, J. Upton, W. C. Puelacher, and C. A. Vacanti. Cartilage engineered in predetermined shapes employing cell transplantation on synthetic biodegradable polymers. Plast Reconstr Surg 94 (2):233-237, 1994.

In a preferred embodiment, the cell carrier portion of the device will be preformed into particulates. The sizes and shapes of these particulates can range from less than 1 mm to up to 10mm in diameter and from abstract shaped granules to defined shapes, respectively. These particulates can either be highly porous (greater than 95% void volume) or non-porous. The cell carrier particulates can be used to contain the cells into the center of or around the outside of the containment device and can be preloaded into or onto the containment device before packaging and sterilization or can be packaged and sterilized separately from the containment device.

Sponge or foam based cell carriers are also encompassed by the present invention and can be manufactured using methods such as solvent casted-particulate leached (SC-PL), melt molded-particulate leached (where melt molding can include *e.g.*, compression molding, injection molding or extrusion), extrusion -particulate leaching, emulsion-freeze drying , freeze drying combined with particulate-leaching, solution casting, gel casting, atomized foams, phase separation, phase separation combined with particulate leaching, high pressure CO2, gas foaming of effervescent salts, 3D printing, fused deposition modeling and methods using electrospun nanofibers or other nanofibers. In a preferred embodiment, the cell carrier portion of the present invention is manufactured using solvent casting/freeze drying methods.

Methods for manufacturing the sponge or foam based cell carrier of the invention are known in the art. For example, solvent casted-particulate leached (SC-PL) cell carriers can be made according to the methods disclosed in A. G. Mikos, A. J. Thorsen, L. A. Czerwonka, Bao Y., and R. Langer. Preparation and characterization of poly(L-lactic acid) foams. Polymer 35 (5):1068-1077, 1994 and P. X. Ma and J. W. Choi. Biodegradable polymer cell carriers with well-defined interconnected spherical pore network. Tissue Eng 7 (1):23-33, 2001. SC-melt molded cell carriers can be made according to R. C. Thomson, M. J. Yaszemski, J. M. Powers, and A. G. Mikos. Hydroxyapatite fiber reinforced poly(alpha-hydroxy ester) foams for bone regeneration. Biomaterials 19 (21):1935-43, 1998. Extrusion - particulate leaching based cell carriers can be made according to M. S. Widmer, P. K. Gupta, L. Lu, R. K. Meszlenyi, G. R. Evans, K. Brandt, T. Savel, A. Gurlek, C. W. Patrick, Jr., and A. G. Mikos. Manufacture of porous biodegradable polymer conduits by an extrusion process for guided tissue regeneration. Biomaterials 19 (21):1945-1955,1998.

Freeze-drying (Phase separation) based cell carriers can be made according to H. Lo, Ponticiello M.S., and K. W. Leong. Fabrication of controlled release biodegradable foams by phase separation. Tissue Eng 1 (1):15-28, 1995 and C. Schugens, V. Maquet, C. Grandfils, R. Jerome, and P. Teyssie. Polylactide macroporous biodegradable implants for cell transplantation. II. Preparation of polylactide foams by liquid-liquid phase separation. J Biomed Mater.Res 30 (4):449-461, 1996. Scaffolds manufactured using freeze drying (Phase separation) combined with particulate-leaching can be made according to J. H. de Groot, A. J. Nijenhuis, Bruin P., A. J. Pennings, R. P. H. Veth, J. Klompmaker, and H. W. B. Jansen. Use of porous biodegradable polymer implants in meniscus reconstruction. 1. Preparation of porous biodegradable polyurethanes for the reconstruction of meniscus lesions. Colloid Polym.Sci. 268:1073-1081, 1990 and Q. Hou, D. W. Grijpma, and J. Feijen. Preparation of interconnected highly porous polymeric structures by a replication and freeze-drying process. J Biomed Mater.Res 67B (2):732-740, 2003. Freeze-extraction based cell carriers can be made according to M. H. Ho, P. Y. Kuo, H. J. Hsieh, T. Y. Hsien, L. T. Hou, J. Y. Lai, and D. M. Wang. Preparation of porous cell carriers by using freeze-extraction and freeze-gelation methods. Biomaterials 25 (1):129-138, 2004. Emulsion - Freeze drying based cell carriers can be made using the methods disclosed in K. Whang, C. H. Thomas, and K. E. Healy. A novel method to fabricate bioabsorbable cell carriers. Polymer 36 (4):837-842, 1995.

Scaffolds manufactured using gel casting or solution casting methods can be manufactured using J. P. Schmitz and J. O. Hollinger. A preliminary study of the osteogenic potential of a biodegradable alloplastic-osteoinductive alloimplant. Clin Orthop. (237):245-255, 1988, A. G. Coombes and J. D. Heckman. Gel casting of resorbable polymers. 1. Processing and applications. Biomaterials 13 (4):217-224, 1992 and the methods disclosed in U.S. Patent No. 5,716,416 (1998). Methods of manufacturing cell carriers using atomized foams can found in H. Lo, Ponticiello M.S., and K. W. Leong. Fabrication of controlled release biodegradable foams by phase separation. Tissue Eng 1 (1):15-28, 1995. Methods using gas Foaming-particulate leaching can be found in Y. S. Nam, J. J. Yoon, and T. G. Park. A novel fabrication method of macroporous biodegradable polymer cell carriers using gas foaming salt as a porogen additive. J Biomed Mater.Res 53 (1):1-7, 2000.

The 3D printing (Theriform™ process) is described in, for example, R. A. Giordano, B. M. Wu, S. W. Borland, L. G. Cima, E. M. Sachs, and M. J. Cima. Mechanical properties of dense polylactic acid structures fabricated by three dimensional printing. J Biomater.Sci Polym.Ed 8 (1):63-75, 1996 and S. S. Kim, H. Utsunomiya, J. A. Koski, B. M. Wu, M. J. Cima, J. Sohn, K. Mukai, L. G. Griffith, and J. P. Vacanti. Survival and function of hepatocytes on a novel three-dimensional synthetic biodegradable polymer cell carrier with an intrinsic network of channels. Ann Surg 228 (1):8-13, 1998. Fused deposition modeling based methods can be found in D. W. Hutmacher, T. Schantz, I. Zein, K. W. Ng, S. H. Teoh, and K. C. Tan. Mechanical properties and cell cultural response of polycaprolactone cell carriers designed and fabricated via fused deposition modeling. J Biomed Mater.Res 55 (2):203-216, 2001. Methods using electrospun nanofibers can be found in W. J. Li, C. T. Laurencin, E. J. Caterson, R. S. Tuan, and F. K. Ko. Electrospun nanofibrous structure: a novel cell carrier for tissue engineering. J Biomed Mater.Res 60 (4):613-621, 2002. Methods using nanofibers with porogen leaching can be found in R. Zhang and P. X. Ma. Synthetic nano-fibrillar extracellular matrices with predesigned macroporous architectures. J Biomed Mater.Res 52 (2):430-438, 2000.

Both natural (*e.g.*, collagen, elastin, poly(amino acids); and polysaccharides such as hyaluronic acid, glycosamino glycan, carboxymethylcellulose; and ceramic based-cell carriers such as porous hydroxyapatite, tricalcium phosphate, and chitosan) and synthetic materials may be used to manufacture the cell carriers of the present invention. In a preferred embodiment, the cell carrier is constructed of a resorbable material to allow room for tissue growth in the cell carrier while eliminating the need for a second surgery to remove the cell carrier. Exemplary synthetic resorbable polymers that may be used to manufacture the cell carriers of the present invention include, poly(glycolic acid) (PGA), poly(L-lactic acid) (PLLA), poly (D-lactide) (PDLA), poly (D,L-lactide)(PDLLA) and their copolymers, as well as PCL, PDO and PTMC and their co-polymers. These polymers offer distinct advantages in that their sterilizability and relative biocompatibility have been well documented. Also, their resorption rates can be tailored to match that of new tissue formation. In a preferred embodiment, the cell carrier is constructed of 70:30 poly(L-lactide-co-D,L-lactide). In another embodiment, the cell carrier is constructed of 85:15 poly (D,L-lactide-co-glycolide).

In certain embodiments, any of the scaffolds or cell carrier portions described above may be coated with apatite using a simulated body fluid (SBF) solution. The SBF solutions may be prepared with ion concentrations approximately 0-10 times that of human blood plasma and can be sterile filtered through a 22µm PES membrane or a similar membrane filter. Methods of making art-recognized SBF solutions and variations thereof for use in the present invention can be found in, *e.g.*, Chou et al. (2004) The Effect of Biomimetic Apatite Structure on Osteoblast Vitality, Proliferation and Gene Expression Biomaterials (In press); Oyane et al. (2003) Preparation and Assessment of Revised Simulated Body Fluids J. Biomed mater Res 65A: 188-195; Murphy et al. (1999) Growth of Continuous Bonelike Mineral Within Porous Poly(lactic-co-glycolide) Scaffolds In Vitro J. Biomed. Mater. Res. 50: 50-58. The scaffolds or cell carriers may be treated with glow discharge, argon-plasma etching prior to being soaked in the SBF solution to improve wettability and affinity for the SBF ions. Different apatite microenvironments can be created on the scaffold or cell carrier surfaces by controlling the SBF concentration, components, pH and the duration of the scaffold or cell carrier in each SBF solution. Vacuum or fluid flow (directed or non-directed) can be used to force the SBF into the pores of the scaffold or cell carrier portion.

It is understood in the art that desired resorption rates of the cell carrier portion will vary based on the particular therapeutic application. It is believed that the cell carrier portion of the present invention having a thickness of 0.1 millimeters to about 5 millimeters should maintain its structural integrity for a period in excess of about two weeks to multiple months, preferably three to six months, before substantially degrading, so that the bone forming can be achieved and optimized.

The rates of resorption of the cell carrier may also be selectively controlled. For example, the cell carrier portion may be manufactured to degrade at different rates depending on the rate of recovery of the patient from a surgical procedure. Thus, a patient who recovers more quickly from a surgical procedure relative to an average patient, may be administered an agent that for example is selective for the polymeric material of the cell carrier portion and causes the cell carrier portion to degrade more quickly. Or, if the polymeric material is, for example, temperature sensitive or is influenced by electrical charge, the area in which the device has been implanted may be locally heated or cooled, or otherwise exposed to an electrical charge that causes the device to dissolve at a desired rate for the individual patient.

In addition to resorption rates, certain physical characteristics of the cell carriers must also be considered. Generally, the cell carrier must have large a large surface area to allow cell attachment. One method of achieving this result may be to create a highly porous polymer foam. In these foams, the pore size should be large enough so that cells penetrate the pores, and the pores must be interconnected to facilitate nutrient exchange by cells deep within the construct. These characteristics (porosity and pore size) are often dependent on the method of cell carrier fabrication (Mikos, A. G., Bao, Y., Cima, L. G., Ingber, D. E., Vacanti, J. P. and Langer, R. (1993a). Preparation of Poly(glycolic acid) bonded fiber structures for cell attachment and transplantation. Journal of Biomedical Materials Research 27:183-189; Mikos, A. G., Sarakinos, G., Leite, S. M., Vacanti, J. P. and Langer, R. (1993b) Mikos, A. G., Thorsen, A. J., Czerwonka, L. A., Bao, Y., Langer, R., Winslow, D. N. and Vacanti, J. P. (1994). Preparation and characterization of poly(L-lactic acid) foams. Polymer 35:1068-1077; Nam, Y. S. and Park, T. G. (1999a). Biodegradable polymeric microcellular foams by modified thermally induced phase separation method. Biomaterials 20:1783-1790; Nam, Y. S., Yoon, J. J. and Park, T. G. (2000). A novel fabrication method of macroporous biodegradable polymer scaffolds using gas foaming salt as a porogen additive. Journal of Biomedical Materials Research (Applied Biomaterials) 53:1-7).

Optimal pore sizes for bone formation generally range from 40 to 400 microns. Several methods have been developed to create highly porous cell carriers , including fiber bonding (Mikos et al. 1993 above), solvent casting/particulate leaching (Mikos et al. 1993 above; Mikos et al. 1994 above), gas foaming (Nam et al. 2000 above) and phase separation (Lo, H., Ponticiello, M. S. and Leong, K. W. (1995). Fabrication of controlled release biodegradable foams by phase separation. Tissue Engineering 1:15-28; Whang, K., Thomas, C. H., Healy, K. E. and Nuber, G. (1995). A novel method to fabricate bioabsorbable scaffolds. Polymer 36:837-842; Lo, H., Kadiyala, S., Guggino, S. E. and Leong, K. W. (1996). Poly(L-lactic acid) foams with cell seeding and controlled-release capacity. Journal of Biomedical Materials Research 30:475-484; Schugens, C., Maquet, V., Grandfils, C., Jerome, R. and Teyssie, P. (1996). Polylactide macroporous biodegradable implants for cell transplantation II. Preparation of polylactide foams for liquid-liquid phase separation. Journal of Biomedical Materials Research 30:449-461; Nam and Park 1999 above). Of these methods, fiber bonding, solvent casting/particulate leaching, gas foaming/particulate leaching and liquid-liquid phase separation produce large, interconnected pores to facilitate cell seeding and migration. The fiber bonding, solvent casting/particulate leaching and gas foaming/particulate leaching methods exhibit clinically acceptable biocompatibility.

All of the methods described herein, as well as all art-recognized methods for manufacturing cell carriers, must be appropriately modified to remove organic solvents, which could reduce the ability of cells to form new tissues *in vivo*. Some methods of manufacturing the cell carriers of the present invention are described herein by way of example and not limitation.

For example, as set forth above, one technique used for constructing three-dimensional cell carriers is known as "melt molding," wherein a mixture of fine PLGA powder and gelatin microspheres is loaded in a Teflon.RTM. mold and heated above the glass-transition temperature of the polymer. The PLGA-gelatin composite is removed from the mold and gelatin microspheres are leached out by selective dissolution in distilled de-ionized water. Other cell carrier manufacturing techniques include polymer/ceramic fiber composite foam processing, phase separation, and high-pressure processing.

Another technique for manufacturing cell carriers is known as "solvent-casting and particulate-leaching." In this technique, sieved salt particles, such as sodium chloride crystals, are disbursed in a PLLA/chloroform solution which is then used to cast a membrane. After evaporating the solvent, the PLLA/salt composite membranes are heated above the PLLA melting temperature and then quenched or annealed by cooling at controlled rates to yield amorphous or semi-crystalline forms with regulated crystallinity. The salt particles are eventually leached out by selective dissolution to produce a porous polymer matrix.

Yet another technique for manufacturing the cell carriers of the present invention is known as "fiber bonding", and involves preparing a mold in the shape of the desired cell carrier and placing fibers, such as polyglycolic acid (PGA) into the mold and embedding the PGA fibers in a poly (L-lactic acid) (PLLA)/methylene chloride solution. The solvent is evaporated, and the PLLA-PGA composite is heated above the melting temperatures of both polymers. The PLLA is then removed by selective dissolution after cooling, leaving the PGA fibers physically joined at their cross-points without any surface or bulk of modifications and maintaining their initial diameter. Fiber bonding is particularly useful for fabrication of thin cell carriers.

Another technique may be solid freeform fabrication (SFF) which refers to computer-aided-design and computer-aided-manufacturing (GAD/CAM) methodologies which have been used in industrial applications to quickly and automatically fabricate arbitrarily complex shapes. SFF processes construct shapes by incremental material buildup and fusion of cross-sectional layers. In these approaches, a three-dimensional (3D) CAD model is first decomposed, or "sliced", via an automatic process planner into thin cross-sectional layer representations which are typically 0.004 to 0.020 of an inch thick. To build the physical shape, each layer is then selectively added or deposited and fused to the previous layer in an automated fabrication machine. These and other cell carrier manufacturing techniques are discussed in U. S. Patent No. 6,143,293, the entire contents of which are incorporated herein by this reference.

In accordance with another aspect of the present invention, the cell carrier portion may comprise a substance for cellular control, such as a chemotactic substance for influencing cell-migration, an inhibitory substance for influencing cell-migration, a mitogenic growth factor for influencing cell proliferation, a growth factor for influencing cell differentiation, and factors which promote angiogenesis (formation of new blood vessels). Cellular control substances may be located at one or more predetermined locations on the cell carrier. For example, substances that generally inhibit or otherwise reduce cellular growth and/or differentiation may be located on one surface of the cell carrier (*e.g.*, the surface that will be in proximity to the area within the area of intended bone formation where bone growth is not desired). Similarly, substances that generally promote or otherwise enhance cellular growth and/or differentiation may be located on one surface of the cell carrier (*e.g.*, the surface that will be in proximity to the area of intended bone formation). Additionally, the inhibiting and promoting substances may be interspersed through the cell carrier at predetermined locations to help influence rates of cellular growth at different regions over the surface of the device.

Preferably, an appropriate cell carrier portion of the device will be pre-formed into specific shapes, configurations and sizes that conform to the shape of the cell carrier containment portion by the manufacturer (*e.g.*, by MacroPore Biosurgery, Inc.) before packaging and sterilization. However, a pre-formed cell carrier portion can also be shaped at the time of surgery by bringing the material to its glass transition temperature, using heating iron, hot air, heated sponge or hot water bath methods. In a certain embodiment, the cell carrier could be cut and mechanically press-fit into a containment portion and held in place by the resulting interference fit. In another embodiment, the containment portion could be heated to glass transition temperature to revert to a pre-determined and pre-formed geometry, resulting in a clamping of the cell carrier, resulting in stabilization. In yet another embodiment, the cell carrier may be affixed to the containment portion by one or more appropriately sized mating resorbable or non-resorbable screw, tack or pin inserted through one or more of the apertures in said containment portion. Resorbable screws, tacks or pins can be obtained from MacroPore Biosurgery, Inc. (San Diego, CA). Alternatively, once inserted into an intended area of bone formation, the influx of water to the area could expand the cell carrier and shrink the containment portion or shrink the cell carrier and expand the containment portion, resulting in fixation. The cell carrier and the containment portion could also be affixed to each other using polymer solutions, solvents or appropriate glues.

### i. Illustrated Embodiment of a Porous Cell Carrier Portion of the Device

As shown in Figure 5, a porous cell carrier portion of the device of the invention 210 is of parabolic shape, having a top face 211, a bottom face 212, side faces 213, a front end 214 and a back end 215, and a plurality of interconnected pores 216.

### 3. Methods of Adding Regenerative Cells to a Cell Carrier Portion of the Device

The cell carrier must be appropriately seeded with the regenerative cells to optimize bone formation in the intended area of bone formation. The function of the cell carrier is to deliver cells in a manner compatible with optimal cell mediated bone formation at the intended site of bone formation. The carrier may be a solid, resorbable or non-resorbable polymer based scaffold with a fiber or sponge macrostructure into which cells are seeded, or a hydrogel, *e.g.*, fibrin, polyethylene glycol, in which cells are encapsulated. The carrier may be modified by a chemical, peptide, protein, or gene to support cell attachment , *e.g.*, RGD, cell differentiation, *e.g.*, CBFA-1gene, calcification, e.g., phosphor group. The cells can either be seeded *in vivo* by injection or other appropriate method at the treatment site, or seeded *ex vivo*.

*Ex vivo* seeding methods for solid based scaffolds encompassed in this invention include art-recognized methods such as static seeding (or capillary action), injection, dynamic seeding, including uni- and bidirectional perfusion, spinner flask, orbital or random shaker, rotating or end-over-end bioreactor seeding, and vacuum assisted seeding, or a mixture of the methods, to name a few. Seeding methods for natural or synthetic gel based carrier encompassed in this invention include art-recognized methods such as mixing the cells with the liquid components of the gel based carrier and forming a gel by physical interaction crosslinks (e.g. hydrogen, van der waals forces, polar forces, ionic bonds) or covalent crosslinks. Seeding using gel based carriers can be accomplished either *in vivo* or ex *vivo*. If the cell carrier portion is of a particulate form, the seeding methods may be as described above, or may be different. For example, the cells may simply be mixed with the cell carrier portion and place, press fit or inject the cell/particulate mixture into the intended area of bone formation, such as in the center of the containment device and/or around the containment device.

The cells to be incorporated into the carrier may be previously incubated or cultured with any substance that may promote the cells' ability to adhere to the carrier or to stimulate the cells' bone forming capacity, the latter exemplified by bone growth promoting proteins or genes (*e.g.*) BMPs, or other bone growth regulatory molecules (*e.g.*, CBFA-1). In an *ex vivo* seeding approach, the cell laden cell carriers are then placed into the intended area of bone formation with the cell carrier containment portion of the present invention to induce bone formation. Once implanted, host cell influx and ADC proliferation, differentiation, osteoid deposition, mineralization, and, in the case of resorbable scaffolds, concomitant reabsorption of the scaffold, proceeds.

These and other methods of seeding the cell carriers are disclosed in, for example, S. L. Ishaug, G. M. Crane, M. J. Miller, A. W. Yasko, M. J. Yaszemski, and A. G. Mikos. Bone formation by three-dimensional stromal osteoblast culture in biodegradable polymer scaffolds. J Biomed Mater Res 36 (1):17-28, 1997; H. L. Wald, G. Sarakinos, M. D. Lyman, A. G. Mikos, J. P. Vacanti, and R. Langer. Cell seeding in porous transplantation devices. Biomaterials 14 (4):270-278, 1993; N. S. Dunkelman, M. P. Zimber, R. G. LeBaron, R. Pavelec, M. Kwan, and A. F. Purchio. Cartilage production by rabbit articular chondrocytes on polyglycolic acid scaffolds in a closed bioreactor system. Biotechnol Bioeng 46:299-305, 1995; L. E. Freed, A. P. Hollander, I. Martin, J. R. Barry, R. Langer, and G. Vunjak-Novakovic. Chondrogenesis in a cell-polymer-bioreactor system. Exp Cell Res 240 (1):58-65, 1998; R. E. Schreiber, N. S. Dunkelman, G. Naughton, and A. Ratcliffe. A method for tissue engineering of cartilage by cell seeding on bioresorbable scaffolds. Ann N Y Acad Sci 875:398-404, 1999; G. Vunjak-Novakovic, et al (1999) van Wachem PB, tronck JW, oers-Zuideveld R, ijk F, and ildevuur CR. Vacuum cell seeding: a new method for the fast application of an evenly distributed cell layer on porous vascular grafts. Biomaterials 11 (8):602-606, 1990; L. E. Freed, J. C. Marquis, G. Vunjak-Novakovic, J. Emmanual, and R. Langer. Composition of cell-polymer cartilage implants. Biotechnol.Bioeng. 43:605-614,1994; J. L. van Susante, P. Buma, G. J. van Osch, D. Versleyen, P. M. van der Kraan, W. B. van der Berg, and G. N. Homminga. Culture of chondrocytes in alginate and collagen carrier gels. Acta Orthop Scand 66 (6):549-56, 1995; J. Elisseeff, W. McIntosh, K. Anseth, S. Riley, P. Ragan, and R. Langer. Photoencapsulation of chondrocytes in poly(ethylene oxide)-based semi-interpenetrating networks. J.Biomed.Mater.Res. 51:164-171, 2000.

Once seeded on the cell carrier, the device can be inserted into an intended area of bone formation in the recipient. Alternatively, the cells can be seeded onto the cell carrier subsequent to insertion of the device (the cell carrier portion and the containment portion) into an intended area of bone formation in the recipient. Once the device is inserted, *in-vivo* cellular proliferation and, in the case of resorbable cell carriers, concomitant reabsorption of the cell carrier, proceeds.

In accordance with another aspect of the invention, the cell carrier portion may further comprise a substance (*e.g.*, a chemical or biological molecule) to facilitate cell seeding, for example, an adhesion peptide or other biological molecule to enhance cell attachment, a substance that alters the hydrophilicity of the cell carrier, a surface charge, a polar molecule, or a surfactant or wetting agent to increase cell attachment or increase fluid uptake into the cell carrier. The substance used to facilitate cell seeding may be located on the surface of the cell carrier pores, the outer regions of the cell carrier, interspersed in the bulk of the cell carrier material, at predetermined locations, or any combinations thereof.

### i. Illustrated Embodiment of a Cell Carrier Seeded with Regenerative Cells

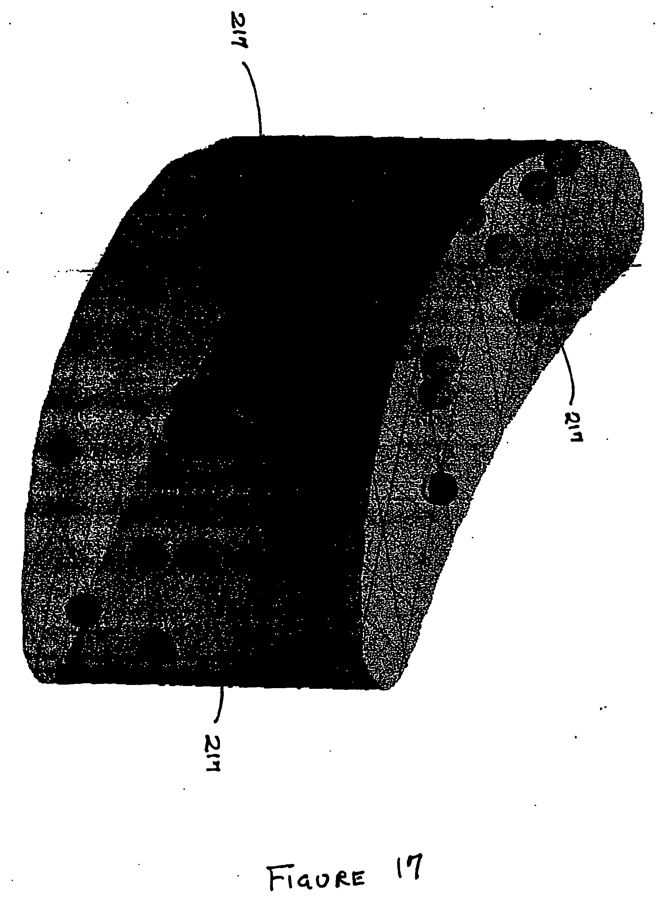

As shown in Figure 17, a porous cell carrier portion of the device of the invention 210 is in the shape of a parabolic sleeve, having a top face 211, a bottom face 212, side faces 213, a front end 214 and a back end 215, a plurality of interconnected pores 216 and seeded with regenerative cells such as stem cells and progenitor cells 217.

### 4. Methods for Manufacturing a Cell Carrier Containment Portion of the Device

In a broad embodiment, the cell carrier containment portion of the device ("the containment portion") may be constructed from any material effective to govern bone formation when used in accordance with the methods disclosed herein. Preferably, the containment portion is resorbable. For example, the containment portion may be constructed from any biodegradable material, such as resorbable polymers. In preferred embodiments, the containment portion is constructed with materials that do not induce a significant antigenic or immunogenic biological response.

In accordance with one embodiment, non-limiting polymers which may be used to form containment portion of the present invention include polymers (*e.g.*, copolymers) of lactide (L, D, DL, or combinations thereof), glycolide, trimethylene carbonate, caprolactone and/or physical and chemical combinations thereof. In one embodiment, the containment portion is comprised of a polylactide, which can be a copolymer of L-lactide and D,L-lactide. For example, the copolymer can comprise about 60-80% of L-lactide and about 20-40% of D,L-lactide, and in a preferred embodiment the copolymer comprises 70:30 poly (L-lactide-coD,L-lactide).

In one embodiment, the containment portion is formed by polymers (*e.g.*, homo and/or copolymers) derived from one or more cyclic esters, such as lactide (*i.e.*, L, D, DL, or combinations thereof), epsilon-caprolactone and glycolide. For instance, in one embodiment, the containment portion can comprise about 1 to 99% epsilon-caprolactone, or in another embodiment can comprise 20 to 40% epsilon-caprolactone. In one example, a membrane comprises 65:35 poly (L-lactide-co-epsilon-caprolactone). In other embodiments, butyrolactone, valerolactone, or dimethyl propiolactone can be used with or as a substitute for epsilon-caprolactone. In another embodiment, the containment portion can comprise a copolymer including lactide and glycolide which is resorbed into the body more rapidly than the above-mentioned poly (L-lactide-co-D,L-lactide).

The polymers (*e.g.*, co-polymers) of the present invention require relatively simple chemical reactions and formulations. The containment portion of the present invention is preferably smooth and substantially non-porous. Preferably, the containment portion may be formed into specific shapes, configurations and sizes by the manufacturer (*e.g.*, MacroPore Biosurgery, Inc.) before packaging and sterilization. However, a pre-formed containment portion can also be shaped at the time of surgery by bringing the material to its glass transition temperature, using heating iron, hot air, heated sponge or hot water bath methods.

The containment portion may have a thickness of about 0.1 millimeters to about 5 millimeters. Preferably, the containment portion has a thickness of about 2 millimeters to about 4 millimeters. The material of the containment portion may be flexible enough to conform to a curvature of a bone and sufficient strength to reduce macro-motion of the bone defect and limit transmission of surrounding motion into the interior space when the device is attached to the targeted area. The containment portion is adapted for protecting the target area from a prolapse of adjacent soft tissues into the target area during bone formation and, further, is adapted for preventing resorption of bone due to stress shielding associated with insertion of the device after the desired new bone formation has occurred.

The containment portion may be provided in any shape which may effectively promote bone growth. In one embodiment, the containment portion may be provided in a parabolic shape. In another embodiment, the containment portion may be provided in a rectangular shape. In yet another embodiment, the containment portion may be provided in a trapezoidal shape. In a further embodiment, the containment portion may be provided in a cylindrical shape. The containment portion of the present invention may be sufficiently flexible to conform around anatomical structures.

In one embodiment, the containment portion comprises two opposing surfaces. On one side of the containment portion, there is a bone-growing substantially-smooth side or surface, and on the other side there is a non-bone growing substantially-smooth side or surface. Preferably, the bone-growing substantially-smooth side is positioned to face the area of intended bone formation, and the non-bone growing substantially-smooth side is positioned to face the area where bone formation is not desired..

As used herein, the term "non-porous" refers to a material which is generally water tight and, in accordance with a preferred embodiment, not fluid permeable. However, in a modified embodiment of the invention micro-pores (*i.e.*, fluid permeable but not cell permeable) may exist in the containment portion of the present invention, to the extent, for example, that they do not substantially disrupt the smoothness of the surfaces of the containment portion to cause scarring of tissue. In substantially modified embodiments for limited applications, pores which are cell permeable but not vessel permeable may be manufactured and used. As presently preferred, the containment portion is manufactured using an injection molding procedure to yield a substantially non-porous containment portion. The containment portion may also be manufactured using extrusion or compression molding procedures. The containment portion may have a semi-rigid construction, and are fully contourable when heated to approximately 55 degrees Celsius.

Accordingly, in particular embodiments, the substantially smooth surface of the containment portion may be comprised of a plurality of apertures with a diameter in a range between 20 microns to 3000 microns. The apertures are adapted for allowing a proliferation of vasculature and connective tissue cells therethrough, while preventing the prolapse of adjacent soft tissues into the biological tissue defect. In addition, the apertures allow vital contributions of blood vessels from surrounding tissues, musculature, and periosteum into the target area. Blood vessels invading the target area through the device can greatly enhance the generation of new bone. The ability for capillaries from surrounding tissues to proliferate through the device can help prevent migrating cells from the osseous bed and the periosteum from outstripping their proliferating blood supply. This proliferation of blood vessels can increase the potential of bone formation within a given target area.

The surface of the containment portion may further be comprised of a plurality of larger apertures with a diameter in a range of 1 to 5 millimeters. Such larger apertures in the containment portion are strategically placed to facilitate insertion of an insertion and/or fixation device for securing the device into an intended area of bone formation. The apertures can also be strategically placed for a subsequent surgical procedure in which additional regenerative cells are injected through the aperture in the event of a delayed union or non-union. It is understood in the art that the size of the larger apertures on the containment portion may vary in accordance with the type of insertion tool and/or fixation device used to secure the device in the target area. In addition to art-recognized tools for insertion of the device, the device may be frictionally secured in the target area using, fixation devices such as clamps, staples, screws, sutures, tacks, pins and other conventional means. In a preferred embodiment, the fixation devices are resorbable, such as those manufactured by MacroPore Biosurgery, Inc. In alternative embodiments, a combination of insertion devices or fixation devices may be used.

It is understood in the art that desired resorption rates will vary based on the particular therapeutic application. It is believed that a containment portion of the present invention having a thickness of 1 millimeters to about 5 millimeters, preferably 2-4 millimeters, should maintain its structural integrity for a period in excess of about one month, preferably in excess of about three months, more preferably for about three to six months, before substantially degrading, so that the bone forming can be achieved and optimized. Similar to the rates of resorption of the cell carrier portion of the device as set forth above, the rates of resorption of the cell carrier containment portion of the device may also be selectively controlled.

Exemplary materials and methods related to making and using all aspects of the present invention are disclosed in, for example, U.S. Patent Nos. 5,919,234, 6,280,473, 6,269,716, 6,343,531, 6,477,923, 6,391,059, 6,531,146 and 6,673,362, the contents of which are incorporated herein by this reference. Resorbable containment portions of the devices of the present invention are available from MacroPore Biosurgery, Inc. (San Diego, CA).

### i. Illustrated Embodiment of a Cell Carrier Containment Portion of the Device

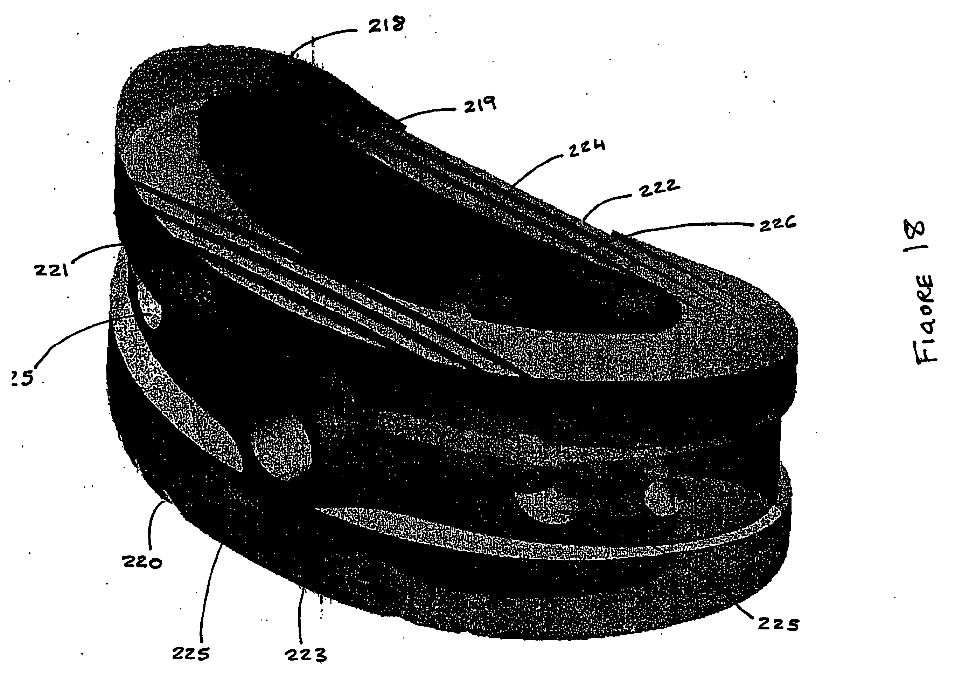

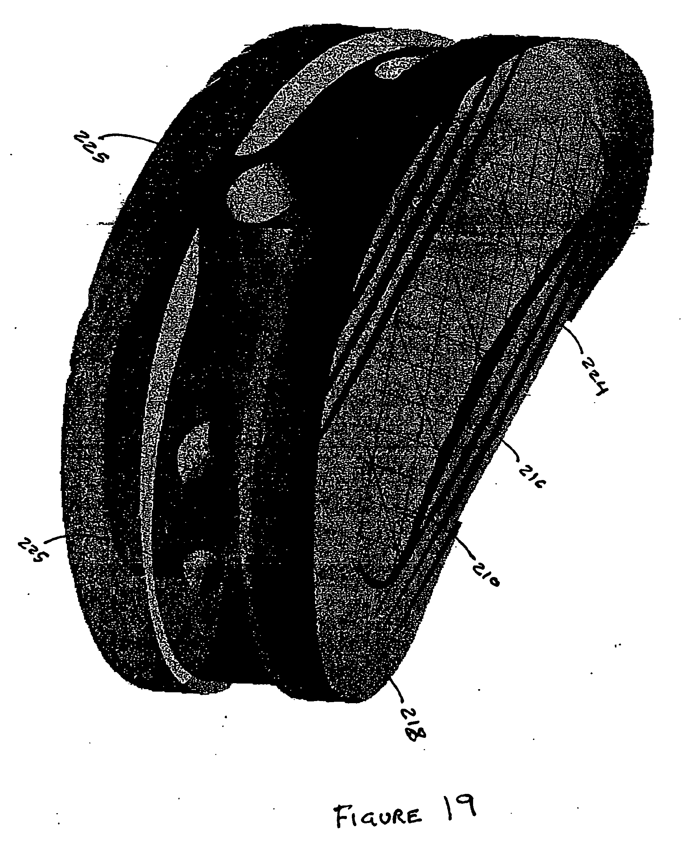

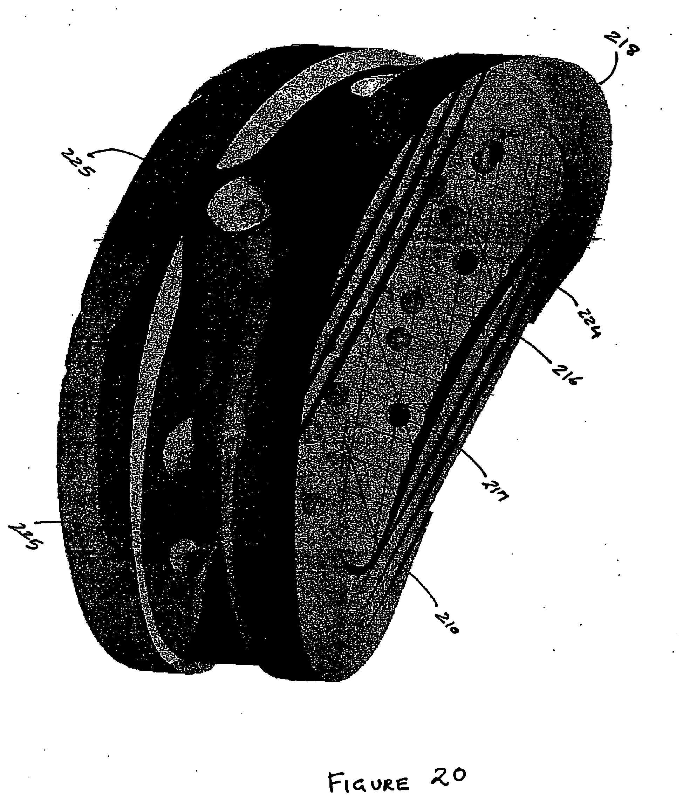

As shown in Figure 18, in one embodiment, a cell carrier containment portion of the device of the invention is of parabolic shape 218, having a top face 219, a bottom face 220, side faces 221, a front end 222 and a back end 223. The surfaces of top and bottom faces 219 and 220 each have ridges 224 to help anchor the containment portion of the device to the surrounding bone in the target area. The recesses between the ridges 224 can enhance mechanical stability that would aid in anchoring the containment portion of the device in place. The cell carrier containment portion 218 may contain apertures 225 of appropriate diameter, that can be used to screw the device into place. The apertures 225 may also be used to insert an art-recognized tool to insert the device into a target area. The cell carrier containment portion has a large center hole 226 for insertion of a cell carrier portion of the present invention. Figure 19 depicts an embodiment of the invention wherein a porous cell carrier of parabolic shape has been inserted into a containment portion of parabolic shape. Figure 20 depicts an embodiment of the invention wherein a porous cell carrier of parabolic shape seeded with the regenerative cells of the invention has been inserted into a containment portion of parabolic shape. Figure 20 depicts an embodiment of the device and the cells which is suitable for insertion into a recipient using art-recognized surgical techniques.

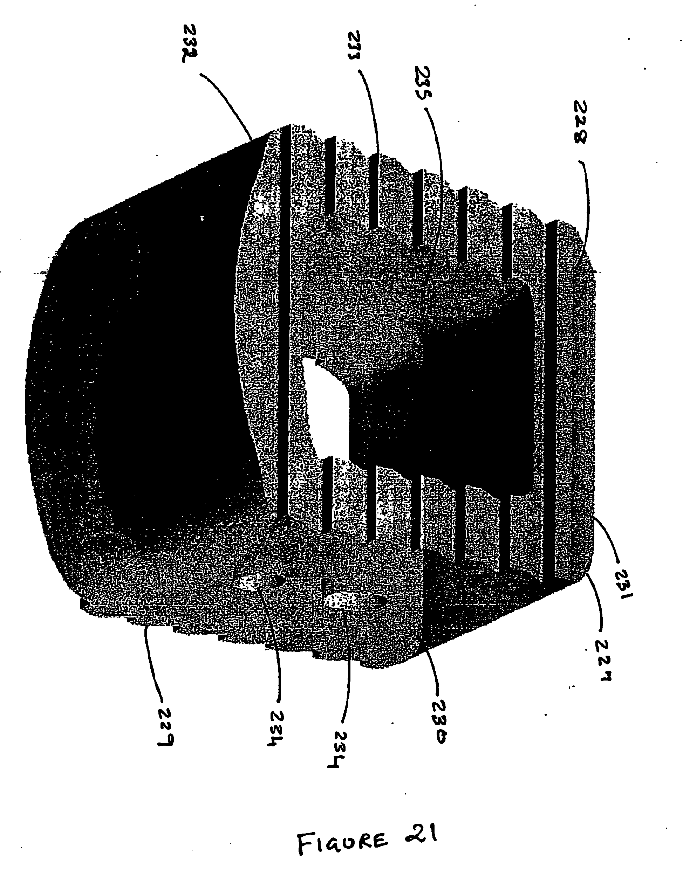

As shown in Figure 21, in one embodiment, a cell carrier containment portion of the device of the invention is of trapezoidal shape 227, having a top face 228, a bottom face 229, side faces 230, a front end 231 and a back end 232. The surfaces of top and bottom faces 228 and 229 each have ridges 233 to help anchor the containment portion of the device to the surrounding bone in the target area. The recesses between the ridges 233 can enhance mechanical stability that would aid in anchoring the containment portion of the device in place. The cell carrier containment portion 227 may contain apertures 234 of appropriate diameter , that can be used to screw the device into place. The apertures 234 may also be used to insert an art-recognized tool to insert the device into a target area. The cell carrier containment portion has a large center hole 235 for insertion of a cell carrier portion of the present invention.

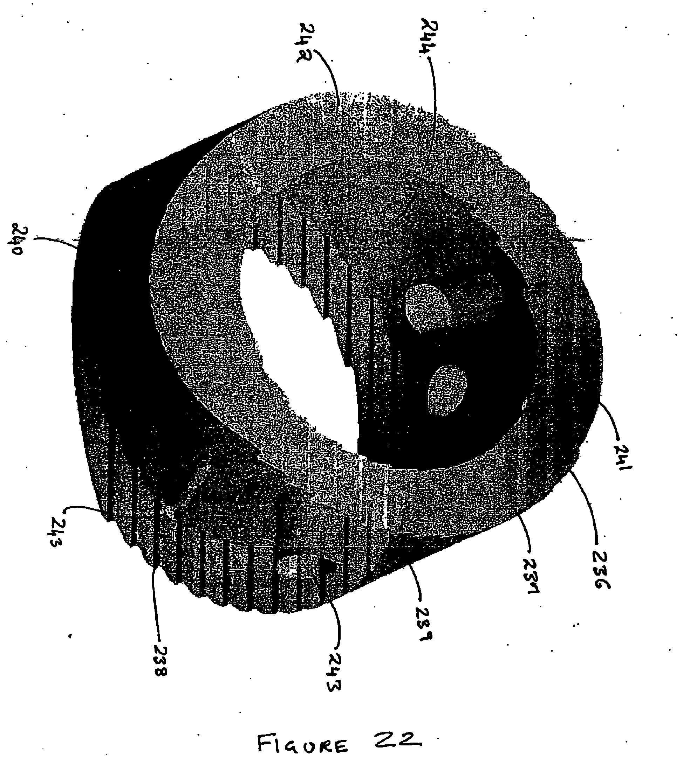

As shown in Figure 22, in one embodiment, a cell carrier containment portion of the device of the invention is of cylindrical shape 236, having a top face 237, a bottom face 238, side faces 239, a front end 240 and a back end 241. The surfaces of top and bottom faces 237 and 238 each have ridges 242 to help anchor the containment portion of the device to the surrounding bone in the target area. The recesses between the ridges 242 can enhance mechanical stability that would aid in anchoring the containment portion of the device in place. The cell carrier containment portion 236 may contain apertures 243 of appropriate diameter , that can be used to screw the device into place. The apertures 243 may also be used to insert an art-recognized tool to insert the device into a target area. The cell carrier containment portion has a large center hole 244 for insertion of a cell carrier portion of the present invention.

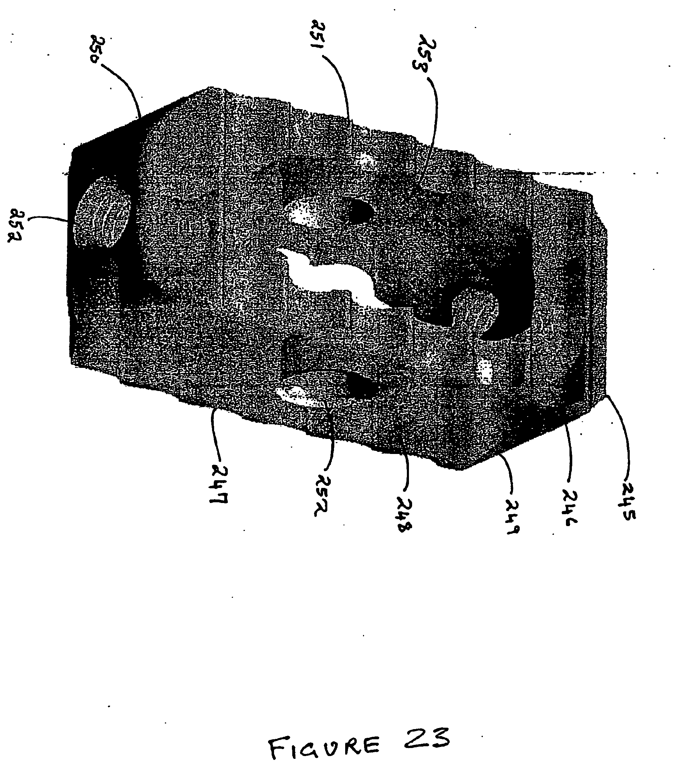

As shown in Figure 23, in one embodiment, a cell carrier containment portion of the device of the invention is of rectangular shape 245, having a top face 246, a bottom face 247, side faces 248, a front end 249 and a back end 250. The surfaces of top and bottom faces 246 and 247 each have ridges 251 to help anchor the containment portion of the device to the surrounding bone in the target area. The recesses between the ridges 251 can enhance mechanical stability that would aid in anchoring the containment portion of the device in place. The cell carrier containment portion 245 may contain apertures 252 of appropriate diameter , that can be used to screw the device into place. The apertures 252 may also be used to insert an art-recognized tool to insert the device into a target area. The cell carrier containment portion has a large center hole 253 for insertion of a cell carrier portion of the present invention.

### 5. Methods for Using the Device for Bone and/or Cartilage Formation

As exemplified herein, the adipose derived regenerative cells of the present invention are a rich source of bone or cartilage precursor cells and they have been shown to form bone and cartilage *in vivo*. Accordingly, the device of the present invention can be used to treat bone related disorders, e.g., by promoting bone and/or cartilage formation.

Accordingly, in one aspect of the present invention, regenerative cells are extracted from a donor's adipose tissue, are added to a porous resorbable cell carrier surrounded by a resorbable cell carrier containment portion, and the combination of the device and the cells is inserted into an intended area of bone formation in a recipient to thereby elicit a therapeutic benefit by promoting bone and/or cartilage formation. In a preferred embodiment the cells are extracted from the adipose tissue of the person into whom they are to be implanted, thereby reducing potential complications associated with antigenic and/or immunogenic responses to the transplant. Patients are typically evaluated to assess bone related disorders by one or more of the following procedures performed by a physician or other clinical provider: patient's health history, physical examination, and objective data including but not limited to radiographs.

In one embodiment, the harvesting procedure is performed prior to the patient receiving any products designed to reduce blood clotting in connection with treatment for the bone related disorder, *e.g.*, spinal fusion surgery. However, in certain embodiments, the patient may have received drugs known to affect coagulation (*e.g.*, aspirin) prior to the harvesting procedure. In addition, one preferred method includes collection of adipose tissue prior to any attempted spinal fusion procedure. However, as understood by persons skilled in the art, the timing of collection is expected to vary and will depend on several factors including, among other things, patient stability, patient coagulation profile, provider availability, and quality care standards. Ultimately, the timing of collection will be determined by the practitioner responsible for administering care to the affected patient.

The volume of adipose tissue collected from the patient can vary from about 1 cc to about 2000 cc and in some embodiments up to about 3000 cc. The volume of fat removed will vary from patient to patient and will depend on a number of factors including but not limited to: age, body habitus, coagulation profile, hemodynamic stability, co-morbidities, and physician preference.

The cells may be extracted in advance and stored in a cryopreserved fashion or they may be extracted at or around the time of defined need. As disclosed herein, the cells may be administered to the patient, or applied directly to the damaged tissue, or in proximity of the damaged tissue, without further processing or following additional procedures to further purify, modify, stimulate, or otherwise change the cells. For example, the cells obtained from a patient may be administered to a patient in need thereof via the resorbable cell carrier and resorbable cell carrier containment portion without culturing the cells before administering them to the patient. In one embodiment, the collection of adipose tissue will be performed at a patient's bedside. Hemodynamic monitoring may be used to monitor the patient's clinical status.

In accordance with the invention disclosed herein, the regenerative cells can be delivered to the patient soon after harvesting the adipose tissue from the patient. For example, the cells may be administered immediately via the device after processing of the adipose tissue and obtaining a composition of regenerative cells. Ultimately, the timing of delivery will depend upon patient availability and the processing time required to process the adipose tissue. In another embodiment, the timing for delivery may be relatively longer if the cells to be re-infused to the patient are subject to additional modification, purification, stimulation, or other manipulation, as discussed herein. The number of cells administered to a patient may be related to, for example, the cell yield after adipose tissue processing. A portion of the total number of cells may be retained for later use or cyropreserved. In one embodiment of the invention, a number of cells, *e.g.*, unpurified cells, to be delivered to the patient is expected to be about 5.5 x 10⁴ cells. However, this number can be adjusted by orders of magnitude to achieve the desired therapeutic effect.

Cells obtained after disaggregation from adipose tissue may be further enriched for bone or cartilage progenitor cells by passage over a biologic based component within or separate from the device that either captures chondroprogenitor cells, osteoprecursor cells or all cells but one of these cells. If the former, then an additional step included within or external to the device would be used to release the captured cells from the biologic based component. An example of this chondroprogenitor or osteoprecursor enrichment would be a chromatographic resin to which an antibody that recognizes chondroprogenitor cells or osteoprecursor cells (*e.g.*, SH3 cells) is attached. An example of a release agent is papain or pepsin that could cleave the antibody/cell complex from the resin.

The cells may also be applied with additives to enhance, control, or otherwise direct the intended therapeutic effect. Cells may be administered following genetic manipulation such that they express gene products that are believed to or are intended to promote the therapeutic response(s) provided by the cells. Examples of manipulations include manipulations to control (increase or decrease) expression of factors promoting bone or cartilage formation, expression of developmental genes promoting differentiation into specific cartilage or bony cell lineages or that stimulate cartilage or bony cell growth and proliferation.

The active cell population may be applied to the resorbable cell carrier of the present device alone or in combination with other cells, tissue, tissue fragments, growth factors (*e.g.*, BMPs), or other additive intended to enhance the delivery, efficacy, tolerability, or function of the population. The cell population may also be modified by insertion of DNA in a plasmid or viral vector or by placement in cell culture in such a way as to change, enhance, or supplement the function of the cells for derivation of a structural or therapeutic purpose. For example, gene transfer techniques for stem cells are known by persons of ordinary skill in the art, as disclosed in (Morizono *et al.*, 2003; Mosca *et al.*, 2000), and may include viral transfection techniques, and more specifically, adeno-associated virus gene transfer techniques, as disclosed in (Walther and Stein, 2000) and (Athanasopoulos *et al.*, 2000). Non-viral based techniques may also be performed as disclosed in (Muramatsu *et al.*, 1998).

In another aspect, the cells could be combined with a gene encoding a pro-chondroprogenitor or pro-osteogenic growth factor(s) which would allow cells to act as their own source of growth factor during cartilage or bone formation. Genes encoding anti-apoptotic factors or agents could also be applied. Addition of the gene (or combination of genes) could be by any technology known in the art including but not limited to adenoviral transduction, "gene guns," liposome-mediated transduction, and retrovirus or lentivirus-mediated transduction, plasmid, adeno-associated virus. Cells could be implanted along with a carrier material bearing gene delivery vehicle capable of releasing and/or presenting genes to the cells over time such that transduction can continue or be initiated *in situ*. Particularly when the cells and/or tissue containing the cells are administered to a patient other than the patient from whom the cells and/or tissue were obtained, one or more immunosuppressive agents may be administered to the patient receiving the cells and/or tissue to reduce, and preferably prevent, rejection of the transplant. As used herein, the term "immunosuppressive drug or agent" is intended to include pharmaceutical agents which inhibit or interfere with normal immune function. Examples of immunosuppressive agents suitable with the methods disclosed herein include agents that inhibit T-cell/B-cell costimulation pathways, such as agents that interfere with the coupling of T-cells and B-cells via the CTLA4 and B7 pathways, as disclosed in U.S. Patent Pub. No. 20020182211. A preferred immunosuppressive agent is cyclosporine A. Other examples include myophenylate mofetil, rapamicin, and anti-thymocyte globulin. In one embodiment, the immunosuppressive drug is administered with at least one other therapeutic agent. The immunosuppressive drug is administered in a formulation which is compatible with the route of administration and is administered to a subject at a dosage sufficient to achieve the desired therapeutic effect. In another embodiment, the immunosuppressive drug is administered transiently for a sufficient time to induce tolerance to the ADC of the invention.

In certain embodiments of the invention, the cells are administered to a patient with one or more cellular differentiation agents, such as cytokines and growth factors. Examples of various cell differentiation agents are disclosed in (Gimble *et al.*, 1995; Lennon *et al.,* 1995; Majumdar *et al.*, 1998; Caplan and Goldberg, 1999; Ohgushi and Caplan, 1999; Pittenger *et al.*, 1999; Caplan and Bruder, 2001; Fukuda, 2001; Worster *et al.,* 2001; Zuk *et al.,* 2001).

### i. Methods of Using the Device for Intervertebral Disc Repair or Interbody Spinal Fusion Procedures

As set forth herein, back pain remains a major public health problem, especially among aged people. Persistent and severe back pain often causes debility and disability. This pain is closely associated with intervertebral disc abnormalities of the spine. By way of background, the human spine is a flexible structure comprised of thirty-three vertebrae. Intervertebral discs separate and cushion adjacent vertebrae, act to minimize loading applied to the spine, and allow motion between the vertebrae (flexion, extension, rotation). An intervertebral disc comprises two major components: the nucleus pulposus and the annulus fibrosis. The nucleus pulposus is centrally located in the disc and occupies 25-40% of the disc's total cross-sectional area. The matrix of the nucleus pulposis is largely composed of a thick gelatinous substance made up of water, polysaccharides and protein molecules. The annulus fibrosis surrounds the nucleus pulposus and resists torsional and bending force applied to the disc. Vertebral end-plates separate the disc from the vertebrae on either side of the disc. The outer ring layer structurally consists mainly of overlapping orthogonal layers of fibrocartilage which are mainly made up of type I collagen in an extracellular matrix. This crisscrossing pattern imparts outstanding structural resilience to the pressure applied by the adjacent vertebrae. The inner core, located in the middle of the annulus fibrosis, consists of a few cells embedded in a matrix rich in hyaluronic acid and type II collagen and acts as a biomechanical shock absorber.

As a result of exertion, injury, illness, accident or abuse, one or more of the vertebrae and/or one or more discs may become damaged. Specifically, disorders of the vertebrae and discs include but are not limited to 1) disruption of the disc annulus such as annular fissures; 2) chronic inflammation of the disc; 3) localized disc herniations with contained or escaped extrusions; 4) relative instability of the vertebrae surrounding the disc; and 5) trauma.

Though intervertebral disc cells in both the annulus fibrosus and the nucleus pulposus only constitute a small percent of the disc volume, they have been shown to have the capacity to a synthesize a proteoglycan rich tissue (similar to cartilage) which comprises the extracellular matrix of the disc. In diseased discs, a decrease in intervertebral disc cell density in the nucleus pulposus is accompanied by a reduced production of the hydrophilic extracellular matrix. A reduction in intervertebral disc cells thus results progressively in the structural decrease of the intervertebral disc space, contributing to degenerative disc disease (DDD). Traditionally, cartilage is classified as either hyaline, elastic, or fibrocartilage. The gel-like anatomical characteristics of the nucleus pulposus (as well as it unique notochordal embryonic origin)does not fit into any of the cartilagenous categories, and is not classically considered a cartilage. It is, nonetheless, permeated with a proteogylcan rich extracellular matrix similar to that of cartilage. Based on the ability of the regenerative cells of the present invention to produce cartilage and bone, these cells can also stimulate the formation of the cartilage like proteoglycan rich extracellular matrix to thereby restore the intervertebral disc.

Various approaches have been developed to treat back pain. Minor back pain can be treated with medication and other non-invasive therapy. Mild to moderate degenerated discs may be treated by restoring the damages tissues within the disc, nucleous pulposis and annulus fibrosis. Accordingly, the regenerative cells of the invention may be used to stimulate cartilage development and thereby restore the intervertabral discs at various stages of degeneration. For example, the regenerative cells of the invention may be delivered via injection in a physiologic solution directly into the disc's nucleus pulposis in cases of mild degeneration, wherein the disc is largely still grossly intact, but beginning to break down. In cases of moderate disc degeneration, where the disc is macroscopically degenerating, the regenerative cells could be mixed with a hydrogel liquid that gels in situ when injected into the nucleus pulposis. The gelation could be thermal or thermochemically mediated.

However, it is often necessary to remove at least a portion of the damaged and/or malfunctioning back component. For example, when a disc becomes ruptured, a discectomy surgical procedure can be performed to remove the ruptured disc and to fuse the two vertebrae between the removed disc together. Details regarding typical implementations of methods for fusing vertebrae are disclosed in U.S. Patent Nos. 6,033,438 and 5,015,247, the contents of which are incorporated in their entireties herein by reference.

Spinal fusion is indicated to provide stabilization of the spinal column for disorders such as structural deformity, traumatic instability, degenerative instability, and post resection iatrogenic instability. Fusion, or arthrodesis, can thus be achieved, for example, by the formation of an osseous bridge between adjacent motion segments. The fusion can be accomplished either anteriorly between contiguous vertebral bodies or posteriorly between consecutive transverse processes, laminae or other posterior aspects of the vertebrae. Surgical techniques for spinal fusion are well-known in the art. The present invention provides an inherent advantage over the art-recognized techniques because it eliminates the need for a second surgery to harvest autologous bone graft. In addition, typically, bone graft materials and/or growth factors are often needed to promote spinal fusions. The present invention provides autologous regenerative cells that promote bone growth cost-effectively and without fear of graft rejection.

Surgical methods for spinal fusion and other surgical techniques for treating bone related disorders are well known to the ordinarily skilled artisan. All such art recognized techniques are intended to be encompassed by the present invention.

The present invention is further illustrated by the following examples which in no way should be construed as being further limiting, The contents of all cited references, including literature references, issued patents, published patent applications, and co-pending patent applications, cited throughout this application are hereby expressly incorporated by reference.

### EXAMPLES

The ADC or regenerative cells used throughout the examples set forth below can be obtained by the method(s) described in the instant disclosure and/or the method(s) described in U.S. Application Serial No. 10/316,127, entitled SYSTEMS AND METHODS FOR TREATING PATIENTS WITH PROCESSED LIPOASPIRATE CELLS, filed December 9, 2002, which claims priority to U.S. Provisional Application Serial No. 60/338,856, filed December 7, 2001, as well as well as the methods described in U.S. Application No. entitled, SYSTEMS AND METHODS FOR SEPARATING AND CONCENTRATING REGENERATIVE CELLS FROM TISSUE, filed June 25, 2004, which claims priority to U.S. Application Serial No. 10/316,127, entitled SYSTEMS AND METHODS FOR TREATING PATIENTS WITH PROCESSED LIPOASPIRATE CELLS, filed December 9, 2002, which are all commonly assigned and the contents of all of which are expressly incorporated herein by this reference.

### EXAMPLE 1: Human Adipose Tissue is a Source of Osteoprogenitor Cells

### Methods and Materials:

*Liposuction:* Human adipose tissue was obtained from individuals undergoing elective lipoplasty (liposuction). In general the liposuction procedure involved creation of a 0.5cm incision in the skin of the abdomen and/or thigh followed by infiltration of the subcutaneous space with tumescent solution (saline supplemented with lidocaine and epinephrine). After approximately 10 minutes the subcutaneous adipose tissue was aspirated through a cannula (3-5mm diameter according to Physician preference) under machine-assisted suction. Adipose tissue, blood, and tumescent solution (lipoaspirate) was collected into a disposable container and stored at 4°C during transport to the laboratory.

*Tissue Digestion:* Adipose tissue was washed several times with warm (37°C) saline to remove excess blood and free lipid. The tissue was then digested at 37°C with Blendzyme collagenase (Blendzyme 1 for 45 minutes or Blendzyme 3 for 20 minutes; Roche, Indianapolis, IN) in approximately two volumes of saline. Constant agitation was applied to ensure adequate mixing of tissue and medium throughout the digestion. After digestion cells non-buoyant cells were removed and washed three times to remove residual enzyme. Cells were counted using a vital dye (propidium iodide:) on a hemocytometer using a fluorescence microscope

*CFU-AP Assay:* Washed cells plated in duplicate at 1,000, 5,000, and 25,000 cells/well in six multiwell plates (Corning, New York). Osteogenic differentiation was induced by culturing cells for 3 weeks in medium consisting of α-MEM (Cellgro, Herndon, VA), 10% FBS/5% Horse serum/1% Antibiotic-antimycotic solution (Omega Scientific, Tarzana, CA) supplemented with 0.1 µM Dexamethasone (Sigma, St. Louis, MO), 10 mM β-glycerophosphate (Sigma, St. Louis, MO) and 50 µM L-Ascorbic acid 2-phosphate (Sigma, St. Louis, MO). After the first week of induction the culture media was changed weekly with Osteogenic Medium without dexamethasone. To detect Alkaline Phosphatase (AP) activity, culture plates were rinsed with PBS and stained with 1% Napthol AS-BI phosphate (Sigma, St. Louis, MO) and 1 mg/ml Fast Red TR (Sigma, St. Louis, MO) for 20 min at 37°C. After staining, the cells were washed with PBS and fixed with 10% Buffered Formalin Phosphate (Fisher Scientific, Pittsburgh, PA) for 15 min. Colonies consisting of more than 50 cells with positive AP staining were defined as CFU-AP. The number of CFU-AP was counted in each well and the average number of CFU-AP from the duplicate sample was calculated.

*Alkaline Phosphatase Assay:* Total alkaline phosphatase activity in day 21 cultures of adipose tissue-derived cells was measured using a colorimetric assay (Sigma Diagnostics, St. Louis, MO). Briefly, day 21 cultures were harvested by scraping, cells were resuspended in saline, and lysed by sonication. Cell lysates were then incubated with p-nitropheyl phosphate at pH 10.2 for 15 minutes at 30°C. Absorbance at 405nm was determined using a Turner SP-830 spectrophotometer (Turner Designs, Sunnyvale, CA).

### Results:

*Donor Characteristics:* The characteristics of the donor population is shown in Table A below. This population is representative of the population undergoing cosmetic lipoplasty. Data from the American Society for Aesthetic Plastic Surgery indicate that 384,626 liposuction procedures were performed in the USA in 2003 making this the most common cosmetic surgery procedure for both men and women. Women accounted for 84% of all lipoplasty procedures (81% in the present study). Approximately half of all patients undergoing lipoplasty in 2003 were between 35 and 50 years of age; median age in this study 46.

| Table A: Donor Characteristics | | | | | |
|---|---|---|---|---|---|
| Gender | 44F | 10M | | | |
| Median Age | 46 (range 19-72) | | | | |
| Mean Volume of Lipoaspirate | | | 510 ±47ml (range 50-1,500) | | |
| Time from Liposuction to Tissue Processing | | | | <6 hours | 17 |
| | | | | Overnight | 37 |
| Body Mass Index | Normal | | 20 | | |
| | Overweight | | 12 | | |
| | Obese | | 6 | | |
| | Unknown | | 16 | | |

*Development and Validation of the CFU-AP Assay:* Tissue processing yielded an average of 7.3±0.1x10⁵ viable non-buoyant nucleated cells per unit volume (ml) of adipose tissue. These cells were then plated in CFU-AP assays at multiple densities in order to validate this assay for this cell source. Further, we have demonstrated that CFU-AP count is linear with cell number plated across the range of 1,000 to 50,000 cells/well. The range of frequency detected is such that for standard assays we routinely plate cells in triplicate at two different cell concentrations in order to ensure that we have cultures in which colony number per well falls into the range of 10-60 per well.

### Osteoprogenitor frequency and yield

Using this approach we tested adipose tissue obtained from 54 donors (Table A). The data shows that CFU-AP frequency in samples processed from fresh tissue (tissue processed within six hours of collection) was significantly higher than that of samples processed on the day following tissue collection (2.0±0.3 CFU-AP/100 cells compared with 0.6±0.2 CFU-AP/100 cells; p=0.001). CFU-AP frequency did not correlate with donor age or gender.

The average yield of osteoprogenitor cells was 7,225±1,760 CFU-AP/ml adipose tissue (range 120-67,500). Unlike CFU-AP frequency there was a trend towards reduced CFU-AP yield per unit volume of adipose tissue with increasing donor age. However, when the upper quartile of age (>48yr) was compared with the lower age quartile (<34 yrs) the difference did not reach statistical significance (4,300±1,500 cells/ml tissue for older donors compared to 9,200±1,800 for younger donors; p=0.09).

### Alkaline Phosphatase Activity

In this study a colony was only counted for the purposes of the assay if it was alkaline phosphatase-positive and contained at least 50 cells. Colony size varied considerably with some colonies consisting of several thousand cells. Total alkaline phosphatase activity might represent a superior way to measure osteogenic activity as it takes into consideration both total osteoblastic cell number (a function of progenitor cell proliferation) and the degree of differentiation of these cells. Thus, alkaline phosphatase assays were performed in replicate wells in 15 consecutive donors. Total alkaline phosphatase activity exhibited a linear relationship with cell number plated. When alkaline phosphatase activity (normalized to cell number plated) was then plotted against age the data demonstrate an inverse relationship between age and alkaline phosphatase activity (Figure 2; r² = 0.57).

### Osteoprogenitor Cell Yield and Body Mass Index

Data on donor height and weight were available for 38 donors. This allowed calculation of Body Mass Index (BMI) and stratification into three groups; 20 donors of normal BMI (between 18.5 and 24.9), 12 in the Overweight category (25.0-25.9), and 6 Obese donors (≥30). The yield of CFU-AP per unit volume of tissue was significantly higher in persons of normal BMI than for those who were overweight (5,279±780 in normal BMI donors versus 1,373±1,150 in overweight donors; p<0.02). Obese donors showed considerable variation in CFU-AP yield per unit volume of tissue (867-18,462 CFU-AP/ml; Figure 3). The average was higher than for overweight donors but this did not reach statistical significance (p=0.08).

### Summary:

The foregoing results indicate that human adipose tissue may be a rich source of osteprogenitor cells. Specifically, the results indicate that human adipose contains an average of approximately 7,200 osteoprogenitor cells per gram of tissue. The frequency of CFU-AP within this population is depended upon the time from tissue collection to tissue processing such that tissue processed within six hours of collection yielded a frequency of 2.0±0.3 CFU-AP/100 nucleated cells. Studies with freshly harvested human bone marrow have estimated CFU-AP yield at 200-2,000 CFU-AP/ml marrow at a frequency of approximately 1 in 50,000 (Banfi, 2001; Galotto, 1999; D'Ippolito 1999) Thus, this data indicates that human adipose tissue contains substantially more osteoprogenitor cells than a similar volume of marrow.

Adipose tissue utilized for the foregoing study was obtained using liposuction procedures which are optimized for tissue extraction and not for recovery of viable cells. However, using the systems and methods of the present invention, in combination with altered parameters for liposuction (i.e., aimed at recovery of viable cells by for example altering the amount of suction force applied and the style and size of the cannula used) will undoubtedly result in an even greater regenerative cell yield. Higher osteoprogenitor cell yield is particularly important in the context of the device of the present invention. For example, a higher frequency of osteoprogenitor cells will allow application of a higher dose and potentially greater clinical efficiency in promoting bone formation.

### EXAMPLE 2: Adipose Derived Regenerative Cells Form Cartilage and Bone In Vivo

### Methods and Materials:

### Cell Scaffolds:

### PLA scaffold fabrication and preparation:

Porous polymer scaffolds having an approximate porosity of 90% were manufactured by a solvent-casting/particulate-leaching method (see e.g., A. G. Mikos, A. J. Thorsen, L. A. Czerwonka, Bao Y., and R. Langer. Preparation and characterization of poly(L-lactic acid) foams. Polymer 35 (5):1068-1077, 1994.). Briefly, sodium chloride was used as the leachable porogen and was sieved into particles ranging in diameter of 300-500 µm. A 3% polymer solution was made by combining 70:30 poly(L-lactide-co-DL-lactide) with chloroform. The sieved salt and polymer solution were mixed in Teflon Petri dishes to make a 9:1 weight ratio of salt to polymer and the solvent was allowed to evaporate in the hood for 2 days. The salt was leached from the scaffold by immersion in water for 3 days, with water changes every 8 - 12 hours , Residual solvent was removed by placing the porous polymeric sponges under low vacuum for 2 days. Scaffolds were sized for implantation by punching 8 mm diameter implants from the polymer sheets. Scaffolds were rendered aseptic by overnight submersion in 70% ethanol and exposure to ultraviolet light, rinsed 3 times with 0.9% injectable saline to, then the residual saline was removed from the scaffold prior to cell seeding.

### Demineralized bone matrix (DBM) scaffold preparation and cell seeding:

Grafton^{®} Demineralized Bone Matrix Putty (Osteotech, New Jersey) implants were prepared for cell seeding by rinsing evenly divided portions (approximately 70mg per implant) with 0.9% injectable saline, 3 times, prior to removal of residual saline from the scaffold.

### Adipose derived regenerative cell isolation, culture, and cell seeding:

ADCs were obtained by excising the white subcutaneous adipose tissue from transgenic mice expressing Green Fluorescent Protein [C57B1/6-TgN(ACTbEGFP)1Osb]. Tissue was subjected to enzymatic digestion. Briefly, cells were washed in 1x PBS and digested in 0.075% Collagenase I (Sigma) for 45 min at 37°C shaking, then neutralized with 10% FBS (Fisher) in Dulbecco's Modified Essential Medium (Gibco), passed through a gradient of cell straining filters (100 µm, 70 µm and 40 µm), and centrifuged at 400g for 5 min. The cell pellet was resusupended in osteogenic culture medium [α-MEM (Cellgro, Hemdon, VA), 10% FBS, 1% Antibiotic-antimycotic solution (Omega Scientific, Tarzana, CA) supplemented with 0.1 µM Dexamethasone (Sigma, St. Louis, MO), 10 mM β-glycerophosphate (Sigma, St. Louis, MO) and 50 µM L-Ascorbic acid 2-phosphate (Sigma, St. Louis, MO)]. After the first 3 days of induction the culture media was changed weekly with osteogenic medium without dexamethasone. Flasks were passaged twice in a 3 week culture period. Cultured ADCs were then incubated overnight with osteogenic medium in the presence or absence of recombinant human BMP-2 (5µg/ml). Cells were then resuspended in 0.9% injectable saline (5 x 10⁷ cells/ml) and kept at 4°C until seeding onto PLA or Grafton DBM scaffolds at 2.5 x 10⁶ cells per scaffold.

### Surgical Procedure:

Athymic rats (approximately 200g) were anesthetized with ketamine/xylazine [100/10 mg/kg (TW Medical Veterinary Supply), respectively]. The rats' abdomens were shaven, swabbed with betadine and 70% isopropanol. Under sterile conditions, a full-thickness incision, approximately 1.5 cm in length through the skin of the leg close to the hip area was made. The tensor muscle was exposed via blunt dissection and a 1.0-cm incision along the tensor muscle was made, dissecting the fascia lata to expose the rectus muscle. Using a microspatula, a channel was then created, into which a single implant was placed. A single pass of 5-0 silk sutures were then used to close off the channel, followed by suturing of the tensor muscle incision with 5-0 coated Vicryl^{®} braided suture, and closure of the skin incision with 9-mm wound clips. Following surgeries, allow animals recovered from anesthesia on heating pads. Buprenorphine (0.02 - 0.05 mg/kg, subcutaneously) was administered upon awakening, in order to relieve post-surgical pain.

### Analysis:

Two weeks after surgery, rats were euthanized via CO₂ exposure, and implants were recovered. Tissues were fixed overnight in 0.4 % paraformaldehyde, embedded in paraffin, cut into 5 micron sections, then stained with Toluidine Blue or Von Kossa to visualize cartilage and mineralized tissue, respectively. Unstained sections were evaluated for the presence of green fluorescent donor cells.

### Results:

Tissues implanted with osteoconductive (DBM) or non-osteoconductive (PLA) scaffold seeded with ADCs that had been primed *ex situ* with BMP-2 contained a mixture of cartilage, osteoid, and mineralized bone. Cartilage was identified in Toluidine Blue stained sections by the presence of metachromatically stained cells and tissue. Chondrocytes were present within lacunae. These were surrounded by a proteoglycan-rich matrix. Osteoid was evident as a smooth matrix in which osteocytes were embedded. Mineralized bone was evident in Von Kossa stained sections. Early hematopoietic elements were identified in bone by the presence of cell laden medullary cavities. Cartilage was also evident in tissues with implants composed of unprimed ADCs and DBM. In contrast, no cartilage or bone was present on either scaffold type implanted without ADCs.

### Summary:

In summary, the foregoing results demonstrate that adipose derived regenerative cells have the capacity to form cartilage and bone when delivered into skeletal muscle on an osteoconductive (DBM) or non-osteoconductive (PLA) scaffold. Thus, adipose derived regenerative cells are in stimulating cartilage formation to restore the intervertebral disc. Moreover, the foregoing study supports the use of adipose derived regenerative cells in stimulating bone formation in both intervertebral body and intertransverse process spine fusion, both environments of which are surrounded by skeletal muscle. It is likely that the skeletal muscle, which contains satellite cells that are known osteoprogenitors, act in concert with adipose derived regenerative cells to stimulate robust bone formation. At a minimum, skeletal muscle provides a supportive environment in which adipose derived regenerative cells can stimulate bone formation.

### EXAMPLE 3: Regenerative Cells Form Cartilage, Bone and Skeletal Muscle in vitro

### Materials and Methods:

### Regenerative Cell Preparation

Human regenerative cells were harvested by enzymatic digestion as described in herein. Isolated regenerative cells were then separated into CD34 (+) and CD34 (-) populations using immuno magnetic beads. Each sub-population of cells were cultured in complete medium (DMEM, 10% fetal bovine serum, 5% horse serum, and 1% antibiotic, antimycotic solution) at multiple densities in multi-well culture plates. At 3 weeks, CD34 (-) clones were selected from these cultures and subcultured for approximately 2 months in one of the following culture mediums: complete medium, osteogenic medium, or chondrogenic medium.

### Analysis:

Differentiation of clonal cells towards cartilage or bone was determined by staining with alcian blue or alkaline phosphatase, respectively. Differentiation of clonal cells towards muscle was assessed by immunohistochemical staining of CD34 (+) and CD34 (-) cells using antibodies directed towards human myosin heavy chain, as well as the skeletal muscle specific markers myf5 and myoD1. RT-PCR was performed to assess gene expression of myosin heavy chain, myf5, and myoD1.

### Results:

Regenerative cells from CD34 (-) clonal colonies differentiated to chondrocytes, osteoblasts, and muscle cells, depending on the culture medium used, regenerative cells cultured in osteogenic medium differentiated to osteoblasts, while regenerative cells cultured in chondrogenic medium differentiated to chondrocytes, as evidenced by histochemical staining with alcian blue and alkaline phosphatase, respectively. Moreover, regenerative cells from CD34 (-) clonal colonies that were cultured in complete medium in the absence of any differentiation medium were immunohistochemically positive for myosin heavy chain, Myf5 and MyoD, demonstrating their differentiation to a myoblast phenotype. Consistent with the immunohistochemical data, these muscle clones expressed the genes for myosin heavy chain, Myf5, and MyoD.

### Summary:

In summary, these data show that human cultured regenerative cell colonies give rise to chondrocytes, osteoblasts, and skeletal myoblasts. Thus, the regenerative cells of the present invention have the ability to stimulate cartilage, bone, and skeletal muscle formation to restore lost tissue, e.g., soft tissue tears in the cartilage, tendon, or ligament, as well as in repairing hard tissue breaks, such as in non-union long bone fractures. Additionally, the regenerative cells of the present invention have the ability to generate tissue *de novo* in order to impart mechanical support, e.g., in spinal fusion applications by bone formation between the transverse processes or vertebrae.

Any feature or combination of features described herein are included within the scope of the present invention provided that the features included in any such combination are not mutually inconsistent as will be apparent from the context, this specification, and the knowledge of one of ordinary skill in the art. For purposes of summarizing the present invention, certain aspects, advantages and novel features of the present invention have been described herein. Of course, it is to be understood that not necessarily all such aspects, advantages or features will be embodied in any particular embodiment of the present invention. Additional advantages and aspects of the present invention are apparent in the following detailed description and claims.

The above-described embodiments have been provided by way of example, and the present invention is not limited to these examples. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein. Accordingly, the present invention is not intended to be limited by the disclosed embodiments, but is to be defined by reference to the appended claims.

## Claims

1. A method of preparing regenerative cells for placement into the person from whom they were obtained, wherein said patient has a bone related disorder selected from the group consisting of spinal fusion defect, spinal stabilization defect, segmental defect, a bone related disorder in a non-osteoconductive area of the body, a disorder of the vertebrae, and a disorder of the discs, comprising:
(1) introducing tissue that comprises regenerative cells removed from a subject into an automated cell processing unit configured to maintain a closed pathway, wherein said automated cell processing unit comprises
(a) a chamber assembly to receive said tissue, wherein said chamber assembly is adapted to facilitate disaggregation of the tissue such that regenerative cells are separated from the tissue;
(b) a concentrating device which can connect to the chamber assembly, wherein the concentrating device is configured to concentrate said regenerative cells from the disaggregated tissue present in the chamber assembly; and
(c) a programmable processing device capable of communicating with and controlling the chamber assembly and the concentrating device;
(2) processing said tissue to obtain a concentration of regenerative cells sufficient to constitute an effective dose for promoting bone and/or cartilage formation; and
(3) adding the regenerative cells separated and concentrated by the automated system to a cell carrier portion of a device comprising the cell carrier portion and a cell carrier containment portion wherein the regenerative cells are not human embryonic stem cells.

2. The method according to claim 1, wherein the regenerative cells comprises stem cells, progenitor cells or a combination thereof.

3. The method according to any of the preceding claims, wherein the regenerative cells are autologous cells.

4. The method according to any of the preceding claims, wherein the regenerative cells are cultured prior to introdudng said regenerative cells into the device.

5. The method according to any of the preceding claims, wherein the prosthetic device Is an interbody spinal fusion device.

6. The method according to any of the preceding claims, wherein the cell carrier containment portion Is resorbable.

7. The method according to any of the preceding claims, wherein the cell carrier containment portion is non-resorbable.

8. The method according to any of the preceding claims wherein the cell containment portion is coated with apatite.

9. The method according to any of the preceding claims, wherein the cell carrier portion is coated using a stimulated body fluid solution.

10. The method according to any of the preceding claims, wherein said concentrated regenerative cells are suitable for infusion into a patient.

11. The method according to claim 4, wherein said culturing step comprises exposing the regenerative cells to culture conditions that promote differentiation towards a osteogenic phenotype.

12. The method according to any of the preceding claims, wherein the tissue is adipose tissue.

13. The method according to any of the preceding claims, further comprising adding one or more additives to the regenerative cells before introducing the regenerative cells to into the device.

14. The method according to any of the preceding claims, further comprising adding one or more additives to the regenerative cells after introducing the cells to the device.

15. The method according to any of the preceding claims, further comprising adding one or more additives to the device before introducing the regenerative cells to Into the device.

16. The method according to any of the preceding claims, wherein the additives are selected from growth factors, re-suspension fluids, cell culture reagents, cell expansion reagents, cell preservation reagents or cell modification reagents, or combinations thereof.

## Patentansprüche

1. Verfahren zur Herstellung von regenerativen Zellen zum Einbringen in die Person, aus welcher sie erhalten wurden, wobei der Patient eine Knochenerkrankung aufweist, welche aus der Gruppe bestehend aus Wirbelsäulenfusionsdefekt, Wirbelsäulenstabilisierungsdefekt, segmentalem Defekt, einer Knochenerkrankung in einem nicht osteokonduktiven Bereich des Körpers, einer Wirbelerkrankung und einer Bandscheibenerkrankung ausgewählt ist, umfassend:
(1) Einführen von Gewebe, welches von einem Individuum entfernte regenerative Zellen umfasst, in eine automatisierte Zellenverarbeitungseinheit, welche zur Aufrechterhaltung eines geschlossenen Pfads ausgebildet ist, wobei die automatisierte Zellenverarbeitungseinheit umfasst
(a) eine Kammereinheit zur Aufnahme des Gewebes, wobei die Kammereinheit dazu angepasst ist, eine Disaggregation des Gewebes derart zu erleichtern, dass regenerative Zellen vom Gewebe getrennt werden;
(b) eine Konzentrationsvorrichtung, welche mit der Kammereinheit verbunden werden kann, wobei die Konzentrationsvorrichtung zur Konzentration der regenerativen Zellen des in der Kammereinheit vorhandenen disaggregierten Gewebes ausgebildet ist; und
(c) eine programmierbare Verarbeitungsvorrichtung, welche dazu im Stande ist, mit der Kammereinheit und der Konzentrationsvorrichtung zu kommunizieren und diese zu steuern;
(2) Verarbeitung des Gewebes zum Erhalten einer Konzentration von regenerativen Zellen, welche dazu ausreichend sind, eine wirksame Dosis zur Förderung von Knochen- und/oder Knorpelbildung auszumachen; und
(3) Zugabe der durch das automatisierte System getrennten und konzentrierten regenerativen Zellen zu einem Zellträgerabschnitt einer Vorrichtung umfassend den Zellträgerabschnitt und einen Zellträgerhaltevorrichtungsabschnitt;
wobei die regenerativen Zellen nicht humane embryonale Stammzellen sind.

2. Verfahren nach Anspruch 1, wobei die regenerativen Zellen Stammzellen, Vorläuferzellen oder eine Kombination davon umfassen.

3. Verfahren nach einem der vorgehenden Ansprüche, wobei die regenerativen Zellen autologe Zellen sind.

4. Verfahren nach einem der vorgehenden Ansprüche, wobei die regenerativen Zellen vor dem Einführen der regenerativen Zellen in die Vorrichtung kultiviert werden.

5. Verfahren nach einem der vorgehenden Ansprüche, wobei die Prothesenvorrichtung eine Vorrichtung zur interkorporellen Wirbelsäulenfusion ist.

6. Verfahren nach einem der vorgehenden Ansprüche, wobei der Zellträgerhaltevorrichtungsabschnitt resorbierbar ist.

7. Verfahren nach einem der vorgehenden Ansprüche, wobei der Zellträgerhaltevorrichtungsabschnitt nicht-resorbierbar ist.

8. Verfahren nach einem der vorgehenden Ansprüche, wobei der Zellträgerhaltevorrichtungsabschnitt mit Apatit beschichtet wird.

9. Verfahren nach einem der vorgehenden Ansprüche, wobei der Zellträgerhaltevorrichtungsabschnitt unter Verwendung einer stimulierten Körperflüssigkeitslösung beschichtet wird.

10. Verfahren nach einem der vorgehenden Ansprüche, wobei die konzentrierten regenerativen Zellen zur Infusion in einen Patienten geeignet sind.

11. Verfahren nach Anspruch 4, wobei der Schritt des Kultivierens den Schritt umfasst, dass die regenerativen Zellen Kultivierungsbedingungen ausgesetzt werden, welche eine Differenzierung gegenüber einem osteogenen Phänotyp fördern.

12. Verfahren nach einem der vorgehenden Ansprüche, wobei das Gewebe Fettgewebe ist.

13. Verfahren nach einem der vorgehenden Ansprüche, welches ferner die Zugabe von einem oder mehreren Additiv(en) zu den regenerativen Zellen vor dem Einführen der regenerativen Zellen in die Vorrichtung umfasst.

14. Verfahren nach einem der vorgehenden Ansprüche, welches ferner die Zugabe von einem oder mehreren Additiv(en) zu den regenerativen Zellen nach dem Einführen der regenerativen Zellen in die Vorrichtung umfasst.

15. Verfahren nach einem der vorgehenden Ansprüche, welches ferner die Zugabe von einem oder mehreren Additiv(en) zur Vorrichtung vor dem Einführen der regenerativen Zellen in die Vorrichtung umfasst.

16. Verfahren nach einem der vorgehenden Ansprüche, wobei die Additive aus Wachstumsfaktoren, Resuspensionsflüssigkeiten, Zellkulturreagenzien, Zellexpansionsreagenzien, Zellkonservierungsreagenzien oder Zellenmodifikationsreagenzien oder Kombinationen davon ausgewählt sind.

## Revendications

1. Procédé de préparation des cellules régénératives pour placement chez la personne à partir de laquelle elles ont été obtenues, ledit patient souffrant d'une maladie osseuse sélectionnée parmi le groupe composé par un défaut de fusion spinale, un défaut de stabilisation spinale, un défaut segmentaire, une maladie osseuse dans une zone non ostéoconductrice du corps, un trouble de la vertèbre et un trouble lié à des disques, comprenant:
(1) l'introduction d'un tissu comprenant les cellules régénératives ôtées d'un sujet dans une unité de traitement automatisé des cellules configurée pour maintenir un trajet fermé, ladite unité de traitement automatisé des cellules comprenant
(a) un ensemble de chambre pour recevoir ledit tissu, ledit ensemble de chambre étant adapté pour faciliter la désagrégation du tissu si bien que les cellules régénératives sont séparées du tissu;
(b) un dispositif de concentration qui peut se raccorder à l'ensemble de chambre, le dispositif de concentration étant configuré pour concentrer lesdites cellules régénératives du tissu désagrégé présent dans l'ensemble de chambre; et
(c) un dispositif de traitement programmable étant capable de communiquer avec et de commander l'ensemble de chambre et le dispositif de concentration;
(2) le traitement dudit tissu pour obtenir une concentration des cellules régénératives suffisante pour constituer une dose efficace pour promouvoir la formation des os et/ou du cartilage; et
(3) l'addition des cellules régénératives séparées et concentrées par le système automatisé à une portion porteuse des cellules d'un dispositif comprenant la portion porteuse des cellules et une portion de confinement et porteuse des cellules;
les cellules régénératives étant les cellules souches embryonnaires non humaines.

2. Procédé selon la revendication 1, dans lequel les cellules régénératives comprennent les cellules souches, les cellules progénitrices ou une combination de celles-ci.

3. Procédé selon l'une quelconque des revendications précédentes, dans lequel les cellules régénératives sont les cellules autologues.

4. Procédé selon l'une quelconque des revendications précédentes, dans lequel les cellules régénératives sont cultivées avant l'introduction desdites cellules régénératives dans le dispositif.

5. Procédé selon l'une quelconque des revendications précédentes, dans lequel le dispositif prothétique est un dispositif de fusion intervertébrale épinière.

6. Procédé selon l'une quelconque des revendications précédentes, dans lequel la portion de confinement et porteuse des cellules est résorbable.

7. Procédé selon l'une quelconque des revendications précédentes, dans lequel la portion de confinement et porteuse des cellules est non résorbable.

8. Procédé selon l'une quelconque des revendications précédentes, dans lequel la portion de confinement et porteuse des cellules est revêtue d'apatite.

9. Procédé selon l'une quelconque des revendications précédentes, dans lequel la portion porteuse des cellules est revêtue à l'aide d'une solution de fluide corporel stimulée.

10. Procédé selon l'une quelconque des revendications précédentes, dans lequel lesdites cellules régénératives concentrées sont adaptées pour perfusion à un patient

11. Procédé selon la revendication 4, dans lequel ladite étape de cultivation comprend l'exposition des cellules régénératives aux conditions de culture qui promeuvent la différenciation vers un phénotype ostéogénique.

12. Procédé selon l'une quelconque des revendications précédentes, dans lequel le tissu est un tissu adipeux.

13. Procédé selon l'une quelconque des revendications précédentes, comprenant en outre l'addition d'un ou de plusieurs additifs aux cellules régénératives avant l'introduction des cellules régénératives dans le dispositif.

14. Procédé selon l'une quelconque des revendications précédentes, comprenant en outre l'addition d'un ou de plusieurs additifs aux cellules régénératives après l'introduction des cellules dans le dispositif.

15. Procédé selon l'une quelconque des revendications précédentes, comprenant en outre l'addition d'un ou de plusieurs additifs au dispositif avant l'introduction des cellules régénératives dans le dispositif.

16. Procédé selon l'une quelconque des revendications précédentes, dans lequel les additifs sont sélectionnés parmi les facteurs de croissance, les fluides de remise en suspension, les réactifs de culture cellulaire, les réactifs d'extension de cellules, les réactifs de préservation de cellules ou les réactifs de modification de cellules, ou des combinaisons de ceux-ci.
